(19) Europäisches Patentamt
European Patent Office
Office européen des brevets

(11) EP 1 670 943 B1

(12) EUROPEAN PATENT SPECIFICATION

(45) Date of publication and mention of the grant of the patent:
**13.11.2013 Bulletin 2013/46**

(21) Application number: **04787244.5**

(22) Date of filing: **29.09.2004**

(51) Int Cl.:
***C12Q 1/48*** (2006.01) ***C12Q 1/68*** (2006.01)
***G01N 33/68*** (2006.01)

(86) International application number:
**PCT/EP2004/052353**

(87) International publication number:
**WO 2005/030947 (07.04.2005 Gazette 2005/14)**

(54) **DIAGNOSTIC AND THERAPEUTIC USE OF A SULFOTRANSFERASE FOR ALZHEIMER'S DISEASE**

DIAGNOSTISCHE UND THERAPEUTISCHE VERWENDUNG EINER SULFOTRANSFERASE FÜR ALZHEIMER-KRANKHEIT

UTILISATION DIAGNOSTIQUE ET THERAPEUTIQUE D'UNE SULFOTRANSFERASE POUR LA MALADIE D'ALZHEIMER

(84) Designated Contracting States:
**AT BE BG CH CY CZ DE DK EE ES FI FR GB GR HU IE IT LI LU MC NL PL PT RO SE SI SK TR**

(30) Priority: **30.09.2003 US 506775 P**

(43) Date of publication of application:
**21.06.2006 Bulletin 2006/25**

(73) Proprietor: **Evotec International GmbH**
**22419 Hamburg (DE)**

(72) Inventors:
- **VON DER KAMMER, Heinz**
**22607 Hamburg (DE)**
- **POHLNER, JOHANNES**
**22175 Hamburg (DE)**

(74) Representative: **von Kreisler Selting Werner**
**Deichmannhaus am Dom**
**Bahnhofsvorplatz 1**
**50667 Köln (DE)**

(56) References cited:
**WO-A-00/14251 WO-A-02/18541**

- **FALANY C N ET AL: "Molecular cloning and expression of novel sulphotransferase-like cDNAs from human and rat brain" BIOCHEMICAL JOURNAL, THE BIOCHEMICAL SOCIETY, LONDON, GB, vol. 346, no. 3, 15 March 2000 (2000-03-15), pages 857-864, XP002275713 ISSN: 0264-6021 cited in the application**
- **GLATT H ET AL: "Human cytosolic sulphotransferases: Genetics, characteristics, toxicological aspects" MUTATION RESEARCH, AMSTERDAM, NL, vol. 482, no. 1-2, 1 October 2001 (2001-10-01), pages 27-40, XP002275714 ISSN: 0027-5107**
- **HAN X ET AL: "Substantial sulfatide deficiency and ceramide elevation in very early Alzheimer's disease" MEDLINE, 1 August 2002 (2002-08-01), XP002965337**
- **KIM S-B ET AL: "Neurosteroids: Cerebrospinal fluid levels for Alzheimer's disease and vascular dementia diagnostics" JOURNAL OF CLINICAL ENDOCRINOLOGY AND METABOLISM, NEW YORK, NY, US, vol. 88, no. 11, November 2003 (2003-11), pages 5199-5206, XP009028705 ISSN: 0021-972X**
- **JANUS C ET AL: "Transgenic mouse models of Alzheimer's disease" BIOCHIMICA ET BIOPHYSICA ACTA. MOLECULAR BASIS OF DISEASE, AMSTERDAM, NL, vol. 1502, no. 1, 26 July 2000 (2000-07-26), pages 63-75, XP004276974 ISSN: 0925-4439**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 670 943 B1

## Description

The present invention relates to methods of diagnosing or prognosticating Alzheimer's disease in a subject.

Neurodegenerative diseases, in particular Alzheimer's disease (AD), have a strongly debilitating impact on a patient's life. Furthermore, these diseases constitute an enormous health, social, and economic burden. AD is the most common neurodegenerative disease, accounting for about 70% of all dementia cases, and It is probably the most devastating age-related neurodegenerative condition affecting about 10% of the population over 65 years of age and up to 45% over age 85 (for a recent review see Vickers et al., Progress in Neurobiology 2000, 60: 139-165). Presently, this amounts to an estimated 12 million cases in the US, Europe, and Japan. This situation will inevitably worsen with the demographic increase in the number of old people ("aging of the baby boomers") in developed countries. The neuropathological hallmarks that occur in the brains of individuals with AD are senile plaques, composed of amyloid-β protein, and profound cytoskeletal changes coinciding with the appearance of abnormal filamentous structures and the formation of neurofibrillary tangles. The amyloid-β (Aβ) protein evolves from the cleavage of the amyloid precursor protein (APP) by different kinds of proteases. The cleavage by the β/γ-secretase leads to the formation of Aβ peptides of different lengths, typically a short more soluble and slow aggregating peptide consisting of 40 amino acids and a longer 42 amino acid peptide, which rapidly aggregates outside the cells, forming the characteristic amyloid plaques (Selkoe, Physiological Rev 2001, 81: 741-66; Greenfield et al., Frontiers Bioscience 2000, 5: D72-83). They are primarily found in the cerebral cortex and hippocampus. The generation of toxic Aβ deposits in the brain starts very early In the course of AD, and it is discussed to be a key player for the subsequent destructive processes leading to AD pathology. The other pathological hallmarks of AD are neurofibrillary tangles (NFTs) and abnormal neurites, described as neuropil threads (Braak and Braak, Acta Neuropathol 1991, 82: 239-259). NFTs emerge Inside neurons and consist of chemically altered tau, which forms paired helical filaments twisted around each other. The appearance of neurofibrillary tangles and their increasing number correlates well with the clinical severity of AD (Schmitt et al., Neurology 2000, 55: 370-376).

AD is a progressive disease that is associated with early deficits in memory formation and ultimately leads to the complete erosion of higher cognitive function. The cognitive disturbances include among other things memory impairment, aphasia, agnosia and the loss of executive functioning. A characteristic feature of the pathogenesis of AD is the selective vulnerability of particular brain regions and subpopulations of nerve cells to the degenerative process. Specifically, the temporal lobe region and the hippocampus are affected early and more severely during the progression of the disease. On the other hand, neurons within the frontal cortex, occipital cortex, and the cerebellum remain largely intact and are protected from neurodegeneration (Terry et al., Annals of Neurology 1981, 10: 184-92). The age of onset of AD may vary within a range of 50 years, with early-onset AD occurring in people younger than 65 years of age, and late-onset of AD occurring in those older than 65 years. About 10% of all AD cases suffer from early-onset AD, with only 1-2% being familial, inherited cases.

Currently, there is no cure for AD, nor is there an effective treatment to halt the progression of AD or even to diagnose AD ante-mortem with high probability. Several risk factors have been identified that predispose an individual to develop AD, among them most prominently the epsilon 4 allele of the three different existing alleles (epsilon 2, 3, and 4) of the apolipoprotein E gene (ApoE) (Strittmatter et al., Proc Natl Acad Sci USA 1993, 90: 1977-81; Roses, Ann NY Acad Sci 1998, 855: 738-43).

Glatt, H. et al. disclose in Mutation Research 482 (2001) 27-40, that cytosolic sulphatransferases transfer the sulpho moiety from the cofactor 6'-phosphoadenosine-3'-phosphosulphate (PAPS) to nucleophilic groups of xenobiotics and small endogenous compounds (such as hormones and neurotransmitters). This reaction often leads to products that can be excreted readily. However, other sulpho conjugates are strong electrophiles and may covalently bind with DNA and proteins. All known cytosolic sulphotransferases are members of an enzyme/gene superfamily termed SULT. In humans, 10 SULT genes are known. One of these genes encodes two different enzyme forms due to the use of alternative first exons. Different SULT forms substantially differ in their substrate specificity and tissue distribution. Genetic polymorphisms have been described for three human SULTs. Several allelic variants differ in functlonal properties, including the activation of promutagens. Only initial results are available from the analysis of SULT allele frequencies in different population groups, e.g. subjects suffering from specific diseases and corresponding controls.

Han, X. et al. disclose In an article in J Neurochem. 2002 Aug; 82(4):809-18 about substantial sulfatide deficiency and ceramide elevation in very early Alzheimer's disease: potential role in disease pathogenesis. In addition to pathology in the gray matter, there are also abnormalities in the white matter in Alzheimer-'s disease (AD). Sulfatide species are a class of myelin-specific sphingolipids and are involved in certain diseases of the central nervous system. To assess whether sulfatide content In gray and white matter in human subjects is associated with both the presence of Alzheimer's disease (AD) pathology as well as the stage of dementia, we analyzed the sulfatide content of brain tissue lipid extracts by electrospray ionization mass spectrometry from 22 subjects whose cognitive status at time of death varied from no dementia to very severe dementia. All subjects with dementia had AD pathology. The results demonstrate that: (i) sulfatides were depleted up to 93% in gray matter and up to 58% in white matter from all examined brain regions from AD subjects with very mild dementia, whereas all other major classes of lipid (except plasmalogen) in these subjects were not altered in comparison to those from age-matched subjects with no dementia; (ii) there was no apparent deficiency in the biosynthesis of sulfatides in very mild AD subjects as characterized by the examination of galactocerebroside sulfotransferase activities in post-mortem brain tissues; (iii) the content of ceramides (a class of potential degradation products of sulfatides) was elevated more than three-fold in white matter and peaked at the stage of very mild dementia. The findings demonstrate that a marked decrease in sulfatides is associated with AD pathology even in subjects with very mild dementia and that these changes may be linked with early events in the pathological process of AD.

Kim et al. report in Journal of clinical endocrinology and Metabolism 88 (11): 5199 that a neurodegenerative disease such as Alzheimer's disease (AD) is associated with significantly higher dehydroepiandrosterone (DHEA) levels in cerebrospinal fluid (CSF). Because the human brain is known to transform DHEA into DHEA sulfate (DHEAS), 7α-hydroxy-DHEA, 7β-hydroxy-DHEA, and 16α-hydroxy-DHEA, it is possible that DHEA accumulation in the brain results from a decreased production of such metabolites. To test this hypothesis, CSF levels of DHEA, DHEAS, 7α-hydroxy-DHEA, 7βhydroxy-DHEA, and 16α-hydroxy-DHEA have been measured and compared in 14 patients with AD, 12 controls, and eight patients with another common dementia, vascular dementia (VD). Results indicated that DHEAS CSF levels were significantly decreased in AD and VD ($P < 0.007$), whereas other metabolite levels were not significantly changed. Use of steroid level ratios, such as DHEA/(7α-hydroxy-DHEA + 7β-hydroxy-DHEA), 7β-hydroxy-DHEA/DHEA, and DHEAS/DHEA ratios, resulted in significant differences between diseased and control patients ($P < 0.0003$, $P < 0.002$, and $P < 0.002$, respectively). In addition, the 7α-hydroxy-DHEA/7β-hydroxy-DHEA ratio was significantly different between AD and VD ($P < 0.0001$) and could be used for differentiating AD from VD. These results Indicate that, in AD and VD, increased DHEA levels are not neuroprotective and are neither better sulfated nor better hydroxylated at the 7α, 7β, and 16α positions than in controls. The results also suggest that, in AD and VD brains, the sulfotransferase and the cytochromes P450 responsible for the 7α-, 7β-, and 16α-hydroxylations of DHEA are either present at lower levels or transformed through natural polymorphism into less-efficient enzymes.

WO 00/14251 discloses human transferase proteins (TRNSFS) and polynucleotides which identify and encode TRNSFS. The invention also provides expression vectors, host cells, antibodies, agonists, and antagonists. The invention also provides methods for diagnosing, treating, or preventing disorders associated with expression of TRNSFS.

Janus, C. et al disclose in Biochim Biophys Acta 1502:63-75 (2000) a transgenic mouse model of Alzheimer's disease.

Although there are rare examples of early-onset AD which have been attributed to genetic defects in the genes for amyloid precursor protein (APP) on chromosome 21, presenilin-1 on chromosome 14, and presenilin-2 on chromosome 1, the prevalent form of late-onset sporadic AD is of hitherto unknown etiologic origin. The late onset and complex pathogenesis of neurodegenerative disorders pose a formidable challenge to the development of therapeutic and diagnostic agents. It is crucial to expand the pool of potential drug targets and diagnostic markers. It is therefore an object of the present invention to provide insight into the pathogenesis of neurological diseases and to provide methods, materials, agents, compositions, and animal models which are suited inter alia for the diagnosis and development of a treatment of these diseases. This object has been solved by the features of the independent claims. The subclaims define preferred embodiments of the present invention.

The present invention is based on the differential expression of a gene coding for a cytosolic sulfotransferase and the protein products in human Alzheimer's disease brain samples. Sulfotransferases play important roles in the metabolism of various drugs, xenobiotics and endogenous molecules (Falany, FASEB J. 1997, 11: 206-216). They are able to conjugate said molecules with negatively charged sulfonate moieties thereby rendering the compounds more soluble. This leads to an improved detoxification by a facilitated excretion of the modified substances. In addition, sulfation may interfere with the biological activity of the compounds. Among those compounds which are enzymatically conjugated by transferring the sulfur trioxide sulfonate from the donor 3'-phosphoadenosine 5'-phosphosulfate are for example thyroid hormones, dopamine, steroids and neurotransmitters.

The family of the sulfotransferases may be subdivided into two classes. One family spans the membrane associated enzymes which mainly reside in the Golgi and have been described to be involved in the sulfation of glycosaminoglycans, glycoproteins and tyrosine-residues. The other family consists of cytosolic enzymes which are responsible for the modification of steroids, monoamine neurotransmitters, xenobiotics and drugs.

SULT4A1 (Homo sapiens cytosolic sulfotransferase, Genbank accession number A251263; also named BR-STL-1, Genbank accession number AF188698; SULTX3, Genbank accession number AF115311, and nervous system cytosolic sulfotransferase, NST or SULTn, Genbank accession number AF176342) was cloned from human, mouse, and rat brain cDNA libraries (Falany et al., Biochem. J. 2000, 346: 857-864; Sakakibara et al. Gene 2002, 285: 39-47; Patent application WO 02/18541). The human 855 bp long open reading frame codes for 284 amino acids with a calculated molecular weight of approximately 33 kDa and shares a 98% sequence identity with the mouse (mouse Sult4A1, gene ID: 29859, locus NT_039621) and rat homologues (Genbank accession number 043728; Falany et al., Biochem. J. 2000, 346: 857-864; Sakakibara et al. Gene 2002, 285: 39-47). SULT4A1 was mapped to chromosome 22q13, consists of 7 exons and spans a total of 47 kbp of genomic DNA (WO 02/18541). Bioinformatic analysis revealed that there exist at least three splice variants. The first splice variant, hereinafter also referred to as SULT4A1sv1, is identical to above mentioned sequence of SULT4A1 (Genbank accession number AF176342). The second splice variant, hereinafter named SULT4A1sv2, lacks two exons which span nucleotides 190-528 from Genbank entry AF176342 (SULT4A1sv1) resulting in an open reading frame of 516 nucleotides which code for 171 amino acids. The third splice variant differs by the lack of one exon located at nucleotides 190-320 which might presumably result in an altered open reading frame at the C-terminus.

A multitude of single nuleotide polymorphisms have been mapped in the region of the SULT4A1 gene (lida et al., J. Hum. Genet. 2001, 46:225-240). SULT4A1 has been assigned a sulfotransferase because of its sequence similarity to other human sulfotransferases, sharing a sequence identity over 30% especially in regions which have been shown to be involved in binding of the sulfate-donor substrate and the catalytic active site of the sulfotransferase family (Falany et al., Biochem. J. 2000, 346: 857-864).

Northern blot analysis revealed that the protein is mainly expressed in brain tissue, the highest expression levels being located in cortical regions (Falany et al., Biochem. J. 2000, 346: 857-864; Sakakibara et al., Gene 2002, 285: 39-47). Two research groups could not demonstrate any enzymatic activity towards a variety of well known sulfotransferase substrates, thus suggesting that SULT4A1 could have a very selective substrate or may be active In a multi-enzyme complex (Falany et al., Biochem. J. 2000, 346: 857-864; Adjel and Weinshilboum, Biochem. Biophys. Res. Comm. 2002, 292: 402-408). However, Sakakibara et al. demonstrated that both, human and mouse SULT4A1 protein are acitve on endogenous and xenobiotic compounds like L-triiodothyronine, thyroxine, estrone, p-nitrophenol, 2-naphtylamine, and 2-naphthol (Sakakibara et al., Gene 2002, 285: 39-47).

Using an unbiased and sensitive differential display approach, a transcription product of the gene coding for splice variants (isoforms) of SULT4A1 is detected in human brain samples. Neurons within the inferior temporal lobe, the entorhinal cortex, the hippocampus, and the amygdala are subject to degenerative processes in AD (Terry et al., Annals of Neurology 1981, 10:184-192). These brain regions are mostly involved in the processing of learning and memory functions and display a selective vulnerability to neuronal loss and degeneration in AD. In contrast, neurons within the frontal cortex, the occipital cortex, and the cerebellum remain largely intact and preserved from neurodegenerative processes. Brain tissues from the frontal cortex (F), the temporal cortex (T), and the hippocampus (H) of AD patients and healthy, age-matched control individuals were used for the herein disclosed examples. Importantly, the present invention discloses the detection and differential regulation, a dysregulation of SULT4A1 transcripts In the inferior temporal lobe or in the hippocampus of brain samples taken from AD patients relative to frontal cortex samples. No such dysregulation is observed in samples obtained from age-matched, healthy controls. To date, no experiments have been described that demonstrate a relationship between the dysregulation of SULT4A1 gene expression and the pathology of neurodegenerative disorders, in particular AD. The link of the SULT4A1 gene and the encoded SULT4A1 proteins to such diseases, as disclosed in the present invention, offers new ways, inter alia, for the diagnosis and treatment of said disorders, in particular AD.

The singular forms "a", "an", and "the" as used herein and in the claims include plural reference unless the context dictates otherwise. For example, "a cell" means as well a plurality of cells, and so forth. The term "and/or" as used in the present specification and in the claims implies that the phrases before and after this term are to be considered either as alternatives or in combination. For instance, the wording "determination of a level and/or an activity" means that either only a level, or only an activity, or both a level and an activity are determined. The term "level" as used herein is meant to comprise a gage of, or a measure of the amount of, or a concentration of a transcription product, for instance an mRNA, or a translation product, for instance a protein or polypeptide. The term "activity" as used herein shall be understood as a measure for the ability of a transcription product or a translation product to produce a biological effect or a measure for a level of biologically active molecules. The term "activity" also refers to enzymatic activity or to biological activity and/or pharmacological activity which refers to binding, antagonization, repression, blocking or neutralization. The terms "level" and/or "activity" as used herein further refer to gene expression levels or gene activity. Gene expression can be defined as the utilization of the information contained in a gene by transcription and translation leading to the production of a gene product. "Dysregulation" shall mean an upregulation or downregulation of gene expression. A gene product comprises either RNA or protein and Is the result of expression of a gene. The amount of a gene product can be used to measure how active a gene is. The term "gene" as used in the present specification and In the claims comprises both coding regions (exons) as well as non-coding regions (e.g. non-coding regulatory elements such as promoters or enhancers, Introns, leader and trailer sequences). The term "ORF" is an acronym for "open reading frame" and refers to a nucleic acid sequence that does not possess a stop codon in at least one reading frame and therefore can potentially be translated into a sequence of amino acids. "Regulatory elements" shall comprise inducible and non-inducible promoters, enhancers, operators, and other elements that drive and regulate gene expression. The term "fragment," as used herein is meant to comprise e.g. an alternatively spliced, or truncated, or otherwise cleaved transcription product or translation product. The term "derivative" as used herein refers to a mutant, or an RNA-edited, or a chemically modified, or otherwise altered transcription product, or to a mutant, or chemically modified, or otherwise altered translation product. For the purpose of clarity, a derivative transcript, for instance, refers to a transcript having alterations in the nucleic acid sequence such as single or multiple nucleotide deletions, insertions, or exchanges. A "derivative" may be generated by processes such as altered phosphorylation, or glycosylation, or acetylation, or lipidation, or by altered signal peptide cleavage or other types of maturation cleavage. These processes may occur post-translationally. The term "modulator" as used in the present invention and in the claims refers to a molecule capable of changing or altering the level and/or the activity of a gene, or a transcription product of a gene, or a translation product of a gene. Preferably, a "modulator" is capable of changing or altering the biological activity of a transcription product or a translation product of a gene. Said modulation, for instance, may be an increase or a decrease in the biological activity and/or pharmacological activity, in enzyme activity, a change in binding characteristics, or any other change or alteration In the biological, functional, or immunological properties of said translation product of a gene. A "modulator" refers to a molecule which has the capacity to either enhance or inhibit, thus to "modulate" a functional property of an ion channel subunit or an ion channel, to "modulate" binding, antagonization, repression, blocking, neutralization or sequestration of an ion channel or ion channel subunit and to "modulate" activation, agonization and upregulation. "Modulation" will be also used to refer to the capacity to affect the biological activity of a cell. The terms "modulator", "agent", "reagent", or "compound" refer to any substance, chemical, composition or extract that have a positive or negative biological effect on a cell, tissue, body fluid, or within the context of any biological system, or any assay system examined. They can be agonists, antagonists, partial agonists or inverse agonists of a target. They may be nucleic acids, natural or synthetic peptides or protein complexes, or fusion proteins. They may also be antibodies, organic or inorganic molecules or compositions, small molecules, drugs and any combinations of any of said agents above. They may be used for testing, for diagnostic or for therapeutic purposes. Such modulators, agents, reagents or compounds can be factors present in cell culture media, or sera used for cell culturing, factors such as trophic factors. "Trophic factors" as used in the present invention include but are not limited to neurotrophic factors, to neuregulins, to cytokines, to neurokines, to neuroimmune factors, to factors derived from the brain (BDNF) and to factors of the TGF beta family. Examples of such trophic factors are neurotrophin 3 (NT-3), neurotrophin 4/5 (NT-4/5), nerve growth factor (NGF), fibroblast growth factor (FGF), epidermal growth factor (EGF), interleukin-beta, glial cell-derived neurotrophic factors (GDNF), ciliary neurotrophic factor (CNTF), Insulin-like growth factor (IGF), transforming growth factor (TGF) and platelet-derived growth factor (PDGF). The terms "oligonucleotide primer" or "primer" refer to short nucleic acid sequences which can anneal to a given target polynucleotide by hybridization of the complementary base pairs and can be extended by a polymerase. They may be chosen to be specific to a particular sequence or they may be randomly selected, e.g. they will prime all possible sequences in a mix. The length of primers used herein may vary from 10 nucleotides to 80 nucleotides. "probes" are short nucleic acid sequences of the nucleic acid sequences described and disclosed herein or sequences complementary therewith. They may comprise full length sequences, or fragments, derivatives, isoforms, or variants of a given sequence. The identification of hybridization complexes between a "probe" and an assayed sample allows the detection of the presence of other similar sequences within that sample. As used herein, "homolog or homology" is a term used in the art to describe the relatedness of a nucleotide or peptide sequence to another nucleotide or peptide sequence, which is determined by the degree of identity and/or similarity between said sequences compared. In the art, the terms "identity" and "similarity" mean the degree of polypeptide or polynucleotide sequence relatedness which are determined by matching a query sequence and other sequences of preferably the same type (nucleic acid or protein sequence) with each other. Preferred computer program methods to calculate and determine "identity" and "similarity" include, but are not limited to GCG BLAST (Basic Local Alignment Search Tool) (Altschul et al., J. Mol. Bio/. 1990, 215: 403-410; Altschul et al., Nucleic Acids Res. 1997, 25: 3389-3402; Devereux et al., Nucleic Acids Res. 1984, 12: 387), BLASTN 2.0 (Gish W., http://blast.wustl.edu, 1996-2002), FASTA (Pearson and Lipman, Proc. Natl. Acad. Sci. USA 1988, 85: 2444-2448), and GCG GelMerge which determines and aligns a pair of contigs with the longest overlap (Wilbur and Lipman, SIAM J. Appl. Math. 1984, 44: 557-567; Needleman and Wunsch, J. Mol. Biol. 1970, 48: 443-453). The term "variant" as used herein refers to any polypeptide or protein, in reference to polypeptides and proteins disclosed in the present invention, in which one or more amino acids are added and/or substituted and/or deleted and/or inserted at the N-terminus, and/or the C-terminus, and/or within the native amino acid sequences of the native polypeptides or proteins of the present invention. Furthermore, the term "variant" shall include any shorter or longer version of a polypeptide or protein. "Variants" shall also comprise a sequence that has at least about 80% sequence identity, more preferably at least about 90% sequence identity, and most preferably at least about 95% sequence identity with the amino acid sequences of SULT4A1, of SEQ ID NO. 1 and SEQ ID NO. 2. "Variants" of a protein molecule include, for example, proteins with conservative amino acid substitutions in highly conservative regions. "Proteins and polypeptides" of the present invention include variants, fragments and chemical derivatives of the protein comprising the amino acid sequences of SULT4A1, of SEQ ID NO. 1 and SEQ ID NO. 2. Sequence variations shall be included wherein a codon are replaced with another codon due to alternative base sequences, but the amino acid sequence translated by the DNA sequence remains unchanged. This known in the art phenomenon is called redundancy of the set of codons which translate specific amino acids. Included shall be such exchange of amino acids which would have no effect on functionality, such as arginine for lysine, valine for leucine, asparagine for glutamine. Proteins and polypeptides can be included which can be isolated from nature or be produced by recombinant and/or synthetic means. Native proteins or polypeptides refer to naturally-occurring truncated or secreted forms, naturally occurring variant forms (e.g. splice-variants) and naturally occurring allelic variants. The term "isolated" as used herein is considered to refer to molecules or substances which have been changed and/or that are removed from their natural environment, i.e. isolated from a cell or from a living organism in which they normally occur, and that are separated or essentially purified from the coexisting components with which they are found to be associated in nature, it is also said that they are "non-native". This notion further means that the sequences encoding such molecules can be linked by the hand of man to polynucleotides to which they are not linked in their natural state and such molecules can be produced by recombinant and/or synthetic means (non-native). Even if for said purposes those sequences may be introduced into living or non-living organisms by methods known to those skilled in the art, and even if those sequences are still present in said organisms, they are still considered to be isolated, to be non-native. In the present invention, the terms "risk", "susceptibility", and "predisposition" are tantamount and are used with respect to the probability of developing a neurodegenerative disease, preferably Alzheimer's disease.

The term "AD" shall mean Alzheimer's disease. "AD-type neuropathology" as used herein refers to neuropathological, neurophysiological, histopathological and clinical hallmarks as described in the instant invention and as commonly known from state-of-the-art literature (see: Iqbal, Swaab, Winblad and Wisniewski, Alzheimer's Disease and Related Disorders (Etiology, Pathogenesis and Therapeutics), Wiley & Sons, New York, Weinheim, Toronto, 1999; Scinto and Daffner, Early Diagnosis of Alzheimer's Disease, Humana Press, Totowa, New Jersey, 2000; Mayeux and Christen, Epidemiology of Alzheimer's Disease: From Gene to Prevention, Springer Press, Berlin, Heidelberg, New York, 1999; Younkin, Tanzi and Christen, Presenilins and Alzheimer's Disease, Springer Press, Berlin, Heidelberg, New York, 1998). The term "Braak stage" or "Braak staging" refers to the classification of brains according to the criteria proposed by Braak and Braak (Braak and Braak, Acta Neuropathology 1991, 82: 239-259). On the basis of the distribution of neurofibrillary tangles and neuropil threads, the neuropathologic progression of AD is divided into six stages (stage 0 to 6). In the instant invention Braak stages 0 to 2 represent healthy control persons ("controls"), and Braak stages 4 to 6 represent persons suffering from Alzheimer's disease ("AD patients"). The values obtained from said "controls" are the "reference values" representing a "known health status" and the values obtained from said "AD patients" are the "reference values" representing a "known disease status". Braak stage 3 may represent either a healthy control persons or an AD patient. The higher the Braak stage the more likely is the possibility to display the symptoms of AD. For a neuropathological assessment, i.e. an estimation of the probability that pathological changes of AD are the underlying cause of dementia, a recommendation is given by Braak H. (www. alzforum.org).

Neurodegenerative disease or disorder according to the present invention is Alzheimer's disease. But also disclosed are relations to Parkinson's disease, Huntington's disease, amyotrophic lateral sclerosis, Pick's disease, fronto-temporal dementia, progressive nuclear palsy, corticobasal degeneration, cerebro-vascular dementia, multiple system atrophy, argyrophilic grain dementia and other tauopathies, and mild-cognitive impairment. Conditions involving neurodegenerative processes are, for Instance, age-related macular degeneration, narcolepsy, motor neuron diseases, prlon diseases and traumatic nerve injury and repair, and multiple sclerosis.

In one aspect, the invention features a method of diagnosing or prognosticating Alzheimer's disease in a subject, or determining whether a subject is at increased risk of developing said disease according to claim 1.

It is further disclosed the construction and the use of primers and probes which are unique to the nucleic acid sequences, or fragments, or variants thereof, as disclosed in the present invention. The oligonucleotide primers and/or probes can be labeled specifically with fluorescent, bioluminescent, magnetic, or radioactive substances. The invention further relates to the detection and the production of said nucleic acid sequences, or fragments and/or variants thereof, using said specific oligonucleotide primers in appropriate combinations. PCR-analysis, a method well known to those skilled in the art, can be performed with said primer combinations to amplify said gene specific nucleic acid sequences from a sample containing nucleic acids. Such sample may be derived either from healthy or diseased subjects. Whether an amplification results in a specific nucleic acid product or not, and whether a fragment of different length can be obtained or not, may be indicative for a neurodegenerative disease, in particular Alzheimer's disease. Thus, the invention provides nucleic acid sequences, oligonucleotide primers, and probes of at least 10 bases in length up to the entire coding and gene sequences, useful for the detection of gene mutations and single nucleotide polymorphisms in a given sample comprising nucleic acid sequences to be examined, which may be associated with neurodegenerative diseases, in particular Alzheimer's disease. This feature has utility for developing rapid DNA-based diagnostic tests, preferably also in the format of a kit.

It is further disclosed a method of monitoring the progression of a neurodegenerative disease in a subject. A level, or an activity, or both said level and said activity, of (i) a transcription product of a gene coding for SULT4A1, and/or of (ii) a translation product of a gene coding for SULT4A1, and/or of (iii) a fragment, or derivative, or variant of said transcription or translation product in a sample from said subject is determined. Said level and/or said activity is compared to a reference value representing a known disease or health status. Thereby the progression of said neurodegenerative disease, of Alzheimer's disease, in said subject is monitored.

It is further disclosed a method of evaluating a treatment for a neurodegenerative disease, comprising determining a level, or an activity, or both said level and said activity of (i) a transcription product of a gene coding for SULT4A1, and/or of (ii) a translation product of a gene coding for SULT4A1, and/or of (iii) a fragment, or derivative, or variant of said transcription or translation product in a sample obtained from a subject being treated for said disease. Said level, or said activity, or both said level and said activity are compared to a reference value representing a known disease or health status, thereby evaluating the treatment for said neurodegenerative disease, for Alzheimer's disease.

In the herein disclosed methods, kits, recombinant animals, molecules, assays, and uses said gene coding for a human sulfotransferase protein is the gene coding for a cytosolic sulfotransferase, a cytosolic sulfotransferase family 4A member 1 protein (SULT4A1) (EC 2.8.2), also termed nervous system cytosolic sulfotransferase NST or SULTn, represented by Genbank accession number AF176342, or also named BA-STL-1, represented by Genbank accession number AF188698 or AF251263, or termed SULTX3, represented by Genbank accession number AF115311. In a further preferred embodiment, said SULT4A1 gene is coding for a SULT4A1 isoform 1, also named splice variant 1 (SULT4A1sv1 gene, SEQ ID NO. 3, GenBank accession number: AF176342), coding for the protein SULT4A1 splice variant 1 (SULT4A1sv1 protein, SEQ ID NO.1, Genbank accession number 043728). It is further disclosed, that the gene coding for said SULT4A1sv1 protein is also generally referred to as the SULT4A1 gene or simply SULT4A1. Further, the protein of SULT4A1 or SULT4A1sv1 is also generally referred to as the SULT4A1 protein or simply SULT4A1.

In the instant invention, said SULT4A1 gene is coding for a SULT4A1 isoform 2, also named splice variant 2 (SULT4A1sv2 gene, SEQ ID NO. 4, GenBank accession number: AF176342 missing nucleotides 190 to 528, represented by the ESTs bi550483 and bm805353), coding for the protein SULT4A1 splice variant 2 (SULT4A1sv2 protein, SEQ ID NO. 2). **In** the instant invention, the gene coding for said SULT4A1sv2 protein is also generally referred to as the SULT4A1 gene, or simply SULT4A1. Further, the protein of SULT4A1 or SULT4A1sv2 is also generally referred to as the SULT4A1 protein or simply SULT4A1. In the instant invention said sequences are "isolated" as the term is employed herein.

The present invention discloses the detection, differential expression and dysregulation of the SULT4A1 gene in specific samples, in specific brain regions of AD patients in comparison to control persons. Further, the present invention discloses that the gene expression of SULT4A1 is dysregulated in AD-affected brains, in that SULT4A1 mRNA levels are down-regulated in the temporal cortex and/or the hippocampus as compared to the frontal cortex or are elevated in the frontal cortex as compared to the temporal cortex and/or the hippocampus. SULT4A1 expression differs between the frontal cortex and the temporal cortex and/or the hippocampus of healthy age-matched control subjects compared to the frontal cortex and the temporal cortex and/or the hippocampus of AD patients. Consequently, the SULT4A1 gene and its corresponding transcription and translation products (SULT4A1sv1, SULT4A1sv2) have a causative role in the regional selective neuronal degeneration typically observed in AD. SULT4A1 confers a neuroprotective function to the remaining surviving nerve cells as disclosed and described in the instant invention. Based on these disclosures, the present invention has utility for the diagnostic evaluation and prognosis as well as for the identification of a predisposition to a neurodegenerative disease, in particular AD. Furthermore, disclosed are methods for the diagnostic monitoring of patients undergoing treatment for such a disease.

It is preferred that said sample to be analyzed and determined is selected from the group comprising brain tissue, brain cells or other tissues or other body cells. The sample can also comprise cerebrospinal fluid or other body fluids including saliva, urine, blood, serum plasma, or mucus. Preferably, the methods of diagnosis, prognosis, monitoring the progression or evaluating a treatment for a neurodegenerative disease, according to the instant invention, can be practiced ex *corpore,* and such methods preferably relate to samples, for instance, body fluids or cells, removed, collected, or isolated from a subject or patient or healthy control person.

In further preferred embodiments, said reference value is that of a level, or an activity, or both said level and said activity of (i) a transcription product of a gene coding for SULT4A1, and/or of (ii) a translation product of a gene coding for SULT4A1, and/or of (iii) a fragment, or derivative, or variant of said transcription or translation product in a sample obtained from a subject not suffering from said neurodegenerative disease (healthy control person, control sample, control) or in a sample obtained from a subject suffering from a neurodegenerative disease, in particular Alzheimer's disease (patient sample, patient).

In preferred embodiments, an alteration in the level and/or activity, a varied level and/or activity of a transcription product of the gene coding for SULT4A1 and/or of a translation product of the gene coding for SULT4A1 and/or of a fragment, or derivative, or variant thereof, in a sample cell, or tissue, or body fluid obtained from a subject relative to a reference value representing a known health status (control sample) indicates a diagnosis, or prognosis, or increased risk of becoming diseased with a neurodegenerative disease, particularly AD.

In further preferred embodiments, an equal or similar level and/or activity of a transcription product of the gene coding for a SULT4A1 protein and/or of a translation product of the gene coding for a SULT4A1 protein and/or of a fragment, or derivative, or variant thereof in a sample cell, or tissue, or body fluid obtained from a subject relative to a reference value representing a known disease status of a neurodegenerative disease, in particular Alzheimer's disease (AD patient sample), indicates a diagnosis, or prognosis, or increased risk of becoming diseased with said neurodegenerative disease.

In preferred embodiments, measurement of the level of transcription products of a gene coding for SULT4A1 is performed in a sample obtained from a subject using a quantitative PCR-analysis with primer combinations to amplify said gene specific sequences from cDNA obtained by reverse transcription of RNA extracted from a sample of a subject.

Primer combinations are given in "Examples" of the instant invention, but also other primers generated from the sequences as disclosed in the instant invention can be used. A Northern blot with probes specific for said gene can also be applied. It might further be preferred to measure transcription products by means of chip-based micro-array technologies. These techniques are known to those of ordinary skill in the art (see Sambrook and Russell, Molecular Cloning: A Laboratory Manual, Cold Spring Harbor Laboratory Press, Cold Spring Harbor, New York, 2001; Schena M., Microarray Biochip Technology, Eaton Publishing, Natick, MA, 2000). An example of an immunoassay is the detection and measurement of enzyme activity as disclosed and described in the patent application WO 02/14543.

Furthermore, a level and/or an activity of a translation product of a gene coding for SULT4A1 and/or of a fragment, or derivative, or variant of said translation product, and/or a level of activity of said translation product and/or of a fragment, or derivative, or variant of said translation product, can be detected using an immunoassay, an activity assay, and/or a binding assay. These assays can measure the amount of binding between said protein molecule and an anti-protein antibody by the use of enzymatic, chromodynamic, radioactive, magnetic, or luminescent labels which are attached to either the anti-protein antibody or a secondary antibody which binds the anti-protein antibody. In addition, other high affinity ligands may be used. Immunoassays which can be used include e.g. ELISAs, Western blots and other techniques known to those of ordinary skill in the art (see Harlow and Lane, Using Antibodies: A Laboratory Manual, Cold Spring Harbor Laboratory Press, Cold Spring Harbor, New York, 1999 and Edwards R, Immunodiagnostics: A Practical Approach, Oxford University Press, Oxford; England, 1999). All these detection techniques may also be employed in the format of microarrays, protein-arrays, antibody microarrays, tissue microarrays, electronic biochip or protein-chip based technologies (see Schena M., Microarray Biochip Technology. Eaton Publishing, Natick, MA, 2000).

In a preferred embodiment, the level, or the activity, or both said level and said activity of (i) a transcription product of a gene coding for SULT4A1, and/or of (ii) a translation product of a gene coding for SULT4A1, and/or of (iii) a fragment, or derivative, or variant of said transcription or translation product in a series of samples taken from said subject over a period of time is compared, in order to monitor the progression of said disease. In further preferred embodiments, said subject receives a treatment prior to one or more of said sample gatherings. In yet another preferred embodiment, said level and/or activity is determined before and after said treatment of said subject.

Further disclosed is a kit for diagnosing or prognosticating neurodegenerative diseases, in particular AD, in a subject, or determining the propensity or predisposition of a subject to develop a neurodegenerative disease, in particular AD, said kit comprising:

(a) at least one reagent which is selected from the group consisting of (i) reagents that selectively detect a transcription product of a gene coding for SULT4A1 (ii) reagents that selectively detect a translation product of a gene coding for SULT4A1; and

(b) Instructions for diagnosing, or prognosticating a neurodegenerative disease, in particular AD, and/or determining the propensity or predisposition of a subject to develop such a disease by describing the steps of:

- detecting a level, or an activity, or both said level and said activity, of said transcription product and/or said translation product of a gene coding for SULT4A1, in a sample obtained from said subject: and
- diagnosing or prognosticating a neurodegenerative disease, in particular AD and/or determining the propensity or predisposition of said subject to develop such a disease, wherein a varied or altered level, or activity, or both said level and said activity, of said transcription product and/or said translation product compared to a reference value representing a known health status (control) and/or wherein a level, or activity, or both said level and said activity, of said transcription product and/or said translation product is similar or equal to a reference value representing a known disease status, preferably a disease status of AD, indicates a diagnosis or prognosis of a neurodegenerative disease, in particular AD, or an increased propensity or predisposition of developing such a disease. The kit, according to the present invention, may be particularly useful for the identification of individuals that are at risk of developing a neurodegenerative disease, in particular AD.

Further disclosed is the use of a kit In a method of diagnosing or prognosticating a neurodegenerative disease, in particular Alzheimer's disease, in a subject, and in a method of determining the propensity or predisposition of a subject to develop such a disease by the steps of: (i) detecting in a sample obtained from said subject a level, or an activity, or both said level and said activity of a transcription product and/or of a translation product of a gene coding for SULT4A1, and (ii) comparing said level or activity, or both said level and said activity of a transcription product and/or of a translation product of a gene coding for SULT4A1 to a reference value representing a known health status and/or to a reference value representing a known disease status, and said level, or activity, or both said level and said activity, of said transcription product and/or said translation product is varied compared to a reference value representing a known health status, and/or is similar or equal to a reference value representing a known disease status.

Consequently, the kit may serve as a means for targeting identified individuals for early preventive measures or therapeutic intervention prior to disease onset, before irreversible damage in the course of the disease has been inflicted. Furthermore, in preferred embodiments, the kit disclosed is useful for monitoring a progression of a neurodegenerative disease, in particular AD in a subject, as well as monitoring and evaluating success or failure of a therapeutic treatment for such a disease of said subject.

Further disclosed is a method of treating or preventing a neurodegenerative disease, in particular AD, in a subject comprising the administration to said subject in a therapeutically or prophylactically effective amount of an agent or agents which directly or indirectly affect a level, or an activity, or both said level and said activity, of (i) a gene coding for SULT4A1, and/or (ii) a transcription product of a gene coding for SULT4A1, and/or (iii) a translation product of a gene coding for SULT4A1, and/or (iv) a fragment, or derivative, or variant of (i) to (iii). Said agent may comprise a small molecule, or it may also comprise a peptide, an oligopeptIde, or a polypeptide. Said peptide, oligopeptide, or polypeptide may comprise an amino acid sequence of a translation product of a gene coding for SULT4A1, or a fragment, or derivative, or a variant thereof. An agent for treating or preventing a neurodegenerative disease, in particular AD, according to the instant invention, may also consist of a nucleotide, an oligonucleotide, or a polynucleotide. Said oligonucleotide or polynucleotide may comprise a nucleotide sequence of the gene coding for SULT4A1, either in sense orientation or in antisense orientation.

The disclosed method comprises the application of per se known methods of gene therapy and/or antisense nucleic acid technology to administer said agent or agents. In general, gene therapy includes several approaches: molecular replacement of a mutated gene, addition of a new gene resulting in the synthesis of a therapeutic protein, and modulation of endogenous cellular gene expression by recombinant expression methods or by drugs. Gene-transfer techniques are described in detail (see e.g. Behr, Acc Chem Res 1993, 26: 274-278 and Mulligan, Science 1993, 260: 926-931) and include direct gene-transfer techniques such as mechanical microinjection of DNA into a cell as well as indirect techniques employing biological vectors (like recombinant viruses, especially retroviruses) or model liposomes, or techniques based on transfection with DNA coprecipitation with polycations, cell membrane pertubation by chemical (solvents, detergents, polymers, enzymes) or physical means (mechanic, osmotic, thermic, electric shocks). The postnatal gene transfer into the central nervous system has been described in detail (see e.g. Wolff, Curr Opin Neurobiol 1993, 3: 743-748).

Further disclosed is a method of treating or preventing a neurodegenerative disease by means of antisense nucleic acid therapy, i.e. the down-regulation of an inappropriately expressed or defective gene by the introduction of antisense nucleic acids or derivatives thereof into certain critical cells (see e.g. Gillespie, DN&P 1992, 5: 389-395; Agrawal and Akhtar, Trends Biotechnol 1995, 13: 197-199; Crooke, Biotechnology 1992, 10: 882-6). Apart from hybridization strategies, the application of ribozymes, i.e. RNA molecules that act as enzymes, destroying RNA that carries the message of disease has also been described (see e.g. Barinaga, Science 1993, 262: 1512-1514). In preferred embodiments, the subject to be treated is a human, and therapeutic antisense nucleic acids or derivatives thereof are directed against transcripts of a gene coding for SULT4A1. It is preferred that cells of the central nervous system, preferably the brain, of a subject are treated in such a way. Cell penetration can be performed by known strategies such as coupling of antisense nucleic acids and derivatives thereof to carrier particles, or the above described techniques. Strategies for administering targeted therapeutic oligo-deoxynucleotides are known to those of skill in the art (see e.g. Wickstrom, Trends Biotechnol 1992, 10: 281-287). In some cases, delivery can be performed by mere topical application. Further approaches are directed to intracellular expression of antisense RNA. In this strategy, cells are transformed *ex vivo* with a recombinant gene that directs the synthesis of an RNA that is complementary to a region of target nucleic acid. Therapeutical use of intracellularly expressed antisense RNA is procedurally similar to gene therapy. A recently developed method of regulating the intracellular expression of genes by the use of doublestranded RNA, known variously as RNA interference (RNAi), can be another effective approach for nucleic acid therapy (Hannon, Nature 2002, 418: 244-251).

In further preferred embodiments, the method comprises grafting donor cells into the central nervous system, preferably the brain, of said subject, or donor cells preferably treated so as to minimize or reduce graft rejection, wherein said donor cells are genetically modified by insertion of at least one transgene encoding said agent or agents. Said transgene might be carried by a viral vector, in particular a retroviral vector. The transgene can be inserted into the donor cells by a nonviral physical transfection of DNA encoding a transgene, in particular by microinjection. Insertion of the transgene can also be performed by electroporation, chemically mediated transfection, in particular calcium phosphate transfection or liposomal mediated transfection (see Mc Celland and Pardee, Expression Genetics: Accelerated and High-Throughput Methods, Eaton Publishing, Natick, MA, 1999).

As further disclosed said agent for treating and preventing a neurodegenerative disease, in particular AD, is a therapeutic protein which can be administered to said subject, preferably a human, by a process comprising introducing subject cells into said subject, said subject cells having been treated *in vitro* to insert a DNA segment encoding said therapeutic protein, said subject cells expressing *in vivo* in said subject a therapeutically effective amount of said therapeutic protein. Said DNA segment can be inserted into said cells *in vitro* by a viral vector, in particular a retroviral vector.

Methods of treatment are disclosed that comprise the application of therapeutic cloning, transplantation, and stem cell therapy using embryonic stem cells or embryonic germ cells and neuronal adult stem cells, combined with any of the previously described cell- and gene therapeutic methods. Stem cells may be totipotent or pluripotent. They may also be organ-specific. Strategies for repairing diseased and/or damaged brain cells or tissue comprise (i) taking donor cells from an adult tissue. Nuclei of those cells are transplanted into unfertilized egg cells from which the genetic material has been removed. Embryonic stem cells are isolated from the blastocyst stage of the cells which underwent somatic cell nuclear transfer. Use of differentiation factors then leads to a directed development of the stem cells to specialized cell types, preferably neuronal cells (Lanza et al., Nature Medicine 1999, 9: 975-977), or (ii) purifying adult stem cells, isolated from the central nervous system, or from bone marrow (mesenchymal stem cells), for in vitro expansion and subsequent grafting and transplantation, or (iii) directly inducing endogenous neural stem cells to proliferate, migrate, and differentiate into functional neurons (Peterson DA, Curr. Opin. Pharmacol. 2002, 2: 34-42). Adult neural stem cells are of great potential for repairing damaged or diseased brain tissues, as the germinal centers of the adult brain are free of neuronal damage or dysfunction (Colman A, Drug Discovery World 2001, 7: 66-71).

The subject for treatment or prevention can be a human, an experimental animal, e.g. a mouse or a rat or a fly, a domestic animal, or a non-human primate. The experimental animal can be a non-human animal model for a neurodegenerative disorder, a genetically altered animal, e.g. a transgenic mouse or fly and/or a knockout mouse or fly preferably displaying symptoms of AD, showing an AD-type neuropathology.

Further disclosed is a modulator of an activity, or a level, or both said activity and said level of at least one substance which is selected from the group consisting of (i) a gene coding for SULT4A1, and/or (ii) a transcription product of a gene coding for SULT4A1, and/or (iii) a translation product of a gene coding for SULT4A1, and/or (iv) a fragment, or derivative, or variant of (i) to (iii).

Further disclosed is a pharmaceutical composition comprising said modulator and preferably a pharmaceutical carrier. Said carrier refers to a diluent, adjuvant, excipient, or vehicle with which the modulator is administered.

Further disclosed is a modulator of an activity, or a level, or both said activity and said level of at least one substance which is selected from the group consisting of (i) a gene coding for SULT4A1, and/or (ii) a transcription product of a gene coding for SULT4A1, and/or (iii) a translation product of a gene coding for SULT4A1, and/or (iv) a fragment, or derivative, or variant of (i) to (iii) for use in a pharmaceutical composition.

Further disclosed is the use of a modulator of an activity, or a level, or both said activity and said level of at least one substance which is selected from the group consisting of (i) a gene coding for SULT4A1 and/or (ii) a transcription product of a gene coding for SULT4A1 and/or (iii) a translation product of a gene coding for SULT4A1, and/or (iv) a fragment, or derivative, or variant of (i) to (iii) for a preparation of a medicament for treating or preventing a neurodegenerative disease, in particular AD.

Further disclosed is a kit comprising one or more containers filled with a therapeutically or prophylactically effective amount of said pharmaceutical composition.

Further disclosed is the use of non-native nucleic acid molecules and of translation products, protein molecules of the gene coding for human and/or mouse SULT4A1 (cytosolic sulfotransferase family 4A member 1) and/or fragments, or derivatives, or variants thereof, of nucleic acid molecules as shown in SEQ ID NO. 3, SEQ ID NO. 4, SEQ ID NO. 6, and protein molecules as shown in SEQ ID NO. 1, SEQ ID NO. 2, as targeting molecules to generate recombinant, genetically altered non-human animals which are transgenic animals and/or knockout animals. It is preferred that said genetically altered non-human animal is a mammal, preferably a rodent, more preferably a mouse or a rat or a guinea pig. It is further preferred that said genetically altered non-human animal is an invertebrate animal, preferably an insect, more preferably a fly such as the fly *Drosophila melanogaster.* Further, said genetically altered non-human animal may be a domestic animal, or a non-human primate. In one embodiment, the expression of said genetic alteration results in a non-human animal exhibiting a predisposition to developing symptoms and/or displaying symptoms of neuropathology similar to a neurodegenerative disease, in particular symptoms of a neuropathology similar to AD (AD-type neuropathology), including, inter alia, histological features of AD and behavioural changes characteristic of AD. In another embodiment, the expression of said genetic alteration results in a non-human animal which has a reduced risk of developing symptoms similar to a neurodegenerative disease, in particular a reduced risk of developing symptoms of a neuropathology similar to AD and/or which shows a reduction of AD symptoms and/or which has no AD symptoms due to a beneficial effect caused by the expression of the gene used to genetically alter said non-human animal.

Further disclosed is a recombinant, genetically altered non-human animal comprising a non-native gene sequence coding for SULT4A1 (cytosolic sulfotransferase family 4A member 1), or a fragment or a derivative, or a variant thereof, as shown in SEQ ID NO. 3, SEQ ID NO. 4, SEQ ID NO. 6, and as shown in SEQ ID NO. 1 and SEQ ID NO. 2. Said non-native gene sequence coding for SULT4A1 may be either the human and/or the mouse SULT4A1 gene sequence. The generation of said recombinant, genetically altered non-human animal comprises (i) providing a gene targeting construct containing a gene sequence of human and/or mouse SULT4A1, or a fragment, or a variant of said gene sequence, and a selectable marker sequence, and (ii) introducing said targeting construct into a stem cell, into an embryonal stem (ES) cell of a non-human animal, and (iii) introducing said non-human animal stem cell Into a non-human embryo, and (iv) transplanting said embryo into a pseudopregnant non-human animal, and (v) allowing said embryo to develop to term, and (vi) identifying a genetically altered non-human animal whose genome comprises a modification of said gene sequence in one or both alleles, and (vii) breeding the genetically altered non-human animal of step (vi) to obtain a genetically altered non-human animal whose genome comprises a modification of said endogenous gene. It is preferred that said genetically altered non-human animal expresses a recombinant, an altered gene wherein said expression is a mis-expression, or under-expression, or over-expression, or non-expression. Examples of such targeting constructs containing a gene sequence of human and/or mouse SULT4A1 and a selectable marker sequence, as well as the expression of said recombinant, altered SULT4A1 genes in non-human genetically altered animals are disclosed in the present invention (see Examples (Vii), (viii) and Figures 20 to 25).

Said gene disruption or suppression or activation or the expression of said genetic alteration results in said non-human animal exhibiting a predisposition to developing symptoms, and/or displaying symptoms of neuropathology similar to a neurodegenerative disease, in particular symptoms of a neuropathology similar to AD (AD-type neuropathology).

The expression of said genetic alteration results in a non-human animal which has a reduced risk of developing symptoms similar to a neurodegenerative disease, in particular a reduced risk of developing symptoms similar to AD and/or which shows a reduction of AD symptoms and/or which has no AD symptoms due to an effect, which can be a beneficial effect, caused by the expression of the gene used to genetically alter said non-human animal.

Said genetically altered non-human animal is a mammal, preferably a rodent, more preferably a mouse or a rat or a guinea pig. It is further preferred that said genetically altered non-human animal is an invertebrate animal, preferably an insect, more preferably a fly such as the fly *Drosophila melanogaster.* Further, said genetically altered non-human animal may be a domestic animal, or a non-human primate. Said genetically altered non-human is a transgenic animal and/or a knockout animal.

Said recombinant, genetically altered non-human animals are crossed to Alzheimer's disease animal models as commonly known in the art. It is preferred to use Alzheimer's disease mouse models such as transgenic mice expressing human Alzheimer Precursor Protein (APP) or mutant forms of APP, e.g. APP with the Swedish mutation, and/or human Presenilin-1 or -2 with known mutations as described in the literature (Janus and Westaway, Physiology Behavior 2001, 873-886; Richards et al., J. Neuroscience 2003, 23:8989-9003) and/or human Tau with known mutations, e.g. the P301L mutation (Götz et al., J. Biological Chemistry 2001, 276:529-534) or double or triple transgenic animals from those or other mouse mutants developing Alzheimer-like pathologies. Other Alzheimer's disease animal models can be recombinant animal models which are capable of producing neurofibrillary tangles and/or amyloid plaques; recombinant animal models which express a recombinant gene coding for a tau protein, such as human or mouse tau or tau isoforms as the four-repeat isoform or the P301 L mutant tau; recombinant animal models which express a recombinant gene coding for an amyloid precursor protein or a mutant amyloid precursor protein, or beta-amyloid; recombinant animal models which express a recombinant gene coding for a secretase, gamma-secretase, beta-secretase or alpha-secretase, Presenilin1 or Presenilin2; and any recombinant animal models which express a combination of the recombinant genes as described above.

Said crossing results in SULT4A1 knockout animals or transgenic SULT4A1 animals on an Alzheimer's diseases background which feature a strengthened and boosted predisposition to develop symptoms, and/or to display symptoms of neuropathology similar to a neurodegenerative disease, in particular symptoms of a neuropathology similar to AD (AD-type neuropathology), including inter alia histological features of AD and behavioural changes characteristic of AD. In another preferred embodiment said crossing results in SULT4A1 knockout animals or transgenic SULT4A1 animals on an Alzheimer's disease background which have a reduction of AD symptoms or a reduced risk of developing symptoms similar to a neurodegenerative disease, in particular a reduced risk of developing symptoms of a neuropathology similar to AD, or showing no AD symptoms due to a beneficial effect caused by the expression of the gene used to genetically alter said non-human animal.

The genetically altered non-human transgenic animal and/or a knockout animal can be used as an experimental animal, as a test animal, as an animal model for a neurodegenerative disorder, preferably as an animal model for Alzheimer.

Examples of such genetically altered non-human animals showing such neuropathological features and/or showing reduced symptoms are disclosed in the present invention (see Examples and Figures).

Strategies and techniques for the generation and construction of such a transgenic and/or knockout animal are known to those of ordinary skill in the art (see e.g. Capecchi, Science 1989, 244: 1288-1292 and Hogan et al., Manipulating the Mouse Embryo: A Laboratory Manual, Cold Spring Harbor Laboratory Press, Cold Spring Harbor, New York, 1994 and Jackson and Abbott, Mouse Genetics and Transgenics: A Practical Approach, Oxford University Press, Oxford, England, 1999) and are described in detail in the present invention (see Examples and Figures).

It is preferred to make use of such a recombinant, genetically altered non-human animal, transgenic or knockout animal, as an animal model for Investigating neurodegenerative diseases, in particular Alzheimer's disease. Such an animal may be a test animal or an experimental animal useful for screening, testing and validating compounds, agents and modulators in the development of diagnostics and therapeutics to treat neurodegenerative diseases, in particular Alzheimer's disease.

In one further aspect, the invention features a screening assay for a modulator of neurodegenerative diseases, in particular AD, or related diseases and disorders of one or more substances selected from the group consisting of (i) a gene coding for SULT4A1, and/or (ii) a transcription product of a gene coding for SULT4A1, and/or (iii) a translation product of a gene coding for SULT4A1, and/or (iv) a fragment, or derivative, or variant of (i) to (iii), comprising (a) administering a test compound to a test animal or experimental animal or animal model, which is predisposed to developing or has already developed symptoms of a neurodegenerative disease or related diseases or disorders, and (b) measuring the activity and/or level of one or more substances recited in (i) to (iv), and (c) measuring the activity and/or level of said substances in a matched control animal which is equally predisposed to developing or has already developed symptoms of said diseases and to which animal no such test compound has been administered, and (d) comparing the activity and/or level of the substance in the animals of step (b) and (c), wherein an alteration in the activity and/or level of substances in the test animal, or experimental animal, or animal model indicates that the test compound is a modulator of said diseases and disorders.

Said test animal, or experimental animal, or animal model and/or said control animal is a recombinant, genetically altered non-human animal which expresses a gene coding for SULT4A1, or a fragment, or a derivative, or a variant thereof, under the control of a transcriptional regulatory element which is not the native SULT4A1 gene transcriptional control regulatory element, as disclosed in the present invention (see below).

The genetically altered non-human animals provide an in-vivo assay to determine or validate the efficacy of therapies, or modulatory agents, or compounds for the treatment of neurodegenerative diseases, in particular Alzheimer's disease.

In another aspect, the invention features an *in vitro* assay for screening for a modulator of AD according to claim 2. Examples of cells used in said screening assay, such as cells over-expressing the SULT4A1 protein, preferably stably over-expressing the SULT4A1 protein, as disclosed in the present invention, are given below (Example (v) and Figure 18). A detailed example of an assay, using said cells is disclosed in the present invention (see Example (vi) and Figure 19).

The examples of the genetically altered animals and cells and screening assays as disclosed, are illustrative only and not intended to limit the remainder of the disclosure in any way.

Further disclosed is a method for producing a medicament comprising the steps of (I) Identifying a modulator of neurodegenorative diseases by a method of the aforementioned screening assays and (ii) admixing the modulator with a pharmaceutical carrier. However, said modulator may also be identifiable by other types of screening assays.

Further disclosed is an assay for testing a compound, preferably for screening a plurality of compounds, for inhibition of binding between a ligand and SULT4A1 protein, or a fragment, or derivative, or variant thereof. Said screening assay comprises the steps of (i) adding a liquid suspension of said SULT4A1 protein, or a fragment, or derivative, or variant thereof, to a plurality of containers, and (ii) adding a compound or a plurality of compounds to be screened for said inhibition to said plurality of containers, and (iii) adding a detectable, preferably a fluorescently labelled ligand to said containers, and (iv) incubating said SULT4A1 protein, or said fragment, or derivative, or variant thereof, and said compound or plurality of compounds, and said detectable, preferably fluorescently labelled ligand, and (v) measuring the amounts of preferably fluorescence associated with said SULT4A1 protein, or with said fragment, or derivative, or variant thereof, and (vi) determining the degree of inhibition by one or more of said compounds of binding of said ligand to said SULT4A1 protein, or said fragment, or derivative, or variant thereof. It might be preferred to reconstitute said SULT4A1 translation product, or fragment, or derivative, or variant thereof into artificial liposomes to generate the corresponding proteoliposomes to determine the inhibition of binding between a ligand and said SULT4A1 translation product. Methods of reconstitution of SULT4A1 translation products from detergent into liposomes have been detailed (Schwarz et al., Biochemistry 1999, 38: 9456-9464; Krivosheev and Usanov, Biochemistry-Moscow 1997, 62: 1064-1073). Instead of utilizing a fluorescently labelled ligand, it might in some aspects be preferred to use any other detectable label known to the person skilled in the art, e.g. radioactive labels, and detect it accordingly. Said method may be useful for the identification of novel compounds as well as for evaluating compounds which have been improved or otherwise optimized in their ability to inhibit the binding of a ligand to a gene product of a gene coding for SULT4A1, or a fragment, or derivative, or variant thereof. One example of a fluorescent binding assay, in this case based on the use of carrier particles, is disclosed and described in patent application WO 00/52451. A further example is the competitive assay method as described in patent WO 02/01226. Preferred signal detection methods for screening assays are described in the following patent applications: WO 96/13744, WO 98/16814, WO 98/23942, WO 99/17086, WO 99/34195, WO 00/66985, WO 01/59436 and WO 01/59416.

Further disclosed is a method for producing a medicament comprising the steps of (i) identifying a compound as an inhibitor of binding between a ligand and a gene product of a gene coding for SULT4A1 by the aforementioned inhibitory binding assay and (ii) admixing the compound with a pharmaceutical carrier. However, said compound may also be identifiable by other types of screening assays.

Further disclosed is an assay for testing a compound, preferably for screening a plurality of compounds to determine the degree of binding of said compounds to SULT4A1 protein, or to a fragment, or derivative, or variant thereof. Said screening assay comprises (i) adding a liquid suspension of said SULT4A1 protein, or a fragment, or derivative, or variant thereof, to a plurality of containers, and (ii) adding a detectable, preferably a fluorescently labelled compound or a plurality of detectable, preferably fluorescently labelled compounds to be screened for said binding to said plurality of containers, and (iii) incubating said SULT4A1 protein, or said fragment, or derivative, or variant thereof, and said detectable, preferably fluorescently labelled compound or detectable, preferably fluorescently labelled compounds, and (iv) measuring the amounts of preferably the fluorescence associated with said SULT4A1 protein, or with said fragment, or derivative, or variant thereof, and (v) determining the degree of binding by one or more of said compounds to said SULT4A1 protein, or said fragment, or derivative, or variant thereof. In this type of assay it might be preferred to use a fluorescent label. However, any other type of detectable label might also be employed. Also in this type of assay it might be preferred to reconstitute a SULT4A1 translation product or fragment, or derivative, or variant thereof into artificial liposomes as described in the present invention.Said assay methods may be useful for the identification of novel compounds as well as for evaluating compounds which have been improved or otherwise optimized in their ability to bind to SULT4A1, or a fragment, or derivative, or variant thereof.

Further disclosed is a method for producing a medicament comprising the steps of (i) identifying a compound as a binder to a gene product of a gene coding for SULT4A1 by the aforementioned binding assays and (ii) admixing the compound with a pharmaceutical carrier. However, said compound may also be identifiable by other types of screening assays.

Further disclosed is a medicament obtainable by any of the methods according to the herein claimed screening assays. Further disclosed is a medicament obtained by any of the methods according to the herein claimed screening assays.

Furthermore, the present invention features the use of protein molecules as shown in SEQ ID NO. 1 and SEQ ID NO. 2, said protein molecules being translation products of the gene coding for SULT4A1, or fragments, or derivatives, or variants thereof, for use as screening targets for reagents or compounds preventing, or treating, or ameliorating a neurodegenerative disease, preferably Alzheimer's disease

The present invention features the use of an antibody - as claimed in claim 5 - which is specifically immunoreactive with an immunogen. The immunogen is disclosed as a translation product of a gene coding for SULT4A1, or a fragment, or derivative, or variant thereof. The immunogen may comprise immunogenic or antigenic epitopes or portions of a translation product of said gene, wherein said immunogenic or antigenic portion of a translation product is a polypeptide, and wherein said polypeptide elicits an antibody response in an animal, and wherein said polypeptide is immunospecifically bound by said antibody. Methods for generating antibodies are well known in the art (see Harlow et al., Antibodies, A Laboratory Manual, Cold Spring Harbor Laboratory Press, Cold Spring Harbor, New York, 1988). The term "antibody", as employed in the present invention, encompasses all forms of antibodies known in the art, such as polyclonal, monoclonal, chimeric, recombinatorial, anti-idiotypic, humanized, or single chain antibodies, as well as fragments thereof (see Dubel and Breitling, Recombinant Antibodies, Wiley-Liss, New York, NY, 1999). Antibodies of the present invention are useful, for instance, in a variety of diagnostic and therapeutic methods, based on state-in-the-art techniques (see Harlow and Lane, Using Antibodies: A Laboratory Manual, Cold Spring Harbor Laboratory Press, Cold Spring Harbor, New York, 1999 and Edwards R., Immunodiagnostics: A Practical Approach, Oxford University Press, Oxford, England, 1999) such as enzyme- immuno assays (e.g. enzyme-linked immunosorbent assay, ELISA), radioimmuno assays, chemoluminescence-immuno assays, Western-blot, immunoprecipitation and antibody microarrays. These methods involve the detection of translation products of a gene coding for SULT4A1, or fragments, or derivatives, or variants thereof.

According to the present invention, said antibodies can be used for detecting the pathological state of a cell in a sample obtained from a subject, comprising immunocytochemical staining of said cell with said antibody, wherein an altered degree of staining, or an altered staining pattern In said cell compared to a cell representing a known health status indicates a pathological state of said cell. Preferably, the pathological state relates to a neurodegenerative disease, in particular to AD. Immunocytochemical staining of a cell can be carried out by a number of different experimental methods well known in the art. It might be preferred, however, to apply an automated method for the detection of antibody binding, wherein the determination of the degree of staining of a cell, or the determination of the cellular or subcellular staining pattern of a cell, or the topological distribution of an antigen on the cell surface or among organelles and other subcellular structures within the cell, are carried out according to the method described in US patent 6150173.

Other features and advantages of the invention will be apparent from the following description of figures and examples which are illustrative only and not intended to limit the remainder of the disclosure in any way.

Figure 1 discloses the initial identification of the differential expression of the gene coding for SULT4A1 in a fluorescence differential display screen. The figure shows a clipping of a large preparative fluorescent differential display gel. PCR products from the frontal cortex (F) and the temporal cortex (T) of two healthy control subjects and six AD patients were loaded in duplicate onto a denaturing polyacrylamide gel (from left to right). PCR products were obtained by amplification of the individual cDNAs with the corresponding one-base-anchor oligonucleotide and the specific Cy3 labelled random primers. The arrow indicates the migration position where significant differences in intensity of the signals for a transcription product of the gene coding for SULT4A1 derived from frontal cortex as compared to the signals derived from the temporal cortex of AD patients and as compared to healthy controls exist. The differential expression reflects a down-regulation of SULT4A1 gene transcription in the temporal cortex compared to the frontal cortex of AD patients and compared to the temporal cortex of Non-AD controls. Comparing the signals derived from temporal cortex and frontal cortex of healthy non-AD control subjects with each other, no difference in signal intensity, i.e. no altered expression level can be detected.

Figures 2 and 3 illustrate the differential expression of the human SULT4A1 gene, in particular of the SULT4A1 splice variant 1 and/or SULT4A1 splice variant 2, In AD brain tissues by quantitative RT-PCR analysis. Quantification of RT-PCR products from RNA samples collected from the frontal cortex (F) and the temporal cortex (T) of AD patients (Figure 2a) and samples from the frontal cortex (F) and the hippocampus (H) of AD patients (Figure 3a) was performed by the LightCycler rapid thermal cycling technique. Likewise, samples of healthy, age-matched control individuals were compared (Figure 2b for frontal cortex and temporal cortex, Figure 3b for frontal cortex and hippocampus). The data were normalized to the combined average values of a set of standard genes which showed no significant differences in their gene expression levels. Said set of standard genes consisted of genes for cyclophilin B, the ribosomal protein S9, the transferrin receptor, GAPDH, and beta-actin. The figures depict the kinetics of amplification by plotting the cycle number against the amount of amplified material as measured by its fluorescence. Note that the amplification kinetics of SULT4A1 splice variant 1 and/or SULT4A1 splice variant 2 cDNAs from both, the frontal and temporal cortices of a normal control individual, and from the frontal cortex and hippocampus of a normal control individual, respectively, during the exponential phase of the reaction are juxtaposed (Figures 2b and 3b, arrowheads), whereas in Alzheimer's disease (Figures 2a and 3a, arrowheads) there is a significant separation of the corresponding curves, indicating a differential expression of the gene coding for SULT4A1, in particular of the SULT4A1 splice variant 1 and/or SULT4A1 splice variant 2, in the respective analyzed brain regions, indicating a dysregulation, preferably a downregulation of a transcription product of the human SULT4A1 gene, in particular of the SULT4A1 splice variant 1 and/or SULT4A1 splice variant 2, or a fragment, or derivative, or variant thereof, in the temporal cortex relative to the frontal cortex, and in the hippocampus relative to the frontal cortex.

Figures 4 and 5 illustrate the differential expression of the human SULT4A1 gene, specifically for the SULT4A1 splice variant 1 (SULT4A1sv1) (Figure 4) and specifically for the SULT4A1 splice variant 2 (SULT4A1sv2) (Figure 5), in AD brain tissues by quantitative RT-PCR analysis. Quantification of RT-PCR products from RNA samples collected from the frontal cortex (F) and the temporal cortex (T) of AD patients (Figures 4a and 5a) were performed by the LightCycler rapid thermal cycling technique. Likewise, samples of healthy, age-matched control individuals were compared (Figures 4b and 5b). The data were normalized to the combined average values of a set of standard genes which showed no significant differences in their gene expression levels. Said set of standard genes consisted of genes for cyclophilin B, the ribosomal protein S9, the transferrin receptor, GAPDH, and beta-actin. The figures depict the kinetics of amplification by plotting the cycle number against the amount of amplified material as measured by its fluorescence. Note that the amplification kinetics of SULT4A1sv1 and of SULT4A1sv2 cDNAs from the frontal and the temporal cortices, respectively, of a normal control individual during the exponential phase of the reaction are juxtaposed (Figures 4b and 5b, arrowheads), whereas in Alzheimer's disease (Figures 4a and 5a, arrowheads) there is a significant separation of the corresponding curves, indicating a differential expression of the gene coding for SULT4A1sv1 (Figure 4) and of SULT4A1sv2 (Figure 5), in the respective analyzed brain regions, further indicating a dysregulation, preferably a downregulation of a transcription product of human SULT4A1sv1 and of SULT4A1sv2 in the temporal cortex relative to the frontal cortex.

Figures 6 and 7 show the analysis of absolute mRNA expression of SULT4A1sv1 and/or SULT4A1sv2 (Figure 6) and specifically of SULT4A1sv1 (Figure 7) by comparison of control and AD stages using statistical method of the median at 98%-confidence level. The data were calculated by defining control groups including subjects with either Braak stages 0 to 1, Braak stages 0 to 2, or Braak stages 0 to 3 which are compared with the data calculated for the defined AD patient groups including Braak stages 2 to 6, Braak stages 3 to 6 and Braak stages 4 to 6, respectively. Additionally, three groups including subjects with either Braak stages 0 to 1, Braak stages 2 to 3 and Braak stages 4 to 6, rspectively, were compared with each other. A significant difference was detected comparing frontal cortex (F) and inferior temporal cortex (T) of AD patients and of control persons with each other. Said difference reflects a strong down-regulation of SULT4A1 in the temporal cortex of AD patients relative to the temporal cortex of control persons and a down-regulation of SULT4A1 in the temporal cortex of AD patients compared to their frontal cortices. The differences reflect as well a down-regulation of SULT4A1 in the frontal cortex of AD patients compared to the frontal cortex of control group subjects. The Braak stages correlate with the progressive course of AD disease which, as shown in the instant invention, is associated with an increasing difference in the regulation, the level and the activity of SULT4A1 as described above. Said significant differences were observed already at Braak stage 3.

Figure 8 discloses SEQ ID NO. 1, the amino acid sequence of human SULT4A1 splice variant 1 comprising 284 amino acids (Genbank accession number 043728).

Figure 9 discloses SEQ ID NO. 2, the polypeptide sequence of human SULT4A1 splice variant 2, comprising 171 amino acids. The protein SULT4A1 splice variant 2 differs from SULT4A1 splice variant 1, in that it lacks 114 amino acids (amino acid 57 to 170) of SEQ ID NO. 1 (Genbank accession number 043728) and harbours an additional amino acid at position 57 of SEQ ID NO. 2.

Figure 10 represents SEQ ID NO. 3, the nucleotide sequence of human SULT4A1 splice variant 1 cDNA (Genbank accession number AF176342), comprising 2419 nucleotides.

Figure 11 represents SEQ ID NO. 4, the nucleotide sequence of human SULT4A1 splice variant 2 cDNA (Genbank accession number AF176342 missing nucleotides 1 90 to 528, represented by the EST bi550483 and bm805353), comprising 2080 nucleotides.

Figure 12 depicts SEQ ID NO. 5, the nucleotide sequence of the 32 bp SULT4A1 cDNA fragment, identified and obtained by fluorescence differential display and subsequent cloning (sequence in 5' to 3' direction).

Figure 13 shows the nucleotide sequence of SEQ ID NO. 6, the coding sequence (cds) of the human SULT4A1 gene, comprising 855 nucleotides, harbouring nucleotides 21 to 875 of SEQ ID NO. 3.

Figure 14 outlines the sequence alignment of SEQ ID NO. 5, the 32 bp human SULT4A1 cDNA fragment, with the nucleotide sequence of the human SULT4A1 splice variant 1 cDNA (Genbank accession number AF176342, SEQ ID NO. 3, nucleotides 2335 to 2366) and SULT4A1 splice variant 2 cDNA (SEQ ID NO. 4, nucleotides 1996 to 2027)

Figures 15 and 16 list the expression levels of SULT4A1 sv1 and/or SULT4A1sv2 (Figure 15) and the expression levels of specially SULT4A1sv1 (Figure 16) in the temporal cortex relative to the frontal cortex in fifteen AD patients, herein identified by internal reference numbers P010, P011, P012, P014, P016, P017, P019, P038, P040, P041, P042, P046, P047, P048, P049 (1.27-7.14 fold for SULT4A1sv1 and/or sv2 and 1.10-16.67 fold for SULT4A1sv1 only; reciprocal values according to the formula described below) and twentyfive age-matched control individuals, herein identified by internal reference numbers C005, C008, C011, C012, C014, C025, C026, C027, C028, C029, C030, C031, C032, C033, C034, C035, C036, C038, C039, C041, C042, DE02, DE03, DE05, DE07 (0.42-2.5 fold for SULT4A1sv1 and/or sv2 and 2.89-3.33 fold for SULT4A1sv1 only; reciprocal values according to the formula described below). For an up-regulation in the temporal cortex, the values shown are calculated according to the formula described herein (see below) and in case of an up-regulation in the frontal cortex the reciprocal values are calculated, respectively. The bar diagram visualizes individual natural logarithmic values of the temporal to frontal cortex, In(IT/IF), and of the frontal to temporal cortex regulation factors, In(IF/IT), in different Braak stages (0 to 6).

Figure 17 lists the gene expression levels in the hippocampus relative to the frontal cortex for the SULT4A1 gene (splice variant 1 and/or splice variant sv2) in six Alzheimer's disease patients, herein identified by internal reference numbers P010, P011, P012, P014, P016, P019 (0.13 to 1.37 fold) and three healthy, age-matched control individuals, herein identified by internal reference numbers C004, C005, C008 (0.56 to 0.82 fold). The values shown are calculated according to the formula described herein (see below). The scatter diagram visualizes individual logarithmic values of the hippocampus to frontal cortex regulation ratios (log (ratio HC/IF)) in control samples (dots) and in AD patient samples (triangles).

Figure 18 shows the immunofluorescence analysis of H4APPsw control cells and H4APPsw cells stably over-expressing the myc-tagged SULT4A1 protein (H4APPsw-SULT4A1-myc). The SULT4A1-myc protein was detected with a rabbit anti-myc antibody (Mobitec) and a Cy3-conjugated anti-rabbit antibody (Amersham) (Figure 18A and B). The cellular nucleus was stained with DAPI (Figure 18C and D). The overlay analysis indicate that the SULT4A1-myc protein is localized to the cytoplasm (Figure 18 E) and is over-expressed in more than 50% of the H4APPsw-SULT4A1-myc transduced cells as compared to the H4APPsw control cells (Figure 18 F).

Figure 19 indicates that the over-expression of SULT4A1-myc protein renders H4-APPsw cells to be more resistant of trophic factor deprivation than H4APPsw control cells. Both cell types have been incubated for 40 hours in cell culture media that contained serum concentrations ranging from 0 to 7.5%. The cellular response to trophic factor deprivation was determined by applying the REDOX indicator AlamarBlue (BioSource) and measuring the relative fluorescence (RFU). Th e % toxicity was calculated according to the formula as given below (Example (vi)). The graph represents mean values from toxicity calculations obtained from two independent experiments.

Figure 20 schematically depicts the targeting strategy used to generate SULT4A1 deficient (knockout) mice. Mouse Sult4A1 gene comprises of seven exons indicated as boxes with roman numbers (I-VII). The targeting vector consists of a short homology arm sequence (SA) of about 2.9 kb and a long homology arm sequence (LA) of about 7 kb. A Neo cassette (Neomycin resistance gene driven by the Pgk promotor) is inserted into exon 2, substituting a main part of exon 2 as well as exons 3, 4 and 5 of the Sult4A1 gene. The Neo selection marker is flanked by FRT sites (recognition sites for FLP recombinase) enabling removal of the Neo cassette by FLP recombinase (site-specific recombinase) if needed. At the 3' site of the Neo cassette a splice acceptor and poly A signal sequence (pA) will guarantee termination of the transcript also in case the selection marker is removed. Pgk (constitutive mouse phoshoglycerate kinase-1 promotor), Neo (Neomycin resistance gene, Neomycinphosphotransferase)

Fig ure 21 schematically depicts the targeting strategy used to generate transgenic mice expressing human Sult4A1. The targeting vector is composed of the transgene and Rosa26 homology sequences in order to integrate the transgene into the Rosa26 gene locus (mouse Rosa beta geo 26 gene). The short homology arm sequence (SA) consists of approximately 1.1 kb, the long homology arm sequence (LA) consists of about 4.3 kb. The three exons of the Rosa26 gene are indicated with roman numbers (I-III). The transgene is composed of the open reading frame of the human Sult4A1 (ORF SULT4A1) which is located in the mu rine Thy1.2 expression cassette containing regulatory sequences of the brain-specific Thyl.2 gene. 5'prime of the transgene a Neomycin resistance gene (Neo) and a splice acceptor are located allowing expression of the Neo selection marker driven by the Rosa promoter from the endogenous Rosa26 gene. 3'prime of the Neo marker a poly A signal (pA) will stop transcription.

Figure 22 shows the detection of SULT4A1sv1 expression in three different SULT4A1sv1 transgenic fly lines under the control of gmr-GAL4 by RT-PCR using SULT4A1sv1 specific primers. Genotypes used were: *w; UAS-SULT4A1sv1#3 /gmr-GAL4; w; UAS- SULT4A1sv1#8lgmr-GAL4; w; UAS- SULT4A1sv1#22/+; gmr-GAL4/+.* The melting temperatures of RT-PCR products of wild-type and SULT4A1sv1 expressing flies are illustrated in Figure 22A. Figure 22B depicts the comparison of the expression efficiency of three different SULT4A1sv1 expressing fly lines. The efficiency is calculated according to the cycle number and efficiency of the RT-PCR reaction of the SULT4A1 splice variant 1 specific primer pair. Measurements were done in triplicate for each genotype. Genotypes used were: *w; UAS- SULT4A1sv1#3/gmr-GAL4; w; UAS- SVLT4A1sv1#8/gmr-GAL4; w; UAS-SULT4A1sv1#22/+; gmr-GAL4/+.*

Figure 23 SULT4A1 rescues photoreceptor cell degeneration in flies expressing hAPP and hBACE under the control of gmr-GAL4. 10μmCryostat adult brain sections were stained with a photoreceptor cell specific antibody 24B10 at 3, 8, 16 and 19 days after eclosion. Expression of SULT4A1sv1#3 (Figure 23A) and SULT4A1sv1#8 (Figure 23B) rescues photoreceptor cell degeneration as judged by the diameter of the retina (white arrows) whereas SULT4A1sv1#22 shows no effect on the degenerative phenotype (Figure 23C). Expression of SULT4A1sv1#3, #8 and #22 alone under the control of gmr-GAL4 is wild-type (left column in Figure 23A, B and C). Genotypes used were: *w; UAS-SULT4A1sv1#3/gmr-GAL4 - w; UAS-SULT4A1sv1#8/gmr-GAL4 - w; UAS- SULT4A1sv1#22/+; gmr-GAL4/+ - w; UAS-hBACE, UAS-hAPP/+; UAS- SULT4A1sv1#3/gmr-GAL4 - w; UAS-hBACE, UAS-hAPP/+; UAS-SULT4A1sv1#8/gmr-GAL - w; UAS-SULT4A1sv1#22/hAPP, hBACE; gmr-GAL4/+:* Re: retina; La: lamina; Me: medulla.

Figure 24 depicts the Western blots of head homogenates of flies expressing hAPP/hBACE or in combination with SULT4A1sv1#3 (Figure 24A). Equal amounts of protein were loaded in each lane. Blot was probed with anti hAPP N-terminal specific antibody 22C11. The antibody detects the full-length and beta-cleaved N-terminal fragment of hAPP. Immunoprecipitation using the Abeta N-terminal specific antibody 6E10 for the precipitation and detection on Western blots is shown in Figure 24B. Abeta-peptides are detected predominantly in oligomeric forms.

Figure 25 Thioflavin S positive plaques on paraffin sections through the retina of flies expressing hAPP/hBACE/DPsnL235P or hAPP/hBACE/DPsnL235P/ SULT4A1sv1#3 (indicated by a star). No difference in the onset of plaque formation was detected between control flies and hAPP/hBACE/DPsnL235P/ SULT4A1sv1#3 expressing flies. *w; UAS-hBACE, UAS-hAPP/+; gmr-GAL, UAS-DPsnL235P/+ and w; UAS-hBACE, UAS-hAPP/+; UAS-SULT4A1sv1#3/gmr-GAL4, UAS-Dpsnl235P.*

EXAMPLES:

(i) Brain tissue dissection from patients with AD:

Brain tissues from AD patients and age-matched control subjects were collected within 6 hours post-mortem and immediately frozen on dry ice. Sample sections from each tissue were fixed in paraformaldehyde for histopathological confirmation of the diagnosis. Brain areas for differential expression analysis were identified and stored at -80 °C until RNA extractions were performed.

(ii) Isolation of total mRNA:

Total RNA was extracted from post-mortem brain tissue by using the RNeasy kit (Qiagen) according to the manufacturer's protocol. The accurate RNA concentration and the RNA quality were determined with the DNA LabChip system using the Agilent 2100 Bioanalyzer (Agilent Technologies). For additional quality testing of the prepared RNA, i.e. exclusion of partial degradation and testing for DNA contamination, specifically designed intronic GAPDH oligonucleotides and genomic DNA as reference control were utilised to generate a melting curve with the LightCycler technology as described in the supplied protocol by the manufacturer (Roche).

(iii) cDNA synthesis and identification of differentially expressed genes by fluorescence differential display (FDD):

In order to identify changes in gene expression in different tissues we employed a modified and improved differential display (DD) screening method. The original DD screening method is known to those skilled in the art (Liang and Pardee, Science 1995, 267: 1186-7). This technique compares two populations of RNA and provides clones of genes that are expressed in one population but not in the other. Several samples can be analyzed simultaneously and both up- and down-regulated genes can be identified in the same experiment. By adjusting and refining several steps in the DD method as well as modifying technical parameters, e.g. increasing redundancy, evaluating optimized reagents and conditions for reverse transcription of total RNA, optimizing polymerase chain reactions (PCR) and separation of the products thereof, a technique was developed which allows for highly reproducible and sensitive results. The applied and improved DD technique was described in detail by von der Kammer et al. (Nucleic Acids Research 1999, 27: 2211-2218). A set of 64 specifically designed random primers was developed (standard set) to achieve a statistically comprehensive analysis of all possible RNA species. Further, the method was modified to generate a preparative DD slab-gel technique, based on the use of fluorescently labelled primers, In the present invention, RNA po pulations from carefully selected post-mortem brain tissues (frontal and temporal co rtex) of AD patients and age-matched control subjects were compared.

As starting material for the DD analysis we used total RNA, extracted as described above (ii). Equal amounts of 0.05 µg RNA each were transcribed into cDNA in 20 µl reactions containing 0.5 mM each dNTP, 1 µl Sensiscript Reverse Transcriptase and 1x RT buffer (Qiagen), 10 U RNase inhibitor (Qiagen) and 1 µM of either one-base-anchor oligonucleotides $HT_{11}A$, $HT_{11}G$ or $HT_{11}C$ (Liang et al., Nucleic Acids Research 1994, 22: 5763-5764; Zhao et al., Biotechniques 1995, 18: 842-850). Reverse transcription was performed for 60 min at 37 °C with a final denaturation step at 93 °C for 5 min. 2 µl of the obtained cDNA each was subjected to a polymerase chain reaction (PCR) employing the corresponding one-base-anchor oligonucleotide (1 µM) along with either one of the Cy3 labelled random DD primers (1 µM), 1x GeneAmp PCR buffer (Applied Biosystems), 1.5 mM $MgCl_2$ (Applied Biosystems), 2 µM dNTP-Mix (dATP, dGTP, dCTP, dTTP Amersham Pharmacia Biotech), 5 % DMSO (Sigma), 1 U AmpliTaq DNA Polymerase (Applied Biosystems) in a 20 µl final volume. PCR conditions were set as follows: one round at 94 °C for 30 sec for denaturing, cooling 1 °C/sec down to 40 °C, 40 °C for 4 min for low-stringency annealing of primer, heating 1 °C/sec up to 72 °C, 72 °C for 1 min for extension. This round was followed by 39 high-stringency cycles: 94 °C for 30 sec, cooling 1 °C/sec down to 60 °C, 60 °C for 2 min, heating 1 °C/sec up to 72 °C, 72 °C for 1 min. One final step at 72 °C for 5 min was added to the last cycle (PCR cycler: Multi Cycler PTC 200, MJ Research). 8 µl DNA loading buffer were added to the 20 µl PCR product preparation, denatured for 5 min and kept on ice until loading onto a gel. 3.5 µl each were separated on 0.4 mm thick, 6 % polyacrylamide (Long Ranger)/ 7 M urea sequencing gels in a slab-gel system (Hitachi Genetic Systems) at 2000 V, 60W, 30 mA, for 1 h 40 min. Following completion of the electrophoresis, gels were scanned with a FMBIO II fluorescence-scanner (Hitachi Genetic Systems), using the appropriate FMBIO II Analysis 8.0 software. A full-scale picture was printed, dfferentially expressed bands marked, excised from the gel, transferred into 1.5 ml containers, overlayed with 200 µl sterile water and kept at -20°C until extraction.

Elution and reamplification of DD products: The differential bands were extracted from the gel by boiling in 200 µl $H_2O$ for 10 min, cooling down on ice and precipitation from the supernatant fluids by using ethanol (Merck) and glycogen/sodium acetate (Merck) at -20 °C over night, and subsequent centrifugation at 13.000 rpm for 25 min at 4 °C. Pellets were washed twice in ice-cold ethanol (80%), resuspended in 10 mM Tris pH 8.3 (Merck) and dialysed against 10 % glycerol (Merck) for 1 h at room temperature on a 0.025 μm VSWP membrane (Millipore). The obtained preparations were used as templates for reamplification by 15 high-stringency cycles in 25-μl PCR mixtures containing the corresponding primer pairs as used for the DD PCR (see above) under identical conditions, with the exception of the initial round at 94 °C for 5 min, followed by 15 cycles of: 94 °C for 45 sec, 60 °C for 45 sec, ramp 1 °C/sec to 70 °C for 45 sec, and one final step at 72 °C for 5 min.

Cloning and sequencing of DD products: Re-amplified cDNAs were analyzed with the DNA LabChip system (Agilent 2100 Bioanalyzer, Agilent Technologies) and ligated into the pCR-Blunt II-TOPO vector and transformed into *E.coli* Top10F' cells (Zero Blunt TOPO PCR Cloning Kit, Invitrogen) according to the manufacturer's instructions. Cloned cDNA fragments were sequenced by commercially available sequencing facilities. The result of one such FDD experiment for the SULT4A1 gene is shown in Figure 1.

(iv) Confirmation of differential expression by quantitative RT-PCR:

Positive corroboration of differential SULT4A1 gene expression, SULT4A1 sv1 and/or sv2 and SULT4A1 sv1, respectively, in the temporal cortex versus frontal cortex and in the hippocampus versus frontal cortex was performed using the LightCycler technology (Roche). This technique features rapid thermal cyling for the polymerase chain reaction as well as real-time measurement of fluorescent signals during amplification and therefore allows for highly accurate quantification of RT-PCR products by using a kinetic, rather than endpoint readout. The ratios of SULT4A1 cDNAs from the temporal cortices of AD patients and of healthy age-matched control individuals, from the frontal cortices of AD patients and of healthy age-matched control individuals, from the hippocampi of AD patients and of healthy age-matched control individuals, and the ratios of SULT4A1 cDNAs from the temporal cortex and frontal cortex of AD patients and of healthy age-matched control individuals, and the ratios of SULT4A1 cDNAs from the hippocampus and frontal cortex of AD patients and of healthy age-matched control individuals, respectively, were determined (relative quantification).

The mRNA expression profiling between frontal cortex tissue (F) and inferior temporal cortex tissue (T) of SULT4A1sv1 and/or sv2 and of SULT4A1sv1 has been analyzed in four up to nine tissues per Braak stage. Because of the lack of high quality tissues from one donor with Braak 3 pathology, tissues of one additional donor with Braak 2 pathology were included, and because of the lack of high quality tissues from one donor with Braak 6 pathology, tissue samples of one additional donor with Braak 5 pathology were included.

For the analysis of the profiling, two general approaches have been applied. Both comparative profiling studies, frontal cortex against inferior temporal cortex as well as control against AD patients, which contribute to the complex view of the relevance of SULT4A1, of SULT4A1sv1 and of SULT4A1sv2, respectively, in AD physiology, are shown in detail below. 1) Relative comparison of the mRNA expression between frontal cortex tissue and inferior temporal cortex tissue of controls and of AD patients.

This approach allowed to verify that the identified gene SULT4A1 (SULT4A1sv1, SULT4A1sv2) is either involved in the protection of the less vulnerable tissue (frontal cortex) against degeneration, or is involved in or enhances the process of degeneration in the more vulnerable tissue (inferior temporal cortex).

First, standard curves were generated to determine the efficiency of the PCR with primers for the SULT4A1 splice variant 1 and/or splice variant 2 gene (primers not discriminating between SULT4A1sv1 and SULT4A1sv2):

5'-CAAAGTGGTGGTCAGGAGGGT-3' (SEQ ID NO. 3, nucleotides 2064-2084; SEQ ID NO. 4, nucleotides 1725-1745) and
5'-CCGTTTCAAATACAGCACCAAG-3' (SEQ ID NO. 3, nucleotides 2110-2131; SEQ ID NO. 4, nucleotides 1771-1792);

and with specific primers for the SULT4A1 splice variant 1 gene only:

5'-CTGACCCCGATGAGATCG-3' (SEQ ID NO. 3, nucleotides 235-252) and
5'-GGCAGGTGGCTCTTGATGA-3' (SEQ ID NO. 3, nucleotides 340-358);

and with specific primers for the SULT4A1 splice variant 2 gene only:

5'-TCACCTACCCCAAGTCCGT-3' (SEQ ID NO. 4, nucleotides 172-190) and
5'-TTCATACTTGAGAAAAAGCACGT-3' (SEQ ID NO. 4, nucleotides 250-272).

PCR amplification (95 °C and 1 sec, 56 °C and 5 sec, and 72 °C and 5 sec) was performed in a volume of 20 μl containing LightCycler-FastStart DNA Master SYBR Green I mix (contains FastStart Taq DNA polymerase, reaction buffer, dNTP mix with dUTP instead of dTTP, SYBR Green I dye, and 1 mM $MgCl_2$; Roche), 0.5 μM primers, 2 μl of a cDNA dilution series (final concentration of 40, 20, 10, 5, 1 and 0.5 ng human total brain cDNA; Clontech) and, depending on the primers used, additional 3 mM $MgCl_2$. Melting curve analysis revealed each a single peak with no visible primer dimers at approximately 84°C for the SULT4A1sv1 and/or SULT4A1sv2 gene primers, at 87.5°C for the SULT4A1sv1 gene specific primers and at 87.2°C for the SULT4A1sv2 gene specific primers, respectively. Quality and size of the PCR product were determined with the DNA LabChip system (Agilent 2100 Bioanalyzer, Agilent Technologies). Single peaks at the expected sizes of 68 bp for the SULT4A1sv1 and/or SULT4A1 sv2 gene, at 124 bp for the SULT4A1sv1 gene and at 101 bp for the SULT4A1 sv2 gene, respectively, were observed in the electropherogram of the sample.

In an analogous manner, the PCR protocol was applied to determine the PCR efficiency of a set of reference genes which were selected as a reference standard for quantification. In the present invention, the mean value of five such reference genes was determined: (1) cyclophilin B, using the specific primers 5'-ACTGAAGCACTACGGGCCTG-3' and 5'-AGCCGTTGGTGTCTTTGCC-3' except for $MgCl_2$ (an additional 1 mM was added instead of 3 mM). Melting curve analysis revealed a single peak at approximately 87 °C with no visible primer dimers. Agarose gel analysis of the PCR product showed one single band of the expected size (62 bp). (2) Ribosomal protein S9 (RPS9), using the specific primers 5'-GGTCAAATTTACCCTGGCCA-3' and 5'-TCTCATCAAGCGTCAGCAGTTC-3' (exception: additional 1 mM $MgCl_2$ was added instead of 3 mM). Melting curve analysis revealed a single peak at approximately 85°C with no visible primer dimers. Agarose gel analysis of the PCR product showed one single band with the expected size (62 bp). (3) beta-actin, using the specific primers 5'-TGGAACGGTGAAGGTGACA-3' and 5'-GGCAAGGGACTTCCTGTAA-3'. Melting curve analysis revealed a single peak at approximately 87°C with no visible primer dimers. Agarose gel analysis of the PCR product showed one single band with the expected size (142 bp). (4) GAPDH, using the specific primers 5'-CGTCATGGGTGTGAACCATG-3' and 5'-GCTAAGCAGTTGGTGGTGCAG-3'. Melting curve analysis revealed a single peak at approximately 83°C with no visible primer dimers. Agarose gel analysis of the PCR product showed one single band with the expected size (81 bp). (5) Transferrin receptor TRR, using the specific primers 5'-GTCGCTGGTCAGTTCGTGATT-3' and 5'-AGCAGTTGGCTGTTGTACCTCTC-3'. Melting curve analysis revealed a single peak at approximately 83°C with no visible primer dimers. Agarose gel analysis of the PCR product showed one single band with the expected size (80 bp).

For calculation of the values, first the logarithm of the cDNA concentration was plotted against the threshold cycle number $C_t$ for SULT4A1, i.e. for SULT4A1sv1 and/or SULT4A1sv2. for SULT4A1 splice variant 1 only, and for the SULT4A1 splice variant 2 only, respectively, and the five reference standard genes. The slopes and the intercepts of the standard curves (i.e. linear regressions) were calculated for all genes. In a second step, cDNAs from frontal cortices of AD patients and of healthy control individuals, from temporal cortices of AD patients and of healthy control individuals, from hippocampi of AD patients and of healthy control individuals, and cDNAs from the frontal cortex and the temporal cortex of AD patients and of control individuals and from the frontal cortex and the hippocampus of AD patients and of control individuals, respectively, were analyzed in parallel and normalized to cyclophilin B. The $C_t$ values were measured and converted to ng total brain cDNA using the corresponding standard curves:

$$10 \wedge ( (C_t \text{ value - intercept}) / \text{slope} ) \quad [\text{ng total brain cDNA}]$$

The values for temporal and frontal cortex SULT4A1 cDNAs (of SULT4A1sv1 and/or SULT4A1sv2, of SULT4A1sv1 and of SULT4A1sv2), the values for hippocampus and frontal cortex SULT4A1 cDNAs, and the values from the frontal cortex SULT4A1 cDNAs of AD patients (P) and control individuals (C), and the values for temporal cortex SULT4A1 cDNAs of AD patients (P) and of control individuals (C), were normalized to cyclophilin B, and the ratios were calculated according to formulas:

$$\text{Ratio} = \frac{\text{SULT4A1 temporal [ng] / cyclophilin B temporal [ng]}}{\text{SULT4A1 frontal [ng] / cyclophilin B frontal [ng]}}$$

$$\text{Ratio} = \frac{\text{SULT4A1 hippocampus [ng] / cyclophilin B hippocampus [ng]}}{\text{SULT4A1 frontal [ng] / cyclophilin B frontal [ng]}}$$

$$\text{Ratio} = \frac{\text{SULT4A1 (P) temporal [ng] / cyclophilin B (P) temporal [ng]}}{\text{SULT4A1 (C) temporal [ng] / cyclophilin B (C) temporal [ng]}}$$

$$\text{Ratio} = \frac{\text{SULT4A1 (P) frontal [ng] / cyclophilin B (P) frontal [ng]}}{\text{SULT4A1 (C) frontal [ng] / cyclophilin B (C) frontal [ng]}}$$

In a third step, the set of reference standard genes was analyzed in parallel to determine the mean average value of the AD patient to control person temporal cortex ratios, of the AD patient to control person frontal cortex ratios, and of the temporal to frontal ratios of AD patients and control persons and the hippocampi to frontal ratios of AD patients and control persons, respectively, of expression levels of the reference standard genes for each individual brain sample. As cyclophilin B was analyzed in step 2 and step 3, and the ratio from one gene to another gene remained constant in different runs, it was possible to normalize the values for SULT4A1, i.e. for SULT4A1sv1 and/or SULT4A1 sv2, for SULT4A1sv1 only, and for SULT4A1 sv2 only, respectively, to the mean average value of the set of reference standard genes instead of normalizing to one single gene alone. The calculation was performed by dividing the respective ratio shown above by the deviation of cyclophilin B from the mean value of all housekeeping genes. The results of such quantitative RT-PCR analysis and the respective calculated values for the gene coding for the SULT4A1 splice variant 1 and/or splice variant 2, for the SULT4A1 splice variant 1 only, and for the SULT4A1 splice variant 2 only, are shown in Figures 2, 3 and 15, in Figures 4 and 16, and in Figure 5, respectively.

2) Comparison of the mRNA expression between controls and AD patients.

For this analysis it was proven that absolute values of real-time quantitative PCR (Lightcycler method) between different experiments at different time points are consistent enough to be used for quantitive comparisons without usage of calibrators. Cyclophilin was used as a standard for normalization in any of the qPCR experiments for more than 100 tissues. Between others it was found to be the most consistently expressed housekeeping gene in our normalization experiments. Therefore a proof of concept was done by using values that were generated for cyclophilin.

First analysis used cyclophilin values from qPCR experiments of frontal cortex and inferior temporal cortex tissues from three different donors. From each tissue the same cDNA preparation was used in all analyzed experiments. Within this analysis no normal distribution of values was achieved due to small number of data. Therefore the method of median and its 98 %-conficence level was applied. This analysis revealed a middle deviation of 8.7 % from the median for comparison of absolute values and a middle deviation of 6.6 % from the median for relative comparison.

Second analysis used cyclophilin values from qPCR experiments of frontal cortex and inferior temporal cortex tissues from two different donors each, but different cDNA preparations from different time points were used. This analysis revealed a middle deviation of 29.2 % from the median for comparison of absolute values and a middle deviation of 17.6 % from the median for relative comparison. From this analysis it was concluded, that absolute values from qPCR experiments can be used, but the middle deviation from median should be taken into further considerations. A detailed analysis of absolute values for SULT4A1 was performed. Therefore, absolute levels of SULT4A1 were used after relative normalization with cyclophilin. The median as well as the 98 %-confidence level was calculated for the control group (Braak 0 - Braak 3) and the patient group (Braak 4 - Braak 6), respectively. Same analysis was done redefining the control group (Braak 0 - Braak 2) and the patient group (Braak 3 - Braak 6) as well as redefining the control group (Braak 0 - Braak 1) and the patient group (Braak 2 - Braak 6). The latter analysis was aimed to identify early onset of mRNA expression differences between controls and AD patients. In another view of this analysis, three groups comprising Braak stages 0-1, Braak stages 2-3, and Braak stages 4-6, respectively, were compared to each other in order to identify tendencies of gene expression regulation as well as early onset differences. Said analysis as described above are shown in Figures 6 and 7.

(v) Immunofluorescence Analysis (IF):

For the immunofluorescence staining of SULT4A1 protein in cells, a human neuroglioma cell line was used (H4 cells) which stably expresses the human APP695 isoform carrying the Swedish mutation (K670N, M671 L) (H4APPsw cells). The H4APPsw cells were transduced with a pFB-Neo vector (Stratagene, #217561, 6.6 kb) containing the coding sequence of SULT4A1 (SEQ ID NO. 6, 855 bp) (pFB-Neo-SULT4A1cds-myc, SULT4A1 vector, 7483 bp) and a myc-tag. For the generation of the SULT4A1 vector, the SULT4AIcds-myc sequence was introduced into the XhoI-blunt/ BamHI restriction sites of the multiple cloning site (MCS) of the pFB-Neo vector. For transduction of the H4APPsw cells with the SULT4A1 vector the retroviral expression system ViraPort from Stratagene was used.

The myc-tagged SULT4A1 over-expressing cells (H4APPsw-SULT4A1-myc) were seeded onto glass cover slips in a 24 well plate (Nunc, Roskilde, Denmark; #143982) at a density of $5x10^4$ cells and incubated at 37°C at 5% $CO_2$ over night. To fix the cells onto the cover slip, medium was removed and chilled methanol (-20°C) was added. After an incubation period of 15 minutes at -20°C, methanol was removed and the fixed cells were blocked for 1 hour in blocking solution (200μl PBS/ 5% BSA/ 3% goat serum) at room temperature. The first antibody (polyclonal anti-myc antibody, rabbit, 1:500, Mobitec) and DAPI (DNA-stain, 0.05μg/m), 1 :1000) in PBS / 1% goat serum was added and incubated for 1 hour at room temperature. After removing the first antibody, the fixed cells were washed 3 times with PBS for 5 minutes. The second antibody (Cy3-conjugated anti-rabbit antibody, 1 :1000, Amersham Pharmacia, Germany) was applied in blocking solution and incubated for 1 hour at room temperature. The cells were washed 3 times in PBS for 5 minutes. Coverslips were mounted onto microscope slides using Permafluor (Beckman Coulter) and stored over night at 4°C to harden the mounting media. Cells were visualized using microscopic dark field epifluorescence and bright field phase contrast illumination conditions (IX81, Olympus Optical). Microscopic images (Figure 18) were digitally captured with a PCO SensiCam and analysed using the appropriate software (AnalySiS, Olympus Optical).

(vi) Cytotoxicity/ Viability Assay:

To analyse the effect of trophic factor deprivation on SULT4A1 protein over-expressing cells (SULT4A1 cells described above in Example (v)), H4APPsw control and H4APPsw-SULT4A1-myc over-expressing cells were seeded with a density of $1.5x10^4$ cells/ml in 96 well plates in DMEM (Simga), 10% FCS (Gibco) and Penicillin/Streptomycin (Gibco). After incubating over night at 37°C under $CO_2$ conditions (8.5%), medium was exchanged against DMEM with Penicillin/ Streptomycin. As a positive control for toxicity, 12 concentrations of chloroquine (Sigma) ranging from 0.06-10 mg/ml were added to control cells and to H4APPsw-SULT4A1-myc over-expressing cells (in triplicate). As a negative control for toxicity, DMEM (Simga), 10% FCS (Gibco) and Penicillin/Streptomycin (Gibco) was applied to 24 wells of control cells and H4APPsw-SULT4A1-myc over-expressing cells. For trophic factor deprivation, increasing serum concentrations (0, 0.3, 0.6, 1.25, 2.5, 5% and 7.5%) in DMEM, Penicillin/Steptomycin were added to control cells and H4APPsw-SULT4A1-myc over-expressing cells (in triplicate). After incubation for 40 hours at 37°C under $CO_2$ conditions (8.5%), the REDOX indicator AlamarBlue (BioSource) was added and the relative fluorescence (RFU) was measured 4 hours later at 544nm (excitation) and 590nm (emission) using a BMG Fluostar reader. The % of toxicity was calculated with RFU mean values using the following formula:

$$\% \text{ Toxicity} = \frac{100 \times (\text{RFU negative control} - \text{RFU toxic stimuli})}{(\text{RFU negative control} - \text{RFU positive control})}$$

Graphs have been determined by using GraphPad Prism (non-linear regression curve fit with sigmoidal dose response and variable slope).

(vii) Generation of transgenic and knockout mice expressing Sult4A1:

Sult4A1 deficient mice were generated using the Sult4A1 construct as described below. A homologue of the human SULT4A1 gene has been found in the mouse. It is the mouse sulfotransferase family 4A member 1 (Sult4A1), also named S4A1 (Unigene ID: Mm. 248796; gene ID: 29859, locus NT_039621). The mouse Sult4A1 gene is located on chromosome 15 and consists of 7 exons encoding a protein of 284 amino acids and shares 98% identity to the human SULT4A1 sequence as shown in SEQ ID NO.1. For the mouse Sult4A1 gene no splice variants have been found so far. In humans additional splice variants of the SULT4A1 gene have been discovered. The human SULT4A1 splice variant 1 and splice variant 2 are disclosed herein. It cannot be ruled out that these splice variants also exist in the murine system as they share some homology with the mouse gene. The functional domain of the mouse Sult4A1 protein is a sulfotransferase domain which is encoded from exons 1-7. Within that domain three conserved amino acid motifs of sulfotransferases (Falany et al, Biochem J. 2000, 857-864) can be found which are located on exons 1, 3 and 5.

For the construction of a Sult4A1 gene targeting vector, mouse Sult4A1 homology sequences were amplified from genomic C57BL/6J DNA (mouse strain C57BL/6J) using standard PCR protocols. The short homology arm (SA) consists of approximately 2.9 kb (intronic sequences between exons 1 and 2) and the long horology arm (LA) consists of about 7 kb. The enzym atic function of Sult4A1 was disrupted by the insertion of a Neo cassette (Neomycinphosphotransferase, Neomycin resistance gene driven by the Pgk promotor for clone selection, selectable marker sequence) into exon 2, substituting a main part of exon 2 as well as exons 3, 4 and 5 of the endogenous mouse SULT4A1 gene sequence. The constructed targeting vector and the targeting strategy is shown in Figure 20. The Neo cassette replaces two highly conserved amino acid motifs as outlined above and thereby disrupts the sulfotransferase domain. The Neo selection marker is driven by the PGK promoter and is flanked by FRT sites (recognition sites for FLP recombinase) the enabling removal of the Neo cassette by FLP recombinase (site-specific recombinase) if needed. At the 3' site of the Neo cassette a splice acceptor and poly A signal (pA) will guarantee termination of the transcript also in case the selection marker is removed. Thus, only exons 1 and a part of exon 2 are transcribed which are about 25 amino acids of the sulfotransferase domain. Exons 6 and 7, although still in frame, are not transcribed as the splice acceptor and poly A motif terminate transcription resulting in a functional knockout of the gene. In addition, by replacing exons 2 to 5 functional alternative splice forms as observed in humans are prevented.

The constructed targeting vector as shown above was cloned and further, transfected into C57BL/6N mouse embryonal stem (ES) cells. The transfection of ES cells was performed according to state of the art techniques (Hogan et al., Manipulating the Mouse Embryo: A Laboratory Manual, Cold Spring Harbor Laboratory Press, Cold Spring Harbor, New York, 1994 and Jackson and Abbott, Mouse Genetics and Transgenics: A Practical Approach, Oxford University Press, Oxford, England, 1999). After homologous recombination targeted ES cell clones, which were identified by Southern Blot analysis, were injected into blastocysts to generate chimeric mice using standard techniques (Tymms and Kola: Gene Knock Out Protocols, Humana Press 2001). After successful transmission of the germline of the chimeric mice generated, they were crossed to an Alzheimer's disease mouse model to produce homozygous SULT4A1 knockout mice on an Alzheimer's disease background.

Sult4A1 transgenic mice were generated to specifically expressing human Sult4A1 cDNA in neuronal cells. For this purpose the open reading frame of the human Sult4A1 (ORF SULT4A1) cDNA was cloned into the murine Thy1.2 expression cassette containing a brain-specific Thy-1 regulatory sequence (Thy-1.2 promotor and regulatory elements) to direct neuronal specific expression and thus, causes expression only in brain cells (Luthi and van der Putten, J. Neuroscience 1997, 1 7:4688-4699). The transgene was cloned into a Rosa26 targeting vector in order to integrate the sequences into the Rosa26 gene locus (mouse Rosa beta geo 26 gene on chromosome 6; Seibler et al, Nucleis Acids Research 2003, 31 :e12). 5'prime of the transgene a Neo selection marker was placed (see Figure 21). The Neo cassette contains a splice acceptor allowing expression of the Neo selection marker driven by the Rosa promoter from the endogenous Rosa26 gene (Friedrich and Soriano, Genes Dev. 1991, 5:1513-1523; Zambrowicz et al., Proc Natl Acad Sci USA et al., 94:3789-3794). 3'prime of the Neo marker a poly A signal (pA) will stop transcription and the influence of the Rosa promoter. The targeting vector contains Rosa26 homology sequences from genomic 129S6 DNA to allow homologous recombination into the Rosa26 gene locus. The short homology arm (SA) consists of approximately 1.1 kb, the long horology arm (LA) consists of about 4.3 kb.

The targeting vector was cloned and further, transfected into C57Bl/6N mouse ES cells (Hogan et al., Manipulating the Mouse Embryo: A Laboratory Manual, Cold Spring Harbor Laboratory Press, Cold Spring Harbor, New York, 1994 and Jackson and Abbott, Mouse Genetics and Transgenics: A Practical Approach, Oxford University Press, Oxford, England, 1999). After homologous recombination targeted ES cell clones, which were identified by Southern Blot analysis, were injected into blastocyst to generate chimeric mice using standard techniques (Tymms and Kola, Gene Knockout Protocols, Humana Press 2001). After successful transmission of the germline of the chimeric mice generated, they were crossed to an Alzheimer's disease mouse model to produce transgenic SULT4A1 mice on an Alzheimer's disease background.

(viii) Generation of transgenic Drosophila melanogaster:

Human BACE transgenic flies and human SULT4A1 transgenic flies were generated according to Greeve et al. (Greeve et al., J. Neurosci. 2004, 24: 3899-3906) and as described in the present invention. A 918 bp NotI/BssHI-blunt fragment containing the entire open reading frame of SULT4A1sv1 (SEQ ID NO.6) was subcloned into the vector pUAST NotI/XbaI-blunt downstream of the GAL4 binding sites UAS (Brand and Perrimon, Development 1993, 118: 401-15). P-element-mediated germline transformation was performed as described by Spradling and Rubin (Rubin and Spradling, Science 1982, 218: 348-53; Spradling and Rubin, Science 1982, 218: 341-7). Twenty independent human SULT4A1 transgenic fly lines were generated and three different lines were used for the analysis.

Human APP and *Drosophila* Presenilin transgenic flies, the UAS-APP69511 and the UAS-DPsn-mutants (L235P), were kindly provided by R. Paro and E. Fortini (Fossgreen et al., Proc Natl Acad Sci U S A 1998, 95: 13703-8; Ye and Fortini, J Cell Biol 1999, 146: 1351-64). The actin-GAL4 line was obtained from the Bloomington stock center. The gmr-GAL4 line from F. Pignoni was used to achieve the eye-specific expression of the transgenes.

Genetic crosses were set up on standard Drosophila culture medium at 25°C. Genotypes used were: w; UAS-$hAPP_{695}$, UAS-hBACE437/CyO; gmr-GAL4/Tm3 - w; UAS-$hAPP_{695}$, UAS-hBACE437/CyO; gmr-GAL4,UAS-DPsnL235P/Tm3 - w; UAS-SULT4A1sv1#3 ($3^{rd}$ chromosome, viable insertion) - w; UAS-SULT4A1sv1 #8/Tm3 - w; UAS-SULT4A1sv1#22/CyO.

For immunohistochemical and histological analysis the adult flies were immunostained and prepared according to the following methods. For immunostaining adult flies were fixed in 4% paraformaldehyde for 3 hours, washed in 1 x PBS and transferred to 25% sucrose for an overnight incubation at 4°C. Flies were decapitated with a razor blade, and the heads were imbedded in Tissue Tek (Sakura) and snap frozen. 10μm horizontal frozen sections were prepared on a cryostat (Leica CM3050S). Immunostaining was done with the Vectastain Elite kit (Vector Laboratories) according to the instructions of the manufacturer. The following primary antibodies were used: 24B10 (alpha-chaoptin, 1:5) provided by the Developmental Studies.

Generation of a Hybridoma Bank: For thioflavin S staining sections were counterstained for 5 minutes in Mayers Hemalum (Sigma), rinsed for 10 minutes in tap water and stained for 3 minutes in 1% thioflavin S (Sigma) watery solution. Slides were rinsed in several changes of distilled water, incubated for 15 minutes in 1% acetic acid, rinsed in tap water and mounted in Vectashield mounting medium (Vector laboratories). Slides were analyzed under an Olympus BX51 fluorescence microscope (430 nm excitation, 550 nm emission).

For the protein analyses by western blotting, fly heads were homogenized in 1×PBS, 5m M EDTA, 0.5% Triton X-100 and a protease-inhibitor mix Complete (Roche Applied Science). Equal amounts of protein were separated by 10% SDS-PAGE, transferred to Immobilon membranes (Millipore GmbH), blocked in 5% low fat milk for two hours at room temperature and incubated with the monoclonal antibody 22C11 (APP N-terminal specific, Chemicon international). Bound antibodies were detected with goat anti-mouse peroxidase conjugated secondary antibodies (Dianova). For immunoprecipitation fly heads were homogenized as described above and lysates were treated as described in the antibodies protocol guide from Clontech. The antibodies mab 6E10 (alpha-Abetal-16, Signet Pathology Systems) and mab 4G8 (alpha-Abeta17-24, Signet Pathology Systems) were used for immunoprecipitation. Samples were separated on 10-20% gradient Novex Tris-Tricine gels (Invitrogen) and blotted onto Protran BA 79 Cellulosenitrate membranes (0.1μm, Schleicher/Schuell, Dassel, Germany). Detection of beta-amyloid was performed as described (Ida et al., J Biol Chem 1996, 271: 22908-14) using mab 6E10 and goat anti-mouse peroxidase conjugated secondary antibody (Dianova).

For the detection of human SULT4A1 expression in transgenic *Drosophila* a reverse transcriptase PCR (RT-PCR Reaction) reaction was performed using SULT4A1 splice variant 1 specific primers as described in the present invention (Example (iv)).

To characterize the potential impact of human SULT4A1 expression on the ne uropathology associated with amyloidogenic processing of human APP (beta-amyloid precursor protein, hAPP) in transgenic flies SULT4A1 sv1 was co-ex pressed with hAPP and human BACE (Beta site APP cleaving enzyme, hBACE) in the adult retina by using the eye-specific GAL4 line gmr-GAL4. Transgenic expression of SULT4A1sv1 under the control of gmr-GAL4 was confirmed by RT-PCR using splice variant 1 specific primers. The PCR reaction resulted in a fragment with the expected melting temperature and size as shown in Figure 22A. Three different transgenic fly lines were used (SULT4svA1#3, #8 and #22). Re lative differences in the expression efficiency were calculated according to the cycle number (Figure 22B) and normalized to housekeeping genes. Based on this calculation SULT4A1 sv1 transgenic fly line #3 is 1.8 times stronger expressed than fly line #8 and 2.7 times stronger than fly line #22 (Figure 22B).

Expression of hAPP and hBACE in the adult retina of *Drosophila* leads to age-dependent degeneration of photoreceptor cells (Greeve et al., J. Neurosci. 2004, 24: 3899-3906). Co-expression of SULT4A1 rescues photoreceptor cell degeneration in young (Figure 23A and B, 3 days old) and aging flies (Figure 23A and B, 8 and 19 days old flies) depending on the expression efficiency of the SULT4A1sv1 transgene. Fly line SULT4A1sv1#3 (Figure 23A) which shows the highest expression efficiency calculated according to the RT-PCR reaction is leading to a prominent rescue of the degenerative phenotype in young and old flies whereas fly line SULT4A1sv1#8 (Figure 23B) rescues only weakly and SULT4A1sv1 (Figure 23C) shows no interference with degenerative phenotypes in hAPP/hBACE/hSULT4A1sv1 triple transgenic flies. None of the transgenic fly lines interferes with proper assembly and differentiation of photoreceptor cells when expressed under the control of gmr-GAL4 alone (Figure 23A-B, first column).

To further characterize the neuroprotective effect of SULT4A1 on photoreceptor cell degeneration in hAPP/hBACE expressing flies we investigated the expression and processing of hAPP in triple transgenic flies and focused on the co-expression of SULT4A1sv1#3 which shows the highest impact on the neuropathology associated with APP processing in our model system. Western blots of head homogenates of flies expressing hAPP/hBACE and flies expressing hAPP/hBACE/SULT4A1sv1#3 that were probed with a human APP N-terminal antibody 22C11 demonstrate that neither the expression nor the beta-site cleavage of the hAPP full-length protein are effected by the co-expression of SULT4A1sv1#3 (Figure 24B). In addition, the generation of the amyloidogenic peptide Abeta is not effected by co-expressing SULT4A1sv1 as demonstrated in immunoprecipitation experiments using the Abeta N-terminal specific antibody 6E10 (see Figure 23B). Amyloid plaque deposition in the retina of hAPP/hBACE expressing flies is accelerated by the co-expression of mutant forms of Presenilin (Greeve et al., J. Neurosci. 2004, 24: 3899-3906). Therefore, we investigated the onset of Thioflavin S positive amyloid plaques in flies expressing hAPP/hBACE/DPsnL235P and compared them to flies expressing hAPP/hBACE/DPsnL235P and SULT4A1 sv1 #3. No difference in the time of onset of plaque deposition between control flies and flies co-expressing SULT4A1sv1#3 were observed as demonstrated in Figure 25. Thus, the neuroprotective effect of SULT4A1sv1#3 does not depend on the expression and, amyloidogenic processing of hAPP or amyloid plaque formation in this invertebrate model system of Alzheimer's disease. Said analysis as described above are shown in Figures 23, 24 and 25.

## Claims

1. A method of diagnosing or prognosticating Alzheimer's disease in a subject, or determining whether a subject is at increased risk of developing said disease, **characterized in that** said method comprises determining

   (i) a level and/or an activity of a cytosolic sulfotransferase family 4A member 1 splice variant 1 of SEQ ID NO: 1 and/or
   (ii) a level and/or an activity of a cytosolic sulfotransferase family 4A member 1 splice variant 2 of SEQ ID NO: 2

   in a sample obtained from said subject and comparing said level and/or said activity to a reference value representing a known disease or health status, thereby diagnosing or prognosticating Alzheimer's disease in said subject, or determining whether said subject is at increased risk of developing Alzheimer's disease.

2. An in vitro assay for screening for a modulator of Alzheimer's disease by measuring

   (i) a level and/or an activity of a cytosolic sulfotransferase family 4A member 1 splice variant 1 of SEQ ID NO: 1 and/or
   (ii) a level and/or an activity of a cytosolic sulfotransferase family 4A member 1 splice variant 2 of SEQ ID NO: 2

   **characterized in that** said method comprises:

   (a) contacting a cell with a test compound;
   (b) measuring the activity and/or level of one or more substances recited in (i) or (ii);
   (c) measuring the activity and/or level of one or more substances recited in (i) or (ii) in a control cell not contacted with said test compound; and
   (d) comparing the levels and/or activities of the substance in the cells of step (b) and (c), wherein an alteration in the activity and/or level of substances in the contacted cells indicates that the test compound is a modulator of said disease.

3. In vitro use of protein molecules of SEQ ID NO. 1 and/or SEQ ID NO. 2 as diagnostic targets for detecting Alzheimer's disease.

4. In vitro use of protein molecules of SEQ ID NO. 1 and/or SEQ ID NO. 2 as screening targets for reagents or compounds preventing, or treating, or ameliorating Alzheimer's disease.

5. Use of an antibody specifically immunoreactive with an immunogen, **characterized in that** said immunogen is the cytosolic sulfotransferase family 4A member 1 splice variant 1 of SEQ ID NO: 1 or the cytosolic sulfotransferase family 4A member 1 splice variant 2 of SEQ ID NO: 2 for detecting the pathological state of a cell in a sample obtained from a subject, comprising immunocytochemical staining of said cell with said antibody, wherein an altered degree of staining, or an altered staining pattern in said cell compared to a cell representing a known health status indicates an Alzheimer's disease pathological state of said cell.

## Patentansprüche

1. Verfahren zum Diagnostizieren oder Prognostizieren der Alzheimer-Krankheit bei einem Patienten oder zum Bestimmen, ob ein Patient ein erhöhtes Risiko hat, diese Krankheit zu entwickeln, **dadurch gekennzeichnet, dass** das Verfahren das Bestimmen umfasst von:

   (i) einem Pegel und/oder einer Aktivität einer Spleißvariante 1 des Mitglieds 1 der Familie 4A der cytosolischen Sulfotransferasen mit der SEQ ID Nr. 1; und/oder
   (ii) einem Pegel und/oder einer Aktivität einer Spleißvariante 2 des Mitglieds 1 der Familie 4A der cytosolischen Sulfotransferasen mit der SEQ ID Nr. 2;

   in einer Probe, die von dem Patienten erhalten wurde, und Vergleichen der Pegel und/oder der Aktivität mit einem Referenzwert, der einen bekannten Krankheits- oder Gesundheitszustand darstellt, wodurch Alzheimer-Krankheit bei dem Patienten diagnostiziert oder prognostiziert wird oder bestimmt wird, ob der Patient ein erhöhtes Risiko hat, Alzheimer-Krankheit zu entwickeln.

2. In-vitro-Assay zum Suchen nach einem Modulator der Alzheimer-Krankheit durch Messen von:

   (i) einem Pegel und/oder einer Aktivität einer Spleißvariante 1 des Mitglieds 1 der Familie 4A der cytosolischen Sulfotransferasen mit der SEQ ID Nr. 1; und/oder
   (ii) einem Pegel und/oder einer Aktivität einer Spleißvariante 2 des Mitglieds 1 der Familie 4A der cytosolischen Sulfotransferasen mit der SEQ ID Nr. 2;

   **dadurch gekennzeichnet, dass** das Verfahren Folgendes umfasst:

   a) In-Kontakt-Bringen einer Zelle mit einer Testverbindung;
   b) Messen der Aktivität und/oder des Pegels von einer oder mehreren Substanzen, die in (i) oder (ii) genannt sind;
   c) Messen der Aktivität und/oder des Pegels von einer oder mehreren Substanzen, die in (i) oder (ii) genannt sind, in einer Kontrollzelle, die nicht mit der Testverbindung in Kontakt gebracht wurde; und
   d) Vergleichen der Pegel und/oder Aktivitäten der Substanz in den Zellen der Schritte (b) und (c), wobei eine Veränderung der Aktivität und/oder des Pegels der Substanzen in den kontaktierten Zellen anzeigt, dass die Testverbindung ein Modulator dieser Krankheit ist.

3. In-vitro-Verwendung von Proteinmolekülen der SEQ ID Nr. 1 und/oder SEQ ID Nr. 2 als diagnostische Targets zum Nachweis der Alzheimer-Krankheit.

4. In-vitro-Verwendung von Proteinmolekülen der SEQ ID Nr. 1 und/oder SEQ ID Nr. 2 als Screening-Targets für Reagentien oder Verbindungen, die die Alzheimer-Krankheit verhindern oder behandeln oder lindern.

5. Verwendung eines Antikörpers, der gegenüber einem Immunogen spezifisch immunreaktiv ist, **dadurch gekennzeichnet, dass** das Immunogen die Spleißvariante 1 des Mitglieds 1 der Familie 4A der cytosolischen Sulfotransferasen mit der SEQ ID Nr. 1 oder die Spleißvariante 2 des Mitglieds 1 der Familie 4A der cytosolischen Sulfotransferasen mit der SEQ ID Nr. 2 ist, zum Nachweis des pathologischen Zustands einer Zelle in einer Probe, die von einem Patienten erhalten wurde, umfassend das immuncytochemische Anfärben der Zelle mit dem Antikörper, wobei ein veränderter Grad der Anfärbung oder ein verändertes Anfärbungsmuster in der Zelle im Vergleich zu einer Zelle, die einen bekannten Gesundheitszustand repräsentiert, einen pathologischen Zustand der Zelle bezüglich Alzheimer-Krankheit anzeigt.

## Revendications

1. Procédé pour diagnostiquer ou pronostiquer la maladie d'Alzheimer chez un sujet, ou déterminer si un sujet présente un risque accru de développer ladite maladie, **caractérisé en ce que** ledit procédé comprend la détermination

   (i) d'un taux et/ou d'une activité d'un variant d'épissage 1 d'un membre 1 de la famille de sulfotransférase cytosolique 4A de SEQ ID NO : 1 et/ou
(ii) d'un taux et/ou d'une activité d'un variant d'épissage 2 d'un membre 1 de la famille de sulfotransférase cytosolique 4A de SEQ ID NO : 2

dans un échantillon obtenu à partir dudit sujet et la comparaison dudit taux et/ou de ladite activité à une valeur de référence représentant une maladie ou un état de santé connu, diagnostiquant ou pronostiquant ainsi la maladie d'Alzheimer chez ledit sujet, ou déterminant si ledit sujet présente un risque accru de développer la maladie d'Alzheimer.

**2.** Dosage *in vitro* permettant de cribler un modulateur de la maladie d'Alzheimer en mesurant

(i) un taux et/ou une activité d'un variant d'épissage 1 d'un membre 1 de la famille de sulfotransférase cytosolique 4A de SEQ ID NO : 1 et/ou
(ii) un taux et/ou une activité d'un variant d'épissage 2 d'un membre 1 de la famille de sulfotransférase cytosolique 4A de SEQ ID NO : 2

**caractérisé en ce que** ledit procédé comprend :

(a) la mise en contact d'une cellule avec un composé d'essai ;
(b) la mesure de l'activité et/ou du taux d'une ou plusieurs substances citées en (i) ou (ii) ;
(c) la mesure de l'activité et/ou du taux d'une ou plusieurs substances citées en (i) ou (ii) dans une cellule témoin non mise en contact avec ledit composé d'essai ; et
(d) la comparaison des taux et/ou des activités de la substance dans les cellules de l'étape (b) et de l'étape (c), où une modification de l'activité et/ou du taux de substances dans les cellules mises en contact indique que le composé d'essai est un modulateur de ladite maladie.

**3.** Utilisation *in vitro* de molécules de protéine de SEQ ID NO : 1 et/ou SEQ ID NO : 2 comme cibles de diagnostic permettant de détecter la maladie d'Alzheimer.

**4.** Utilisation *in vitro* de molécules de protéine de SEQ ID NO : 1 et/ou SEQ ID NO : 2 comme cibles de criblage pour des réactifs ou composés prévenant, ou traitant, ou améliorant la maladie d'Alzheimer.

**5.** Utilisation d'un anticorps spécifiquement immunoréactif avec un immunogène, **caractérisé en ce que** ledit immunogène est le variant d'épissage 1 du membre 1 de la famille de sulfotransférase cytosolique 4A de SEQ ID NO : 1 ou le variant d'épissage 2 du membre 1 de la famille de sulfotransférase cytosolique 4A de SEQ ID NO : 2 pour détecter l'état pathologique d'une cellule dans un échantillon obtenu à partir d'un sujet, comprenant la coloration immunocytochimique de ladite cellule avec ledit anticorps, dans laquelle un degré modifié de coloration, ou un motif de coloration modifié dans ladite cellule en comparaison à une cellule représentant un état de santé connu indique un état pathologique de la maladie d'Alzheimer pour ladite cellule.

Fig. 1: **Identification of differentially expressed genes in a fluorescence differential display screen**

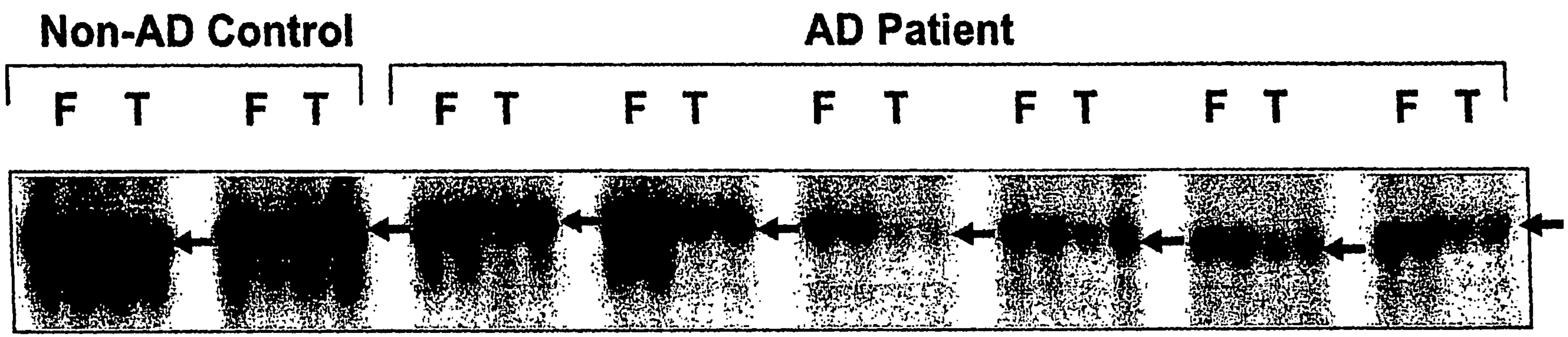

**Fig. 2: Verification of differential expression of SULT4A1 splice variant 1 and/or splice variant 2 by quantitative RT-PCR**

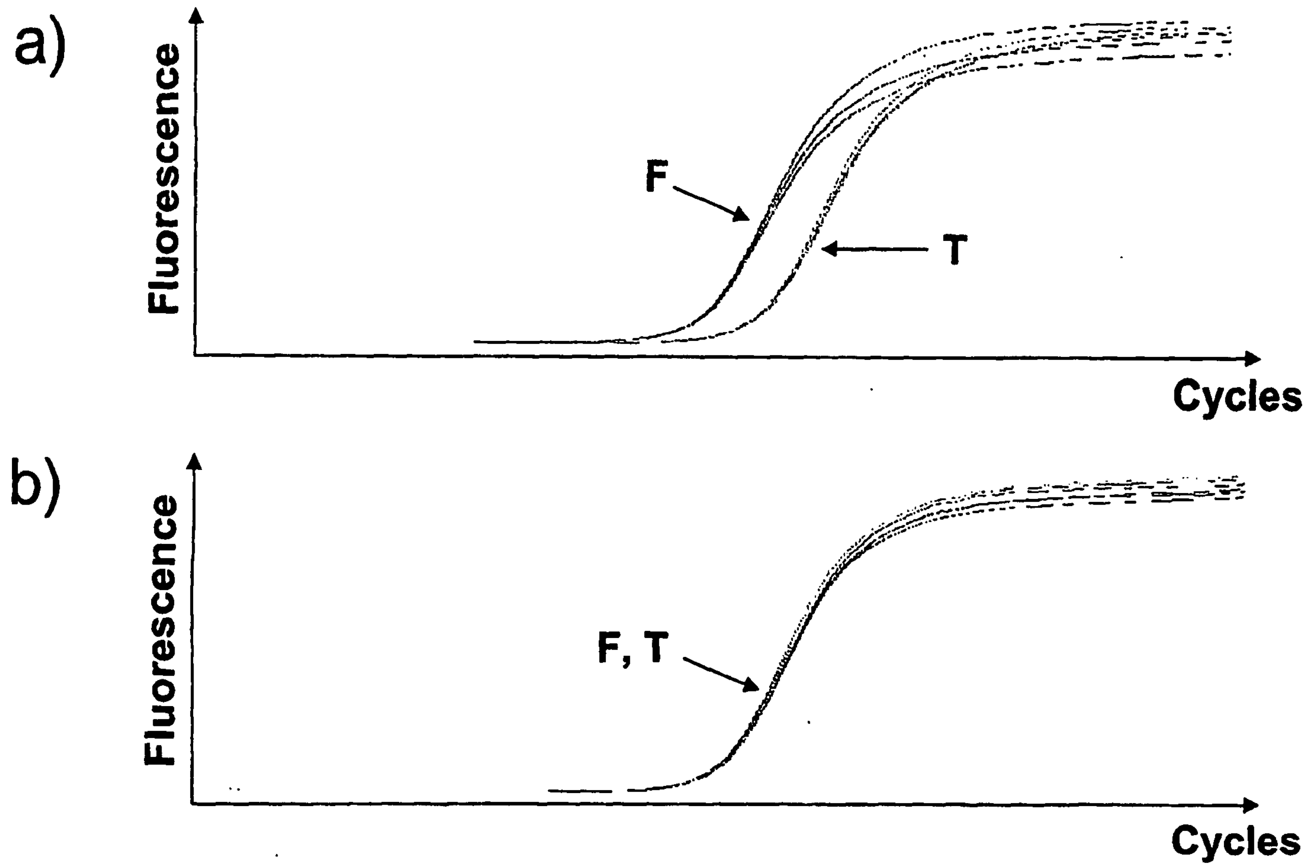

Fig. 3: Verification of differential expression of SULT4A1 splice variant 1 and/or splice variant 2 by quantitative RT-PCR

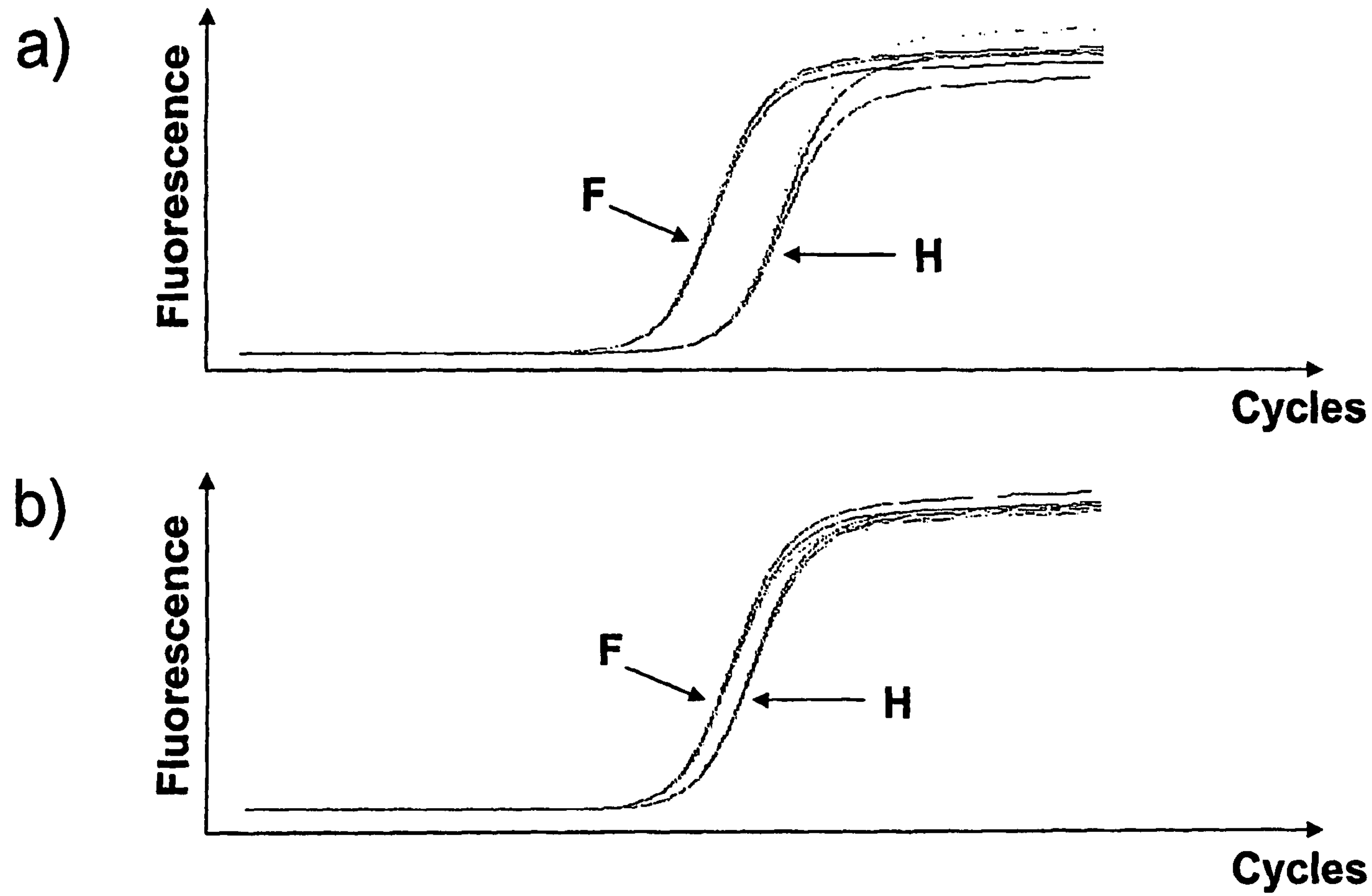

**Fig. 4: Verification of differential expression of SULT4A1 splice variant 1 by quantitative RT-PCR**

a)

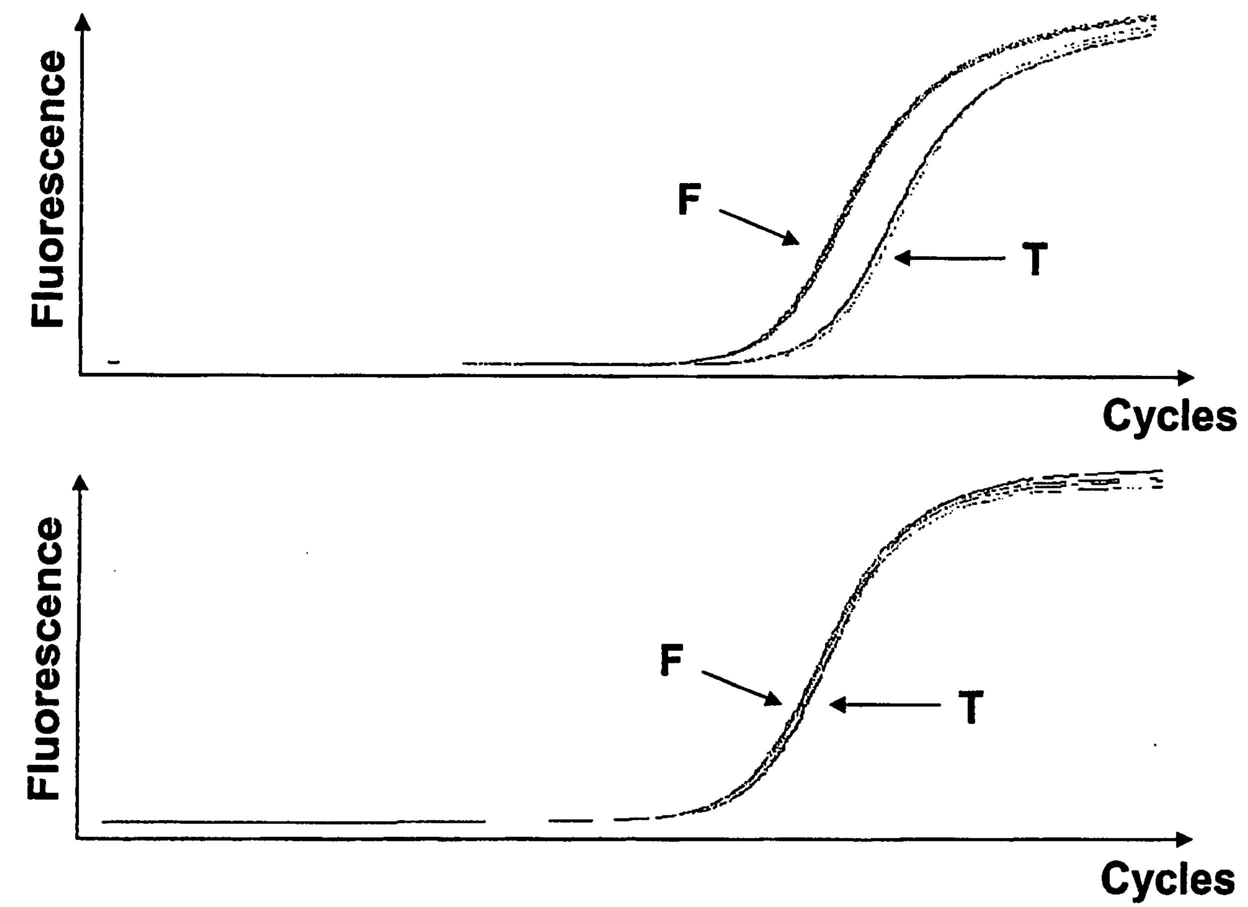

**Fig. 5: Verification of differential expression of SULT4A1 splice variant 2 by quantitative RT-PCR**

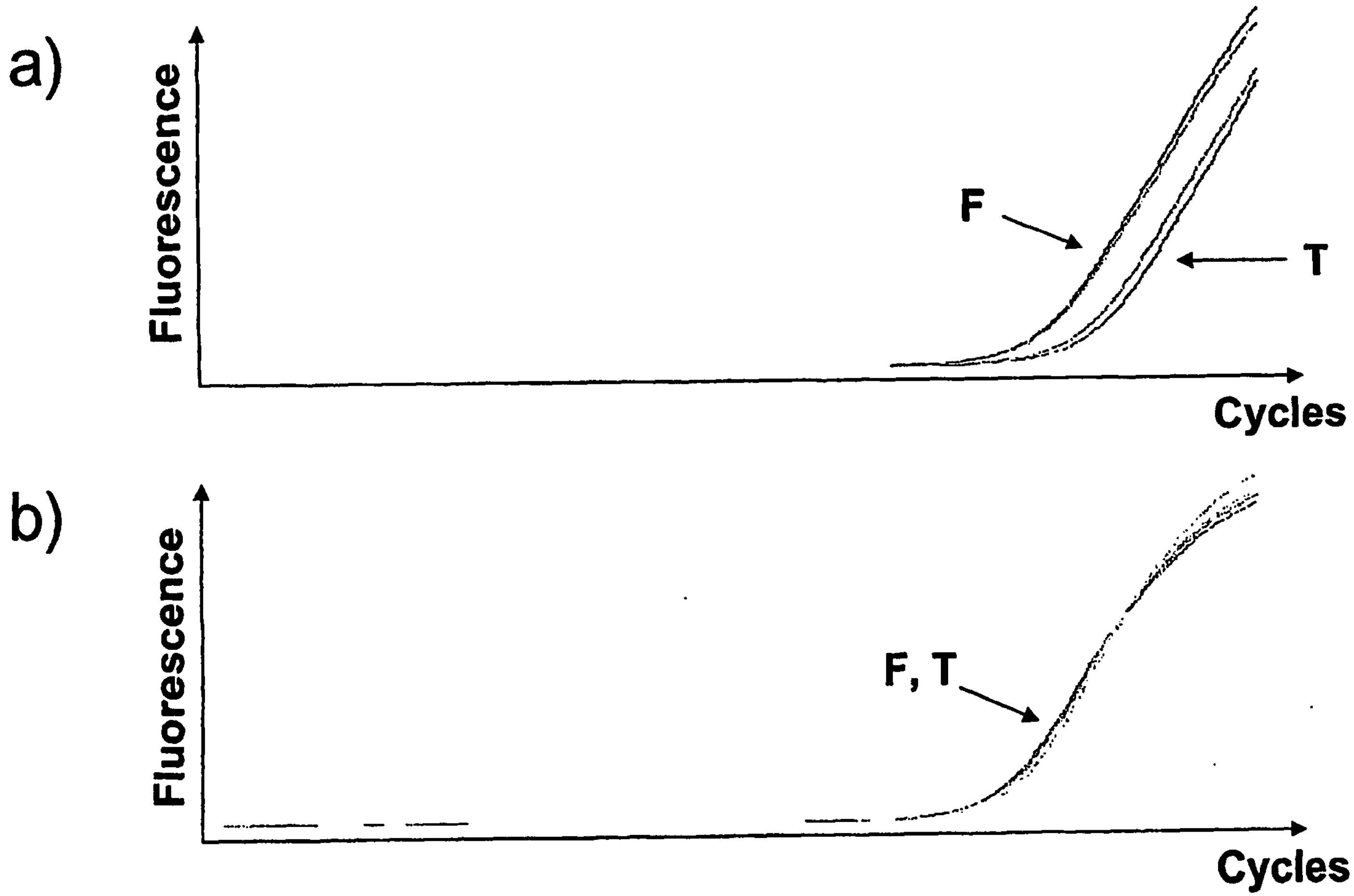

## Fig. 6: Analysis of absolute mRNA expression of SULT4A1sv1 and/or SULT4A1sv2

Comparison of Braak 0-3 with 4-6

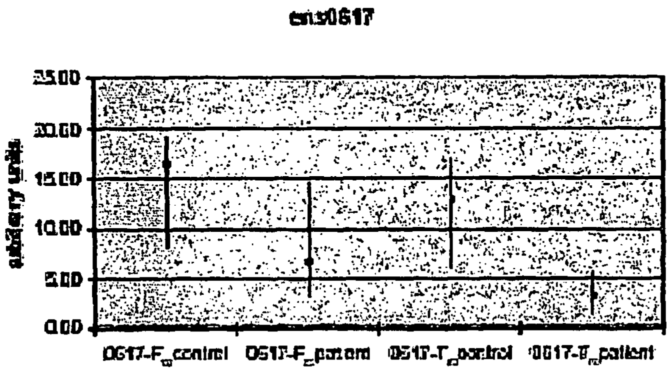


Comparison of Braak 0-2 with Braak 3-6

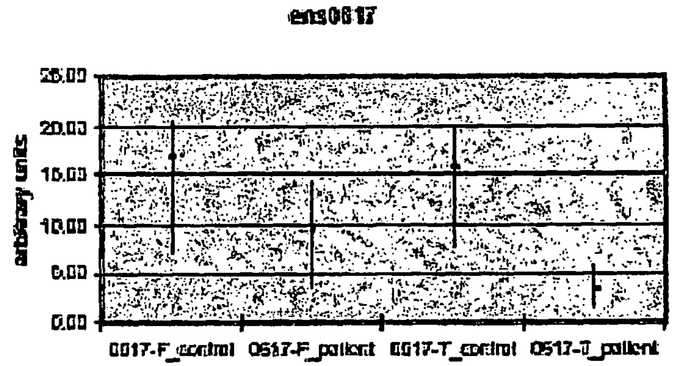


Comparison of Braak 0-1 with 2-6

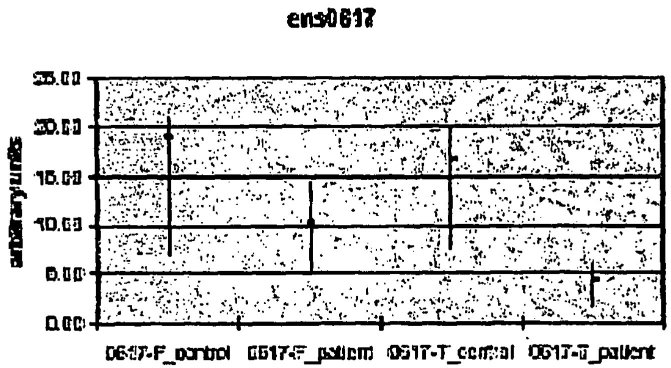


Comparison of Braak 0-1 with 2-3 and 4-6

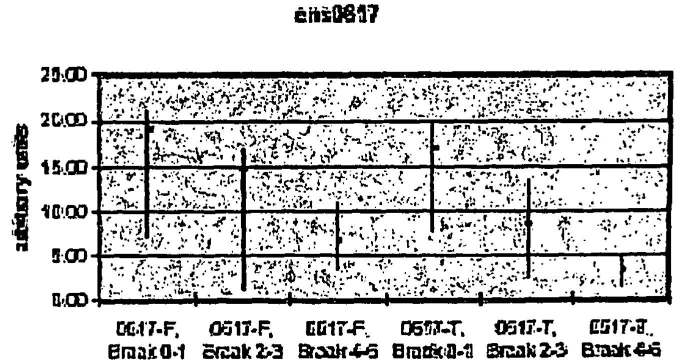

# Fig. 7: Analysis of absolute mRNA expression of SULT4A1sv1

Comparison of Braak 0-3 with 4-6

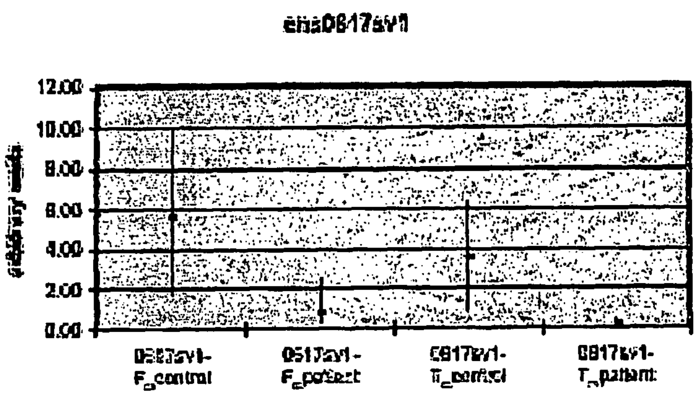


Comparison of Braak 0-2 with Braak 3-6

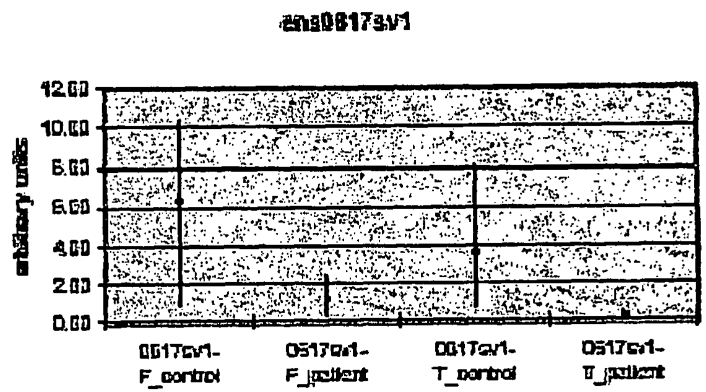


Comparison of Braak 0-1 with 2-6

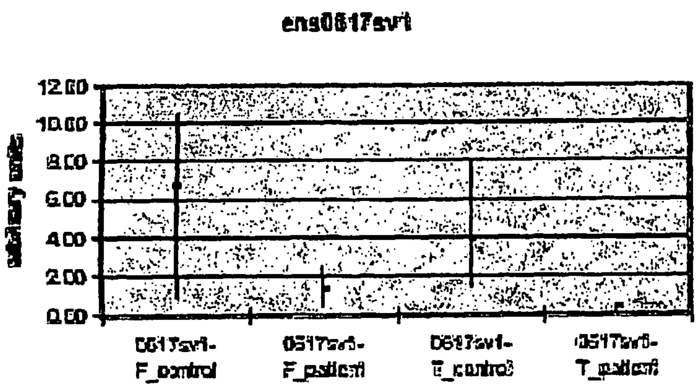


Comparison of Braak 0-1 with 2-3 and 4-6

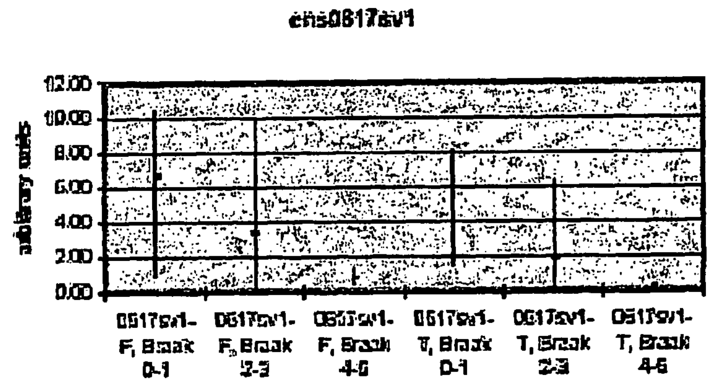

**Fig. 8: SEQ ID NO. 1:**
**amino acid sequence of**
**human SULT4A1 protein,**
**splice variant 1**

**Length: 284 aa**

```
  1 MAESEAETPS TPGEFESKYF EFHGVRLPPF CRGKMEEIAN FPVRPSDVWI
 51 VTYPKSGTSL LQEVVYLVSQ GADPDEIGLM NIDEQLPVLE YPQPGLDIIK
101 ELTSPRLIKS HLPYRFLPSD LHNGDSKVIY MARNPKDLVV SYYQFHRSLR
151 TMSYRGTFQE FCRRFMNDKL GYGSWFEHVQ EFWEHRMDSN VLFLKYEDMH
201 RDLVTMVEQL ARFLGVSCDK AQLEALTEHC HQLVDQCCNA EALPVGRGRV
251 GLWKDIFTVS MNEKFDLVYK QKMGKCDLTF DFYL
```

**Fig. 9: SEQ ID NO. 2:**
**amino acid sequence of human SULT4A1 protein, splice variant 2**

Length: 171 aa

```
  1  MAESEAETPS TPGEFESKYF EFHGVRLPPF CRGKMEEIAN FPVRPSDVWI
 51  VTYPKSVGYG SWFEHVQEFW EHRMDSNVLF LKYEDMHRDL VTMVEQLARF
101  LGVSCDKAQL EALTEHCHQL VDQCCNAEAL PVGRGRVGLW KDIFTVSMNE
151  KFDLVYKQKM GKCDLTFDFY L
```

## Fig. 10: SEQ ID NO. 3: nucleotide sequence of human SULT4A1 cDNA, splice variant 1

**Length: 2419 bp**

```
   1 GCGACGGCGA CGGCGGCGGC ATGGCGGAGA GCGAGGCCGA GACCCCCAGC
  51 ACCCCGGGGG AGTTCGAGAG CAAGTACTTC GAGTTCCATG GCGTGCGGCT
 101 GCCGCCCTTC TGCCGCGGGA AGATGGAGGA GATCGCCAAC TTCCCGGTGC
 151 GGCCCAGCGA CGTGTGGATC GTCACCTACC CCAAGTCCGG CACCAGCTTG
 201 CTGCAGGAGG TGGTCTACTT GGTGAGCCAG GGCGCTGACC CCGATGAGAT
 251 CGGCTTGATG AACATCGACG AGCAGCTCCC GGTCCTGGAG TACCCACAGC
 301 CGGGCCTGGA CATCATCAAG GAACTGACCT CTCCCCGCCT CATCAAGAGC
 351 CACCTGCCCT ACCGCTTTCT GCCCTCTGAC CTCCACAATG GAGACTCCAA
 401 GGTCATCTAT ATGGCTCGCA ACCCCAAGGA TCTGGTGGTG TCTTATTATC
 451 AGTTCCACCG CTCTCTGCGG ACCATGAGCT ACCGAGGCAC CTTTCAAGAA
 501 TTCTGCCGGA GGTTTATGAA TGATAAGCTG GGCTACGGCT CCTGGTTTGA
 551 GCACGTGCAG GAGTTCTGGG AGCACCGCAT GGACTCGAAC GTGCTTTTTC
 601 TCAAGTATGA AGACATGCAT CGGGACCTGG TGACGATGGT GGAGCAGCTG
 651 GCCAGATTCC TGGGGGTGTC CTGTGACAAG GCCCAGCTGG AAGCCCTGAC
 701 GGAGCACTGC CACCAGCTGG TGGACCAGTG CTGCAACGCT GAGGCCCTGC
 751 CCGTGGGCCG GGGAAGAGTT GGGCTGTGGA AGGACATCTT CACCGTCTCC
 801 ATGAATGAGA AGTTTGACTT GGTGTATAAA CAGAAGATGG GAAAGTGTGA
 851 CCTCACGTTT GACTTTTATT TATAATAACA GAAACAACAA CCTGCATGCT
 901 CACAATACCC AGACAGTCTA CTAGCCAAAA GTCCTGTATG CATTCATTTA
 951 TTCCTTGCTG GACAAACTCT GGAAGCAGCG TGTGAAACAG CGGGGGAAGG
1001 GAAGAGCGGC GTGAGCGGAG GGAGTGTGAT GATTCCCAAC CGAAGCAGCT
1051 GTCTCGCCTT TAGAACGTGC AGCCTCTCCA TGTCTGATTA CAAACAGTCT
1101 CCACATTGCA GTTCCAATGG CCTGGACCGT AAGGATAAAG CCTGTAATAT
1151 ATGCAACTAG AATGTCTGCC TTTTCAACCC CGTATTATTG TATTTTATAG
1201 AGCTTTTCAC TGGAAATCTA CATAAATGTC AGTAAACCAA ATAAAAGTTC
1251 ATTTCCAAGG GGAATCAGGA GCGAGCCACA CCCGAATGGT AGAAAGATCT
1301 CAGGGTTAAC TCTTTATTTT TGTAGTTTTA TTATCTAAGG CACAGCCATT
1351 CTGTTCTCAC TTGGTTCTGA GATAGTGGTG AGAACAGAGG ATGAGTTGGG
1401 TCTGTTGGGG GGAATCTGGA CACTTGTTTA TTCTGACGGA GTTCACTTCT
1451 TCAGAACCTT CCTGAAATGA GCAGAAATTG TTCACTAGGT CTTCAGAATG
1501 GACGTCCTTC TGCCAGAGAC TTCCAGCGGG CGGCTCCAAA GGCCCAATGC
1551 AGAGGAGCCC GCGGAGCATG TGCTGAGGGA AGTCTGCCTG GTGAGGCTGG
1601 CAGGTGGGAG TCTAATGCAG TCAGGAGCAT TTGCATGCAG TGGGTGGAGA
1651 GTCGGCCACC AAAGGACCGA GTTGCGCTCG GAATTTGAGC TGAATTCCAC
1701 AGCCTTACTT TGTTTCCTGA AGTGATAGCC TACTAATGCT GGCAAGCAGA
1751 TGCTTAATAG TAAATTTCTA AAATCCCCGG GTCTTTATCA TTCAGTTTGT
1801 TCTGTGCACC TGAGGCGCTC AGCCGTGGGA GGACCATTTT GCGAGTGTAG
1851 CCCTGTTTCA CTCGGATCAG GTTGGCACGG CCGCCTGCGT GTCTGTCCAC
1901 CTCATCCCTC CGTGTATCTG AGGGAGTAAA GGTGAGGTCT TTATTGCTTC
1951 ACTGCCTAAT TTTCTCACCC ACATTCGCTG AAGCGATGGA GAGTCGGGGG
2001 CCAGTAGCCA GCCAACCCCG TGGGGACCGG GGTTGTCTGT CATTTATGTG
2051 GCTGGAAAGC ACCCAAAGTG GTGGTCAGGA GGGTCGCTGC TGTGGAAGGG
```

2101 GTCTCCGTTC TTGGTGCTGT ATTTGAAACG GGTGTAGAGA GAAGCTTGTG
2151 TTTTTGTTTG TAATGGGGAG AAGCGTGGCC AGGCAGGTGG CACGTGGCAT
2201 CGCATGGTGG GCTCGGCAGC ACCTTGCCTG TGTTTCTGTG AGGGAGGCTG
2251 CTTTCTGTGA AATTTCATTT ATATTTTTCT ATTTTTAGTA CTGTATGGAT
2301 GTTACTGAGC ACTACACATG ATCCTTCTGT GCTTGCTTGC ATCTTTAATA
2351 AAGACATGTT CCCGGCGTTG CAAAAAAAAA AAAAAAAAAA AAAAAAAAAA
2401 AAAAAAAAAA AAAAAAAAA

# Fig. 11: SEQ ID NO. 4: nucleotide sequence of human SULT4A1 cDNA, splice variant 2

**Length: 2080 bp**

| | | | | | |
|---|---|---|---|---|---|
| 1 | GCGACGGCGA | CGGCGGCGGC | ATGGCGGAGA | GCGAGGCCGA | GACCCCCAGC |
| 51 | ACCCCGGGGG | AGTTCGAGAG | CAAGTACTTC | GAGTTCCATG | GCGTGCGGCT |
| 101 | GCCGCCCTTC | TGCCGCGGGA | AGATGGAGGA | GATCGCCAAC | TTCCCGGTGC |
| 151 | GGCCCAGCGA | CGTGTGGATC | GTCACCTACC | CCAAGTCCGT | GGGCTACGGC |
| 201 | TCCTGGTTTG | AGCACGTGCA | GGAGTTCTGG | GAGCACCGCA | TGGACTCGAA |
| 251 | CGTGCTTTTT | CTCAAGTATG | AAGACATGCA | TCGGGACCTG | GTGACGATGG |
| 301 | TGGAGCAGCT | GGCCAGATTC | CTGGGGGTGT | CCTGTGACAA | GGCCCAGCTG |
| 351 | GAAGCCCTGA | CGGAGCACTG | CCACCAGCTG | GTGGACCAGT | GCTGCAACGC |
| 401 | TGAGGCCCTG | CCCGTGGGCC | GGGGAAGAGT | TGGGCTGTGG | AAGGACATCT |
| 451 | TCACCGTCTC | CATGAATGAG | AAGTTTGACT | TGGTGTATAA | ACAGAAGATG |
| 501 | GGAAAGTGTG | ACCTCACGTT | TGACTTTTAT | TTATAATAAC | AGAAACAACA |
| 551 | ACCTGCATGC | TCACAATACC | CAGACAGTCT | ACTAGCCAAA | AGTCCTGTAT |
| 601 | GCATTCATTT | ATTCCTTGCT | GGACAAACTC | TGGAAGCAGC | GTGTGAAACA |
| 651 | GCGGGGGAAG | GGAAGAGCGG | CGTGAGCGGA | GGGAGTGTGA | TGATTCCCAA |
| 701 | CCGAAGCAGC | TGTCTCGCCT | TTAGAACGTG | CAGCCTCTCC | ATGTCTGATT |
| 751 | ACAAACAGTC | TCCACATTGC | AGTTCCAATG | GCCTGGACCG | TAAGGATAAA |
| 801 | GCCTGTAATA | TATGCAACTA | GAATGTCTGC | CTTTTCAACC | CCGTATTATT |
| 851 | GTATTTTATA | GAGCTTTTCA | CTGGAAATCT | ACATAAATGT | CAGTAAACCA |
| 901 | AATAAAAGTT | CATTTCCAAG | GGGAATCAGG | AGCGAGCCAC | ACCCGAATGG |
| 951 | TAGAAAGATC | TCAGGGTTAA | CTCTTTATTT | TTGTAGTTTT | ATTATCTAAG |
| 1001 | GCACAGCCAT | TCTGTTCTCA | CTTGGTTCTG | AGATAGTGGT | GAGAACAGAG |
| 1051 | GATGAGTTGG | GTCTGTTGGG | GGGAATCTGG | ACACTTGTTT | ATTCTGACGG |
| 1101 | AGTTCACTTC | TTCAGAACCT | TCCTGAAATG | AGCAGAAATT | GTTCACTAGG |
| 1151 | TCTTCAGAAT | GGACGTCCTT | CTGCCAGAGA | CTTCCAGCGG | GCGGCTCCAA |
| 1201 | AGGCCCAATG | CAGAGGAGCC | CGCGGAGCAT | GTGCTGAGGG | AAGTCTGCCT |
| 1251 | GGTGAGGCTG | GCAGGTGGGA | GTCTAATGCA | GTCAGGAGCA | TTTGCATGCA |
| 1301 | GTGGGTGGAG | AGTCGGCCAC | CAAAGGACCG | AGTTGCGCTC | GGAATTTGAG |
| 1351 | CTGAATTCCA | CAGCCTTACT | TTGTTTCCTG | AAGTGATAGC | CTACTAATGC |
| 1401 | TGGCAAGCAG | ATGCTTAATA | GTAAATTTCT | AAAATCCCCG | GGTCTTTATC |
| 1451 | ATTCAGTTTG | TTCTGTGCAC | CTGAGGCGCT | CAGCCGTGGG | AGGACCATTT |
| 1501 | TGCGAGTGTA | GCCCTGTTTC | ACTCGGATCA | GGTTGGCACG | GCCGCCTGCG |
| 1551 | TGTCTGTCCA | CCTCATCCCT | CCGTGTATCT | GAGGGAGTAA | AGGTGAGGTC |
| 1601 | TTTATTGCTT | CACTGCCTAA | TTTTCTCACC | CACATTCGCT | GAAGCGATGG |
| 1651 | AGAGTCGGGG | GCCAGTAGCC | AGCCAACCCC | GTGGGGACCG | GGGTTGTCTG |
| 1701 | TCATTTATGT | GGCTGGAAAG | CACCCAAAGT | GGTGGTCAGG | AGGGTCGCTG |
| 1751 | CTGTGGAAGG | GGTCTCCGTT | CTTGGTGCTG | TATTTGAAAC | GGGTGTAGAG |
| 1801 | AGAAGCTTGT | GTTTTTGTTT | GTAATGGGGA | GAAGCGTGGC | CAGGCAGGTG |
| 1851 | GCACGTGGCA | TCGCATGGTG | GGCTCGGCAG | CACCTTGCCT | GTGTTTCTGT |
| 1901 | GAGGGAGGCT | GCTTTCTGTG | AAATTTCATT | TATATTTTTC | TATTTTTAGT |
| 1951 | ACTGTATGGA | TGTTACTGAG | CACTACACAT | GATCCTTCTG | TGCTTGCTTG |
| 2001 | CATCTTTAAT | AAAGACATGT | TCCCGGCGTT | GCAAAAAAAA | AAAAAAAAAA |
| 2051 | AAAAAAAAAA | AAAAAAAAAA | AAAAAAAAAA | | |

# Fig. 12: SEQ ID NO. 5

**Length: 32 bp**

1 GATTGCATCT TTAATAAAGA CATGTTCCCG GC

## Fig. 13: SEQ ID NO. 6: nucleotide sequence of human SULT4A1 coding sequence

**Length: 855 bp**

```
  1  ATGGCGGAGA GCGAGGCCGA GACCCCCAGC ACCCCGGGGG AGTTCGAGAG
 51  CAAGTACTTC GAGTTCCATG GCGTGCGGCT GCCGCCCTTC TGCCGCGGGA
101  AGATGGAGGA GATCGCCAAC TTCCCGGTGC GGCCCAGCGA CGTGTGGATC
151  GTCACCTACC CCAAGTCCGG CACCAGCTTG CTGCAGGAGG TGGTCTACTT
201  GGTGAGCCAG GGCGCTGACC CCGATGAGAT CGGCTTGATG AACATCGACG
251  AGCAGCTCCC GGTCCTGGAG TACCCACAGC CGGGCCTGGA CATCATCAAG
301  GAACTGACCT CTCCCCGCCT CATCAAGAGC CACCTGCCCT ACCGCTTTCT
351  GCCCTCTGAC CTCCACAATG GAGACTCCAA GGTCATCTAT ATGGCTCGCA
401  ACCCCAAGGA TCTGGTGGTG TCTTATTATC AGTTCCACCG CTCTCTGCGG
451  ACCATGAGCT ACCGAGGCAC CTTTCAAGAA TTCTGCCGGA GGTTTATGAA
501  TGATAAGCTG GGCTACGGCT CCTGGTTTGA GCACGTGCAG GAGTTCTGGG
551  AGCACCGCAT GGACTCGAAC GTGCTTTTTC TCAAGTATGA AGACATGCAT
601  CGGGACCTGG TGACGATGGT GGAGCAGCTG GCCAGATTCC TGGGGGTGTC
651  CTGTGACAAG GCCCAGCTGG AAGCCCTGAC GGAGCACTGC CACCAGCTGG
701  TGGACCAGTG CTGCAACGCT GAGGCCCTGC CCGTGGGCCG GGGAAGAGTT
751  GGGCTGTGGA AGGACATCTT CACCGTCTCC ATGAATGAGA AGTTTGACTT
801  GGTGTATAAA CAGAAGATGG GAAAGTGTGA CCTCACGTTT GACTTTTATT
851  TATAA
```

# Fig. 14: Alignment of SEQ ID NO. 5 with human SULT4A1sv1 and SULT4A1sv2 cDNAs

```
Length: 32 bp

SEQ ID NO.5 : SULT4A1sv1

      1 GATTGCATCTTTAATAAAGACATGTTCCCGGC 32
        | ||||||||||||||||||||||||||||||
   2335 GCTTGCATCTTTAATAAAGACATGTTCCCGGC 2366

SEQ ID NO.5 : SULT4A1sv2

      1 GATTGCATCTTTAATAAAGACATGTTCCCGGC 32
        | ||||||||||||||||||||||||||||||
   1996 GCTTGCATCTTTAATAAAGACATGTTCCCGGC 2027
```

**Fig. 15 :**

| Samples | Controls | | | | AD Patients | | |
|---|---|---|---|---|---|---|---|
| Braak stage | 0 | 1 | 2 | 3 | 4 | 5 | 6 |
| | C011 F1.37 | C005 F1.12 | C008 F1.20 | C025 T1.07 | P012 F2.27 | P010 F7.14 | P014 F2.04 |
| | C012 F1.20 | C014 T2.38 | C031 [illegible] | C035 F1.20 | P016 [illegible] | P011 F2.86 | P017 F1.47 |
| | C026 T1.02 | C028 F1.09 | C033 [illegible] | DE05 F2.50 | P038 F2.44 | P040 F3.03 | P019 F2.38 |
| | C027 T1.17 | C029 F1.27 | C034 F1.09 | DE07 [illegible] | P046 F2.70 | P041 F3.23 | P042 F2.17 |
| | C032 F1.16 | C030 F1.18 | C041 F1.11 | | P047 [illegible] | P048 F1.27 | |
| | | C036 F1.33 | C042 T1.10 | | | P049 [illegible] | |
| | | C038 [illegible] | DE02 F1.35 | | | | |
| | | C039 T1.11 | DE03 T1.17 | | | | |

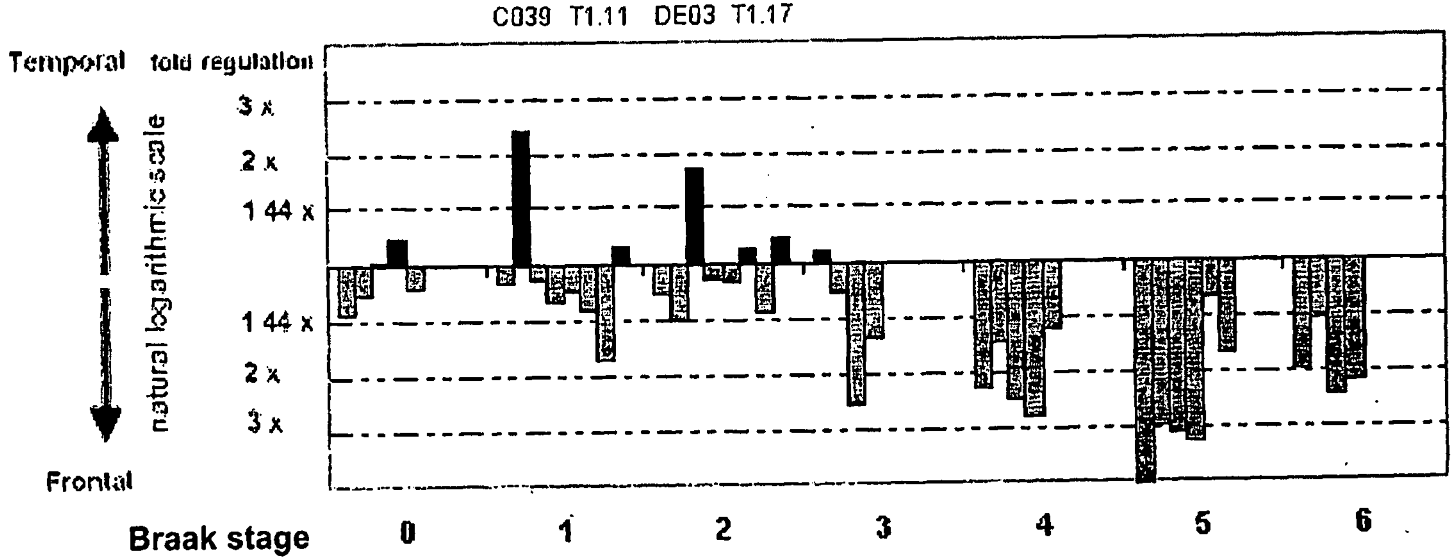

Fig. 16 :

**Samples** — **Controls** (Braak stages 0–3) / **AD Patients** (Braak stages 4–6)

| Braak stage 0 | 1 | 2 | 3 | 4 | 5 | 6 |
|---|---|---|---|---|---|---|
| C011 F2.27 | C005 T1.44 | C008 F2.00 | C025 F2.33 | P012 F4.17 | P010 F7.69 | P014 [illegible] |
| C012 [illegible] | C014 [illegible] | C031 F2.17 | C035 [illegible] | P016 F5.00 | P011 F3.57 | P017 F12.50 |
| C026 F1.15 | C028 [illegible] | C033 | DE05 F3.33 | P038 F10.00 | P040 F4.76 | P019 F16.67 |
| C027 T1.36 | C029 F1.25 | C034 [illegible] | DE07 [illegible] | P046 F2.33 | P041 F9.09 | P042 F2.94 |
| C032 F2.17 | C030 F1.35 | C041 F1.03 | | P047 [illegible] | P048 F1.10 | |
| | C036 [illegible] | C042 F1.30 | | | P049 F1.35 | |
| | C038 F1.33 | DE02 F3.33 | | | | |
| | C039 T1.14 | DE03 T3.45 | | | | |

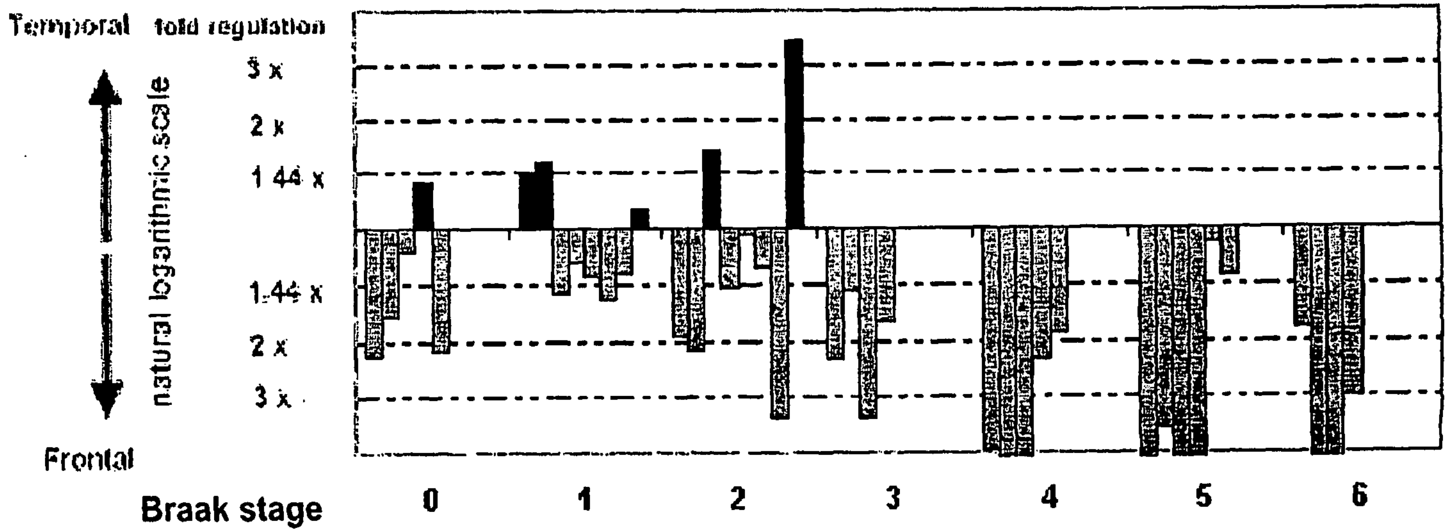

Fig. 17 :

| sample | Δ (fold) (hippocampus/ frontal cortex) |
|---|---|
| control C004 | 0.82 |
| control C005 | 0.56 |
| control C008 | 0.76 |
| patient P010 | 0.13 |
| patient P011 | 0.64 |
| patient P012 | 0.87 |
| patient P014 | 1.37 |
| patient P016 | 0.70 |
| patient P019 | 0.95 |

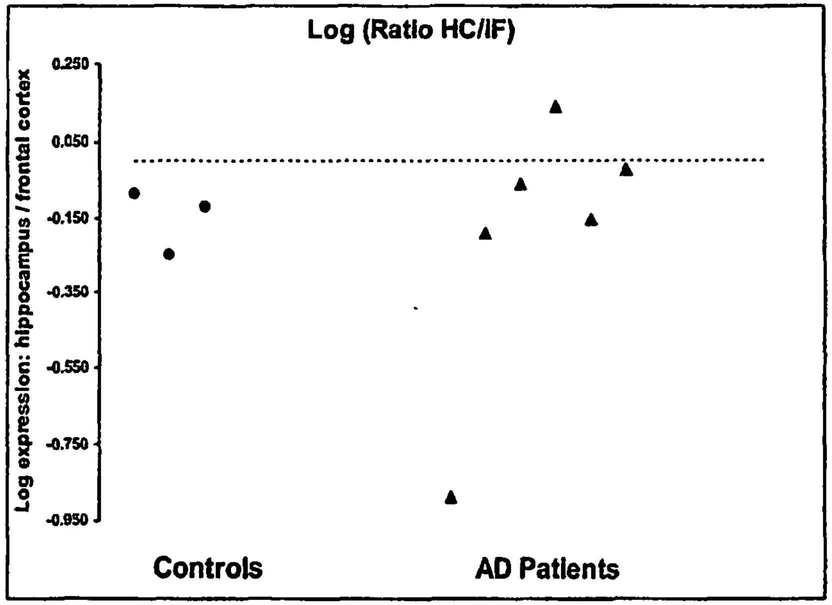

Fig. 18: Immunofluorescence analysis of SULT4A1 protein in neuroglioma cells

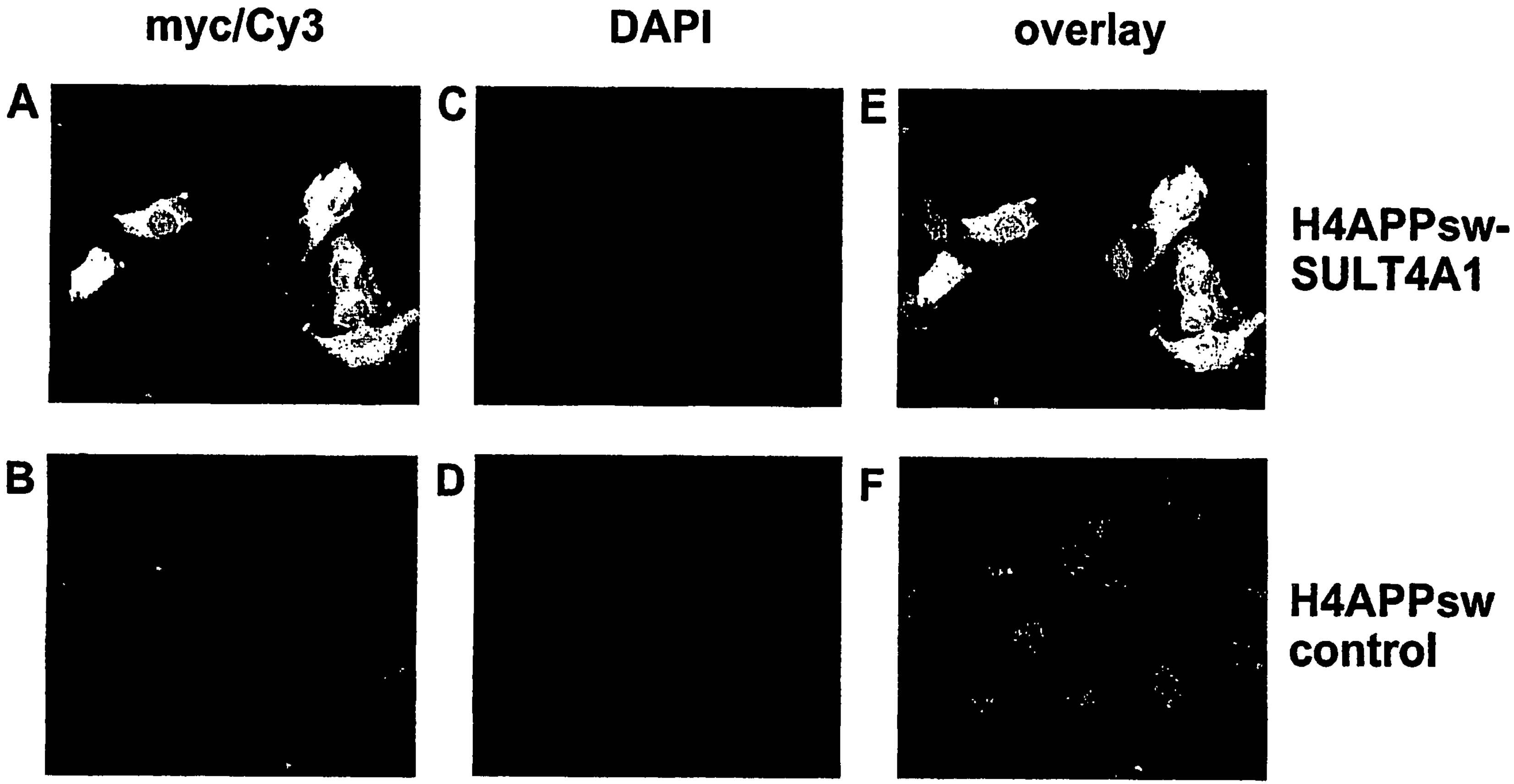

**Fig. 19: Effect of trophic factor deprivation on SULT4A1 over-expressing cells**

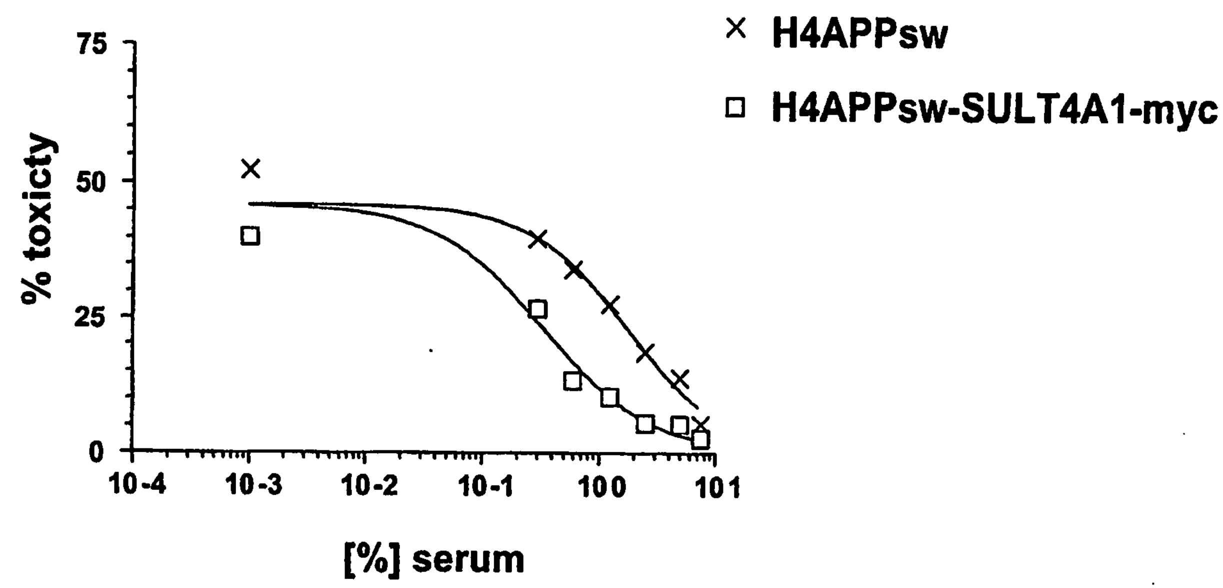

**Fig. 20: Generation of Sult4A1 deficient mice**

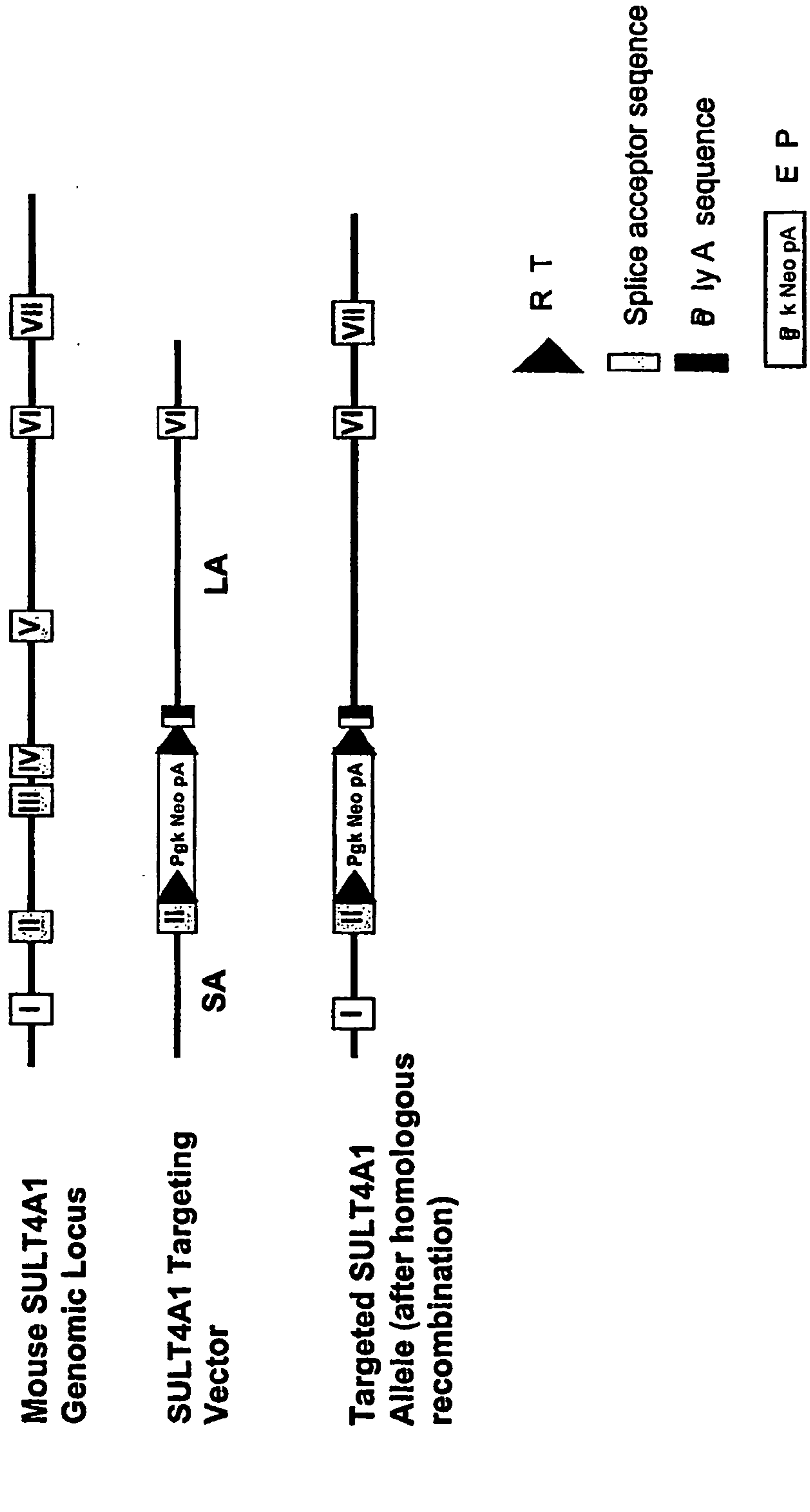

## Fig. 21: Generation of Sult4A1 trangenic mice

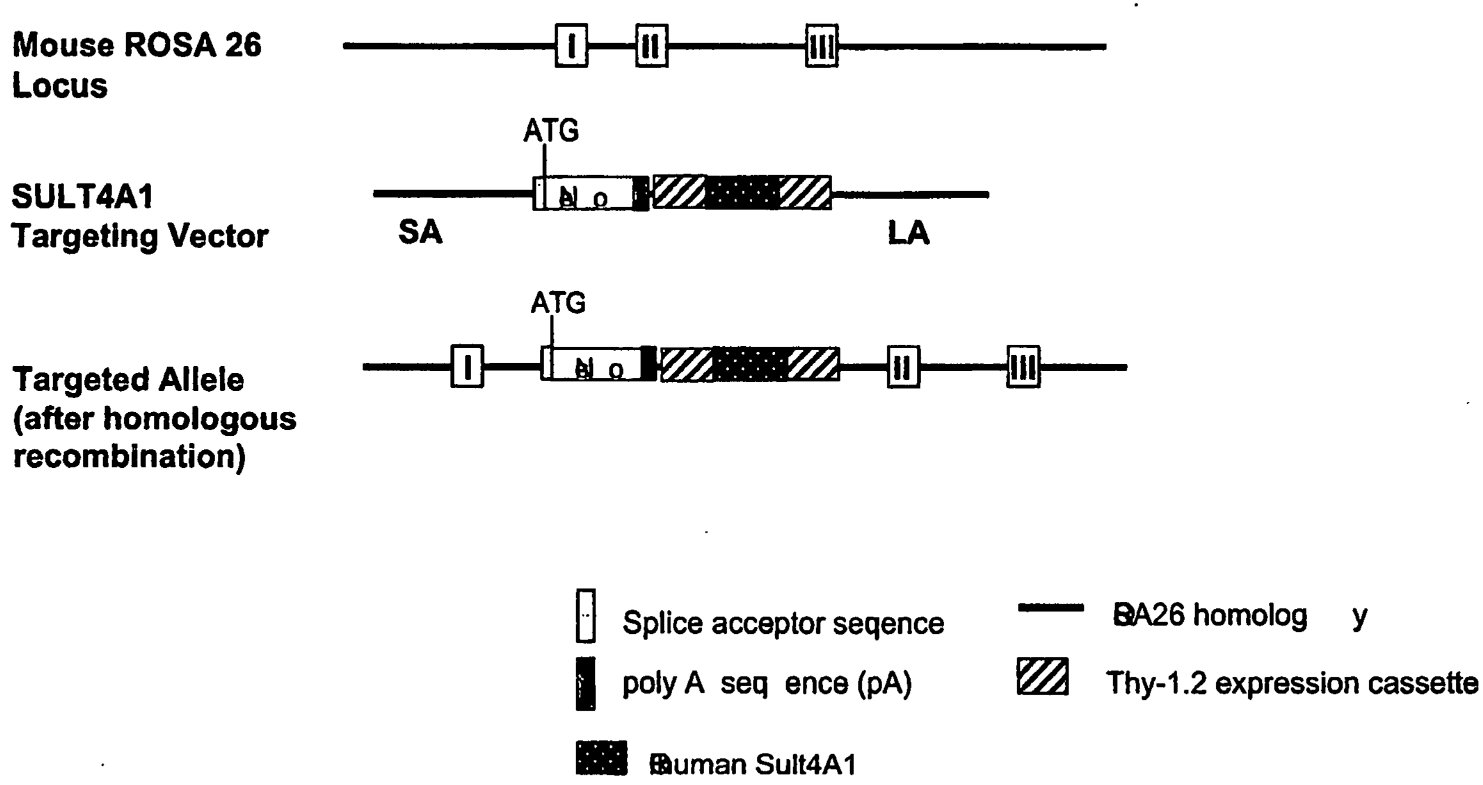

## Fig. 22: Transgenic expression of Sult4A1

A

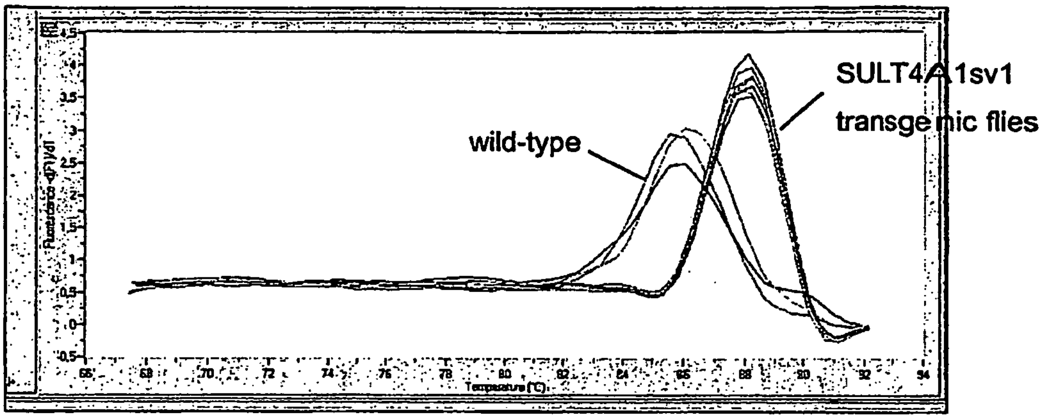


B

| name | cycle number | mean | stdev | error [%] | efficiency (expression normalized to housekeeping gene) |
|---|---|---|---|---|---|
| SULT4A1#3 | 21.34 | 21.300 | 0.14422205 | 0.67709883 | 1.8 times more than SULT4A1#8; 2.7 times more than SULT4A1#22 |
| SULT4A1#3 | 21.14 | | | | |
| SULT4A1#3 | 21.42 | | | | |
| | | | | | |
| SULT4A1#8 | 23.79 | 23.917 | 0.11676187 | 0.48820292 | |
| SULT4A1#8 | 23.94 | | | | |
| SULT4A1#8 | 24.02 | | | | |
| | | | | | |
| SULT4A1#22 | | 23.915 | 0.03535534 | 0.1478375 | |
| SULT4A1#22 | 23.94 | | | | |
| SULT4A1#22 | 23.89 | | | | |

$E = 10^{(-1/slope)}$ slope= -2.960 E= 2.176

Fig. 23: Histological analysis of Sult4A1 expression

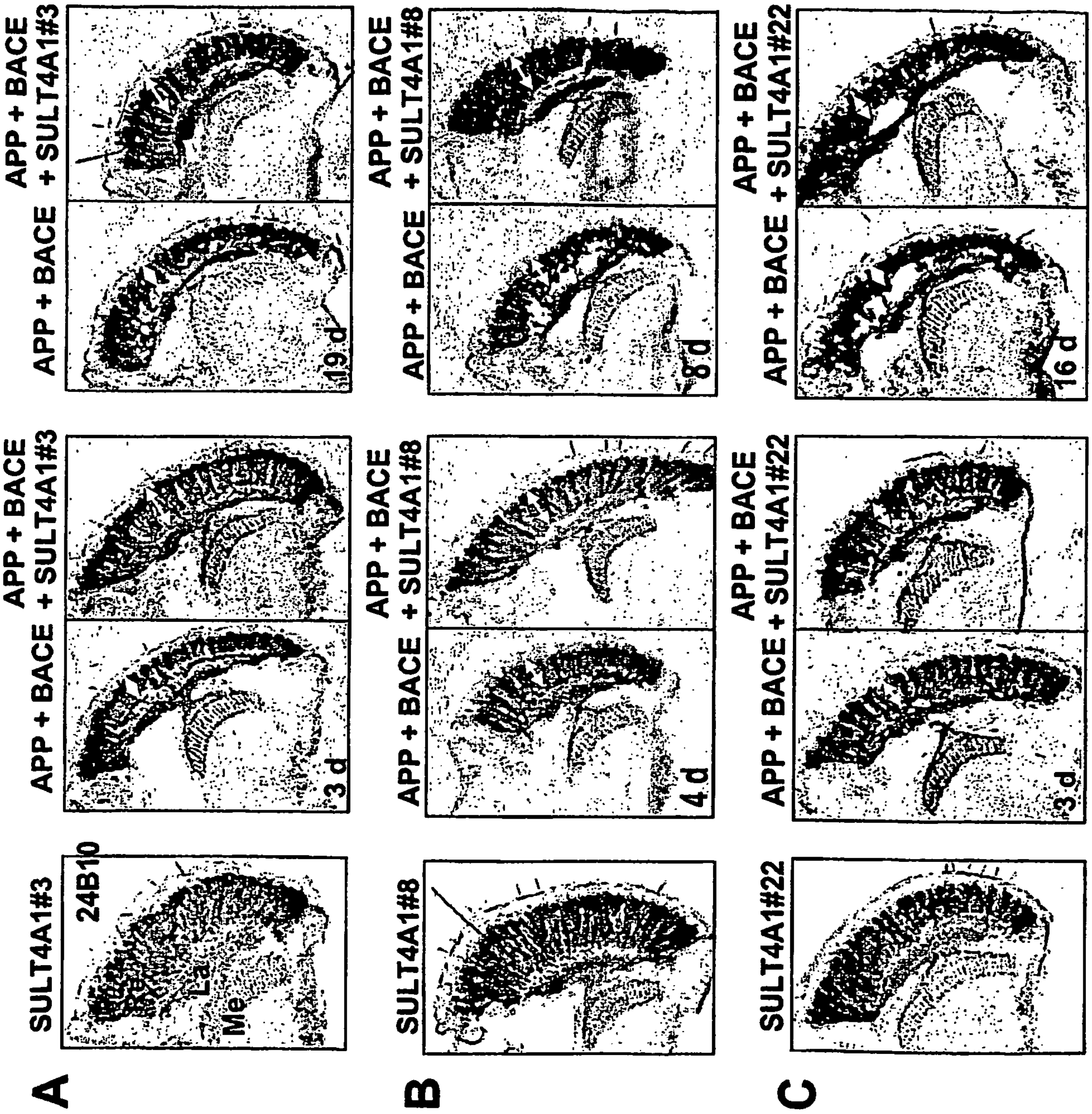

Fig. 24: Expression of full-length and processed hAPP in transgenic flies

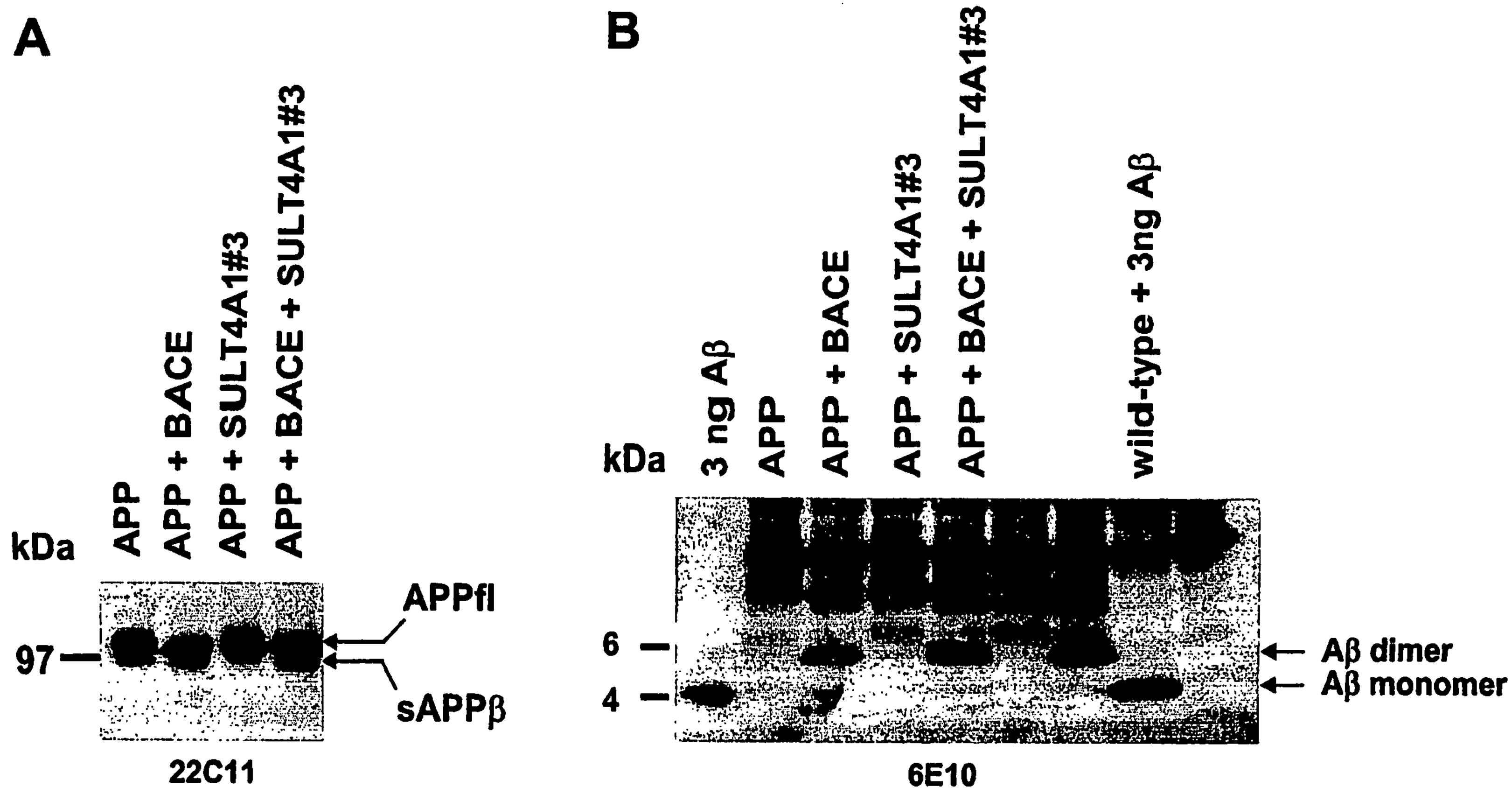

Fig. 25: Thioflavin S positive amyloid plaques in transgenic flies

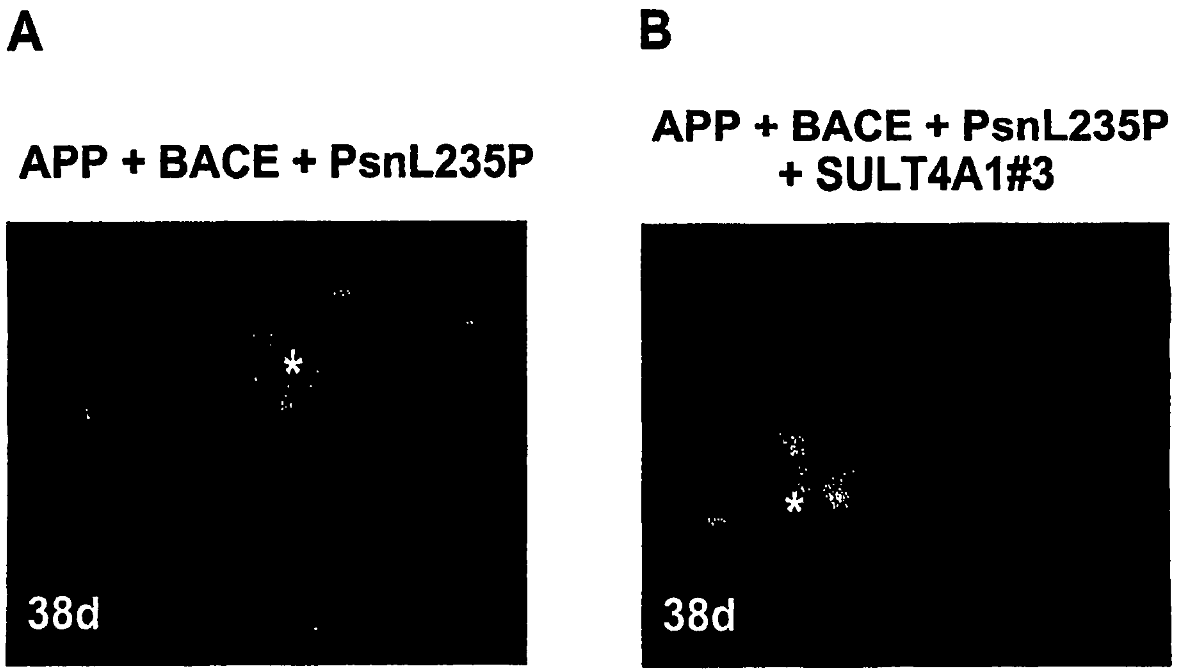

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0014251 A
- WO 0218541 A
- WO 0214543 A
- WO 0052451 A
- WO 0201226 A
- WO 9613744 A
- WO 9816814 A
- WO 9823942 A
- WO 9917086 A
- WO 9934195 A
- WO 0066985 A
- WO 0159436 A
- WO 0159416 A
- US 6150173 A


### Non-patent literature cited in the description


- **VICKERS et al.** *Progress in Neurobiology,* 2000, vol. 60, 139-165
- **SELKOE ; GREENFIELD.** *Physiological Rev,* 2001, vol. 81, 741-66
- *Frontiers Bioscience,* 2000, vol. 5, D72-83
- **BRAAK ; BRAAK.** *Acta Neuropathol,* 1991, vol. 82, 239-259
- **SCHMITT et al.** *Neurology,* 2000, vol. 55, 370-376
- **TERRY et al.** Annals of Neurology. 1981, vol. 10, 184-92
- **STRITTMATTER et al.** *Proc Natl Acad Sci USA,* 1993, vol. 90, 1977-81
- **ROSES.** *Ann NY Acad Sci,* 1998, vol. 855, 738-43
- **GLATT, H. et al.** *Mutation Research,* 2001, vol. 482, 27-40
- *J Neurochem.,* August 2002, vol. 82 (4), 809-18
- **KIM et al.** *Journal of clinical endocrinology and Metabolism,* vol. 88 (11), 5199
- **JANUS, C. et al.** *Biochim Biophys Acta,* 2000, vol. 1502, 63-75
- **FALANY.** *FASEB J.,* 1997, vol. 11, 206-216
- **FALANY et al.** *Biochem. J.,* 2000, vol. 346, 857-864
- **SAKAKIBARA et al.** *Gene,* 2002, vol. 285, 39-47
- **LIDA et al.** *J. Hum. Genet.,* 2001, vol. 46, 225-240
- **ADJEL ; WEINSHILBOUM.** *Biochem. Biophys. Res. Comm.,* 2002, vol. 292, 402-408
- **TERRY et al.** Annals of Neurology. 1981, vol. 10, 184-192
- **ALTSCHUL et al.** *J. Mol. Biol.,* 1990, vol. 215, 403-410
- **ALTSCHUL et al.** *Nucleic Acids Res,* 1997, vol. 25, 3389-3402
- **DEVEREUX et al.** *Nucleic Acids Res.,* 1984, vol. 12, 387
- **PEARSON ; LIPMAN.** *Proc. Natl. Acad. Sci. USA,* 1988, vol. 85, 2444-2448
- **WILBUR ; LIPMAN.** *SIAM J. Appl. Math.,* 1984, vol. 44, 557-567
- **NEEDLEMAN ; WUNSCH.** *J. Mol. Biol.,* 1970, vol. 48, 443-453
- **IQBAL ; SWAAB ; WINBLAD ; WISNIEWSKI.** Alzheimer's Disease and Related Disorders (Etiology, Pathogenesis and Therapeutics). Wiley & Sons, 1999
- **SCINTO ; DAFFNER.** Early Diagnosis of Alzheimer's Disease. Humana Press, 2000
- **MAYEUX ; CHRISTEN.** Epidemiology of Alzheimer's Disease: From Gene to Prevention. Springer Press, 1999
- **YOUNKIN ; TANZI ; CHRISTEN.** Presenilins and Alzheimer's Disease. Springer Press, 1998
- **BRAAK ; BRAAK.** *Acta Neuropathology,* 1991, vol. 82, 239-259
- **SAMBROOK ; RUSSELL.** Molecular Cloning: A Laboratory Manual. Cold Spring Harbor Laboratory Press, 2001
- **SCHENA M.** Microarray Biochip Technology. Eaton Publishing, 2000
- **HARLOW ; LANE.** Using Antibodies: A Laboratory Manual. Cold Spring Harbor Laboratory Press, 1999
- **EDWARDS R.** Immunodiagnostics: A Practical Approach. Oxford University Press, 1999
- **BEHR.** *Acc Chem Res,* 1993, vol. 26, 274-278
- **MULLIGAN.** *Science,* 1993, vol. 260, 926-931
- **WOLFF.** *Curr Opin Neurobiol,* 1993, vol. 3, 743-748
- **GILLESPIE.** *DN&P,* 1992, vol. 5, 389-395
- **AGRAWAL ; AKHTAR.** *Trends Biotechnol,* 1995, vol. 13, 197-199

- **CROOKE.** *Biotechnology,* 1992, vol. 10, 882-6
- **BARINAGA.** *Science,* 1993, vol. 262, 1512-1514
- **WICKSTROM.** *Trends Biotechnol,* 1992, vol. 10, 281-287
- **HANNON.** *Nature,* 2002, vol. 418, 244-251
- **MC CELLAND ; PARDEE.** Expression Genetics: Accelerated and High-Throughput Methods. Eaton Publishing, 1992
- **LANZA et al.** *Nature Medicine,* 1999, vol. 9, 975-977
- **PETERSON DA.** *Curr. Opin. Pharmacol.,* 2002, vol. 2, 34-42
- **COLMAN A.** *Drug Discovery World,* 2001, vol. 7, 66-71
- **JANUS ; WESTAWAY.** *Physiology Behavior,* 2001, 873-886
- **RICHARDS et al.** *J. Neuroscience,* 2003, vol. 23, 8989-9003
- **GÖTZ et al.** *J. Biological Chemistry,* 2001, vol. 276, 529-534
- **CAPECCHI.** *Science,* 1989, vol. 244, 1288-1292
- **HOGAN et al.** Manipulating the Mouse Embryo: A Laboratory Manual. Cold Spring Harbor Laboratory Press, 1994
- **JACKSON ; ABBOTT.** Mouse Genetics and Transgenics: A Practical Approach. Oxford University Press, 1999
- **SCHWARZ et al.** *Biochemistry,* 1999, vol. 38, 9456-9464
- **KRIVOSHEEV ; USANOV.** *Biochemistry-Moscow,* 1997, vol. 62, 1064-1073
- **HARLOW et al.** Antibodies, A Laboratory Manual. Cold Spring Harbor Laboratory Press, 1988
- **DUBEL ; BREITLING.** Recombinant Antibodies. Wiley-Liss, 1999
- **EDWARDS R.** Immunodiagnostics: A Practical Approach. Oxford University Press, 1999
- **LIANG ; PARDEE.** *Science,* 1995, vol. 267, 1186-7
- **VON DER KAMMER et al.** *Nucleic Acids Research,* 1999, vol. 27, 2211-2218
- **LIANG et al.** *Nucleic Acids Research,* 1994, vol. 22, 5763-5764
- **ZHAO et al.** *Biotechniques,* 1995, vol. 18, 842-850
- **FALANY et al.** *Biochem J.,* 2000, 857-864
- **TYMMS ; KOLA.** Gene Knock Out Protocols. Humana Press, 2001
- **LUTHI ; VAN DER PUTTEN.** *J. Neuroscience,* 1997, vol. 1 7, 4688-4699
- **SEIBLER et al.** *Nucleis Acids Research,* 2003, vol. 31, e12
- **FRIEDRICH ; SORIANO.** *Genes Dev.,* 1991, vol. 5, 1513-1523
- **ZAMBROWICZ et al.** *Proc Natl Acad Sci USA,* vol. 94, 3789-3794
- **TYMMS ; KOLA.** Gene Knockout Protocols. Humana Press, 2001
- **GREEVE et al.** *J. Neurosci.,* 2004, vol. 24, 3899-3906
- **BRAND ; PERRIMON.** *Development,* 1993, vol. 118, 401-15
- **RUBIN ; SPRADLING.** *Science,* 1982, vol. 218, 348-53
- **SPRADLING ; RUBIN.** *Science,* 1982, vol. 218, 341-7
- **FOSSGREEN et al.** *Proc Natl Acad Sci U S A,* 1998, vol. 95, 13703-8
- **YE ; FORTINI.** *J Cell Biol,* 1999, vol. 146, 1351-64
- **IDA et al.** *J Biol Chem,* 1996, vol. 271, 22908-14